# EUROPEAN PATENT SPECIFICATION

(11) **EP 1 513 802 B1**
(45) Date of publication and mention of the grant of the patent: **19.08.2009**
(21) Application number: 03740286.4
(22) Date of filing: 18.06.2003
(51) Int. Cl.: C07C 311/13, C07D 285/08, C07C 323/20, C07D 239/60, C07C 317/20, C07D 213/85, C07D 271/10, C07D 241/42, C07D 239/26, C07D 277/34, A01N 41/06

(54) **ALPHA-SULFONYLAMINO-ACETONITRILES**
ALPHA-SULFONYLAMINO-ACETONITRILE
ACETONITRILES ALPHA-SULFONYLAMINES

(30) Priority: 19.06.2002 GB 0214116
(43) Date of publication of application: 16.03.2005
(73) Proprietor: Syngenta Participations AG, 4058 Basel (CH)
(72) Inventor: EBERLE, Martin, CH-4103 Bottmingen (CH); STIERLI, Daniel Syngenta Crop Protection AG, CH-4058 Basel (CH); MÜLLER, Urs Syngenta Crop Protection AG, CH-4058 Basel (CH)
(86) International application number: PCT/EP2003/006482
(87) International publication number: WO 2004/000797

(56) References cited:
- EP-A- 0 176 327
- DE-A- 2 854 932

## Description

The present invention relates to novel α-sulfonylamino-acetonitrile derivatives of formula I. It further encompasses the preparation of the novel active compounds and to agrochemical compositions comprising at least one of these novel compounds as active ingredient. The invention further relates to the preparation of the said compositions and to the use of the compounds or of the compositions for controlling or preventing the infestation of plants by phytopathogenic microorganisms, especially fungi.

The α-sulfonylamino-acetonitrile derivatives according to the present invention correspond to the general formula I 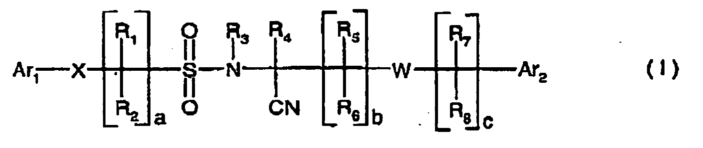 including the optical isomers thereof and mixtures of such isomers, wherein
Ar₁ and Ar₂ independently of each other stand for an optionally substituted aryl
R₁ and R₂ stand independently of each other for hydrogen, optionally substituted C₁-C₅alkyl, optionally substituted C₂-C₅alkenyl, C₂-C₆alkynyl
R₃ designates hydrogen, C₃-C₅alkenyl, C₃-C₅alkynyl or optionally substituted C₁-C₅alkyl;
R₄ is optionally substituted C₁-C₅alkyl, optionally substituted C₂-C₅alkenyl, C₂-C₅alkynyl;
R₅ and R₆ are independently of each other hydrogen or optionally substituted C₁-C₅alkyl, optionally substituted C₂-C₅alkenyl, C₂-C₅alkynyl; R₇ and R₈ are independently of each other hydrogen or optionally substituted C₁-C₅alkyl,
W designates a bridge selected from -O-,
X designates a direct bond
a and b independently of each other stand for a number 1, 2 or 3; and
c is zero.

More specifically the present invention refers to the α-sulfonylamino-acetonitrile derivatives of formula I wherein
Ar₁ stands for an aryl group which is optionally substituted with n radicals independently selected from R₉
Ar₂ stands for an aryl group which is optionally substituted with n radicals independently selected from R'₉ and from one radical R₁₀; or
R₁ and R₂ stand independently of each other for hydrogen or C₁-C₅alkyl optionally substituted by halogen, C₁-C₅alkoxy or -NR₁₂R₁₃; or
stand for C₂-C₅alkenyl optionally substituted by halogen or C₁-C₃alkoxy; or
stand for C₂-C₅alkynyl;
R₃ designates hydrogen, C₃-C₅alkenyl, C₃-C₅alkynyl or C₁-C₃alkyl optionally substituted by C₁-C₃alkoxy; C₃-C₅alkenyloxy or C₃-C₅alkynyloxy;
R₄ is C₁-C₅-alkyl optionally substituted by halogen, C₁-C₃alkoxy or -NR₁₂R₁₃; or
is C₂-C₅alkenyl optionally substituted by halogen or C₁-C₃alkoxy; or
is C₂-C₅alkynyl;
R₅ and R₆ are independently of each other hydrogen or C₁-C₅alkyl optionally substituted by halogen, C₁-C₅alkoxy or -NR₁₂R₁₃; or
are C₂-C₅alkenyl optionally substituted by halogen or C₁-C₃alkoxy; or
are C₂-C₅alkynyl; or
R₇ and R₈ are independently of each other hydrogen or C₁-C₅alkyl optionally substituted by halogen, C₁-C₃alkoxy or -NR₁₂R₁₃;
R₈ and R'₉ independently of each other stand for C₁-C₆alkyl optionally substituted by halogen, C₁-C₄alkoxy, -NR₁₂R₁₃, -CO-R₁₄ or the acyclic or cyclic ketals and acetals of -CO-R₁₄ ; by a -X-aryl which is optionally substituted by halogen, C₁-C₄alkyl, C₁-C₄haloalkyl, C₁-C₄alkoxy, -CN, -NO₂, -NR₁₂R₁₉, -CO-R₁₄ or the acyclic or cyclic ketals and acetals of -CO-R₁₄ ; by a -X-linked-5- or 6-ring-membered heteroaryl group optionally substituted by halogen, C₁-C₄alkyl, C₁-C₄haloalkyl, C₁-C₄alkoxy, -CN, -NO₂, -NR₁₂R₁₃, -CO-R₁₄ or the acyclic or cyclic ketals and acetals of -CO-R₁₄; or
stand for C₃-C₆cycloalkyl, optionally substituted by halogen, hydroxy, =O, C₁-C₄alkoxy, NR₁₂R₁₃; or
stand for C₁-C₄alkoxy optionally substituted by halogen, C₁-C₄alkoxy, by -X-aryl which is optionally substituted by halogen, C₁-C₄alkyl, C₁-C₄haloalkyl, C₁-C₄alkoxy, -CN, -NO₂
-NR₁₂R₁₃, -CO-R₁₄ or the acyclic or cyclic ketals and acetals of -CO-R₁₄; by a X-linked-5- or 6-ring-membered heteroaryl group optionally substituted by halogen, C₁-C₄alkyl,
C₁-C₄haloalkyl, C₁-C₄alkoxy, -CN, -NO₂, -NR₁₂R₁₃, -CO-R₁₄ or the acyclic or cyclic ketals and acetals of -CO-R₁₄; or
stand for C₂-C₅alkenyl optionally substituted by halogen or aryl; or
stand for C₂-C₅alkynyl optionally substituted by halogen, tri-alkyl-silyl or aryl; or
stand for C₂-C₅alkenyloxy optionally substituted by halogen or aryl; or
stand for C₂-C₅alkynyloxy optionally substituted by halogen, tri-alkyl-silyl or aryl; or
stand for C₃-C₆cycloalkoxy optionally substituted by C₁-C₃alkyl, halogen or C₁-C₄alkoxy; or stand for halogen; or
stand for -NR₁₂R₁₃ or
stand for-NR₂-CO-R₁₂ ; or
stand for -NR₂-CO-OR₁₂ ; or
stand for -NR₂-CO-NR₈R₉ ; or
stand for -NR₂-CO-SR₁₂ ; or
stand for -NR₂-CS-OR₁₂ ; or
stand for -NR₂-CS-NR₈R₉ ; or
stand for -NR₂-CS-SR₁₂ ; or
stand for =NR₂-C(=N-O-R₁₂)-S-OR₁₂ ; or
stand for -NR₂-C(=N-O-R₁₂)-NR₈R₉ ; or
stand for -NR₂-C(=N-O-R₁₂)-SR₁₂ ; or
stand for -S(O)_{P}-C₁-C₄alkyl optionally substituted by halogen; or
stand for -NR₂-SO₂-NR₈F₁₉; or
stand for nitro , for cyano or for -CS-NH₂;
R₁₀ stands for hydrogen; or
stands for -X-aryl which is optionally substituted by halogen, C₁-C₄alkyl, C₁-C₄haloalkyl, C₁-C₄alkoxy, -CN, -NO₂ , -NR₁₂R₁₃, -CO-R₁₄ or the acyclic or cyclic ketals and acetals of -CO-R₁₄; or
stands for a X-linked 5-membered aromatic or non-aromatic heterocyclic ring comprising nitrogen, oxygen or sulfur as ring members and being optionally substituted by halogen, C₁-C₄alkyl, C₁-C₄haloalkyl, C₁-C₄alkoxy, -CN, -NO₂, =NR₁₂R₁₃, -CO-R₁₄ or the acyclic or cyclic ketals and acetals of -CO-R₁₄ ; or
stands for a X-linked 6-membered aromatic or non-aromatic heterocyclic ring comprising nitrogen, oxygen or sulfur as ring members and being optionally substituted by halogen, C₁-C₄alkyl, C₁-C₄haloalkyl, C₁-C₄alkoxy, -CN, -NO₂, -NR₁₂R₁₃, -CO-R₁₄ or the cyclic or cyclic ketals and acetals of -CO-R₁₄ ; or
stands for -CO-R₁₄ or the acyclic or cyclic ketals and acetals of -CO-R₁₄ ; or
stands for =O-CO-R₁₄ ; or
stands for -C(=N-O-R₁₂)-R₁₄ ; ;R₁₀ and one R'₉ together form a 3- or 4-membered non-aromatic bridge forming an annellated ring which may contain a carbonyl function or nitrogen, oxygen or sulfur as ring members and is optionally substituted by C₁-C₃alkyl;
R₁₁ is hydrogen, halogen, C₁-C₄alkyl, C₁-C₄haloalkyl, C₁-C₄alkoxy, -NR₁₂R₁₃, -NO₂, -CN, -CO-R₁₄ or the acyclic or cyclic ketals and acetals of -CO-R₁₄ ;
- W: designates a bridge selected from -O-, -S(O)ₘ- or -NR₃-;
- X: designates a direct bond or a bridge selected from -O-, -S(O)ₘ- or -NR₃- ;
- a: stands for a number 1, 2 or 3;
- b: stands for a number 1, 2 or 3;
- c: stands for a number zero, 1 or 2;
- m: stands for a number zero, 1 or 2;
- n: stands for a number 1 or 2;
- p: stands for a number zero, 1 or 2;
R₁₂ and R₁₃ independently of each other stand for hydrogen; C₁-C₅alkyl optionally substituted by halogen, C₁-C₄haloalkyl, C₁-C₄alkoxy, C₁-C₄alkylamino, di(C₁-C₄alkyl)amino_{;} or aryl which in turn is optionally substituted by halogen, C₁-C₄alkyl, C₁-C₄haloalkyl, C₁-C₄alkoxy or -CN ; or
stand for C₃-C₅alkenyl optionally substituted by halogen, C₁-C₄haloalkyl, C₁-C₄alkoxy, C₁-C₄alkylamino, di(C₁-C₄alkyl)amino, or aryl which in turn is optionally substituted by halogen, C₁-C₄alkyl, C₁-C₄haloalkyl, C₁-C₄alkoxy or -CN; or
stand for C₃-C₅alkynyl optionally substituted by halogen, C₁-C₄haloalkyl, C₁-C₄alkoxy, C₁-C₄alkylamino, di(C₁-C₄alkyl)amino, or aryl which in turn is optionally substituted by halogen, C₁-C₄alkyl, C₁-C₄haloalkyl, C₁-C₄alkoxy or -CN; or
together form a 5-ring-membered non-aromatic carbocyclic ring; or together form a 6-ring-membered non-aromatic carbocyclic ring which is interrupted by -O- or -N(C₁-C₄alkyl)-; and
R₁₄ stands for C₁-C₅alkyl optionally substituted by halogen, C₁-C₄alkoxy, C₁-C₄alkylamino, di(C₁-C₄alkyl)amino; aryl which in turn is optionally substituted by halogen, C₁-C₄alkyl, C₁-C₄haloalkyl, C₁-C₄alkoxy, -CN, -NO₂ C₁-C₄alkylamino, di(C₁-C₄alkyl)amino or C₁-C₄alkylcarbonyl, C₁-C₄alkoxycarbonyl, C₁-C₄alkylaminocarbonyl or di(C₁-C₄alkyl)aminocarbonyl; or by a 5- or 6-ring hetero-aromatic ring which in turn is optionally substituted by halogen, C₁-C₄alkyl, C₁-C₄haloalkyl C₁-C₄alkoxy, -CN, -NO₂, C₁-C₄alkylamino, di(C₁-C₄alkyl)amino, C₁-C₄alkylcarbonyl, C₁-C₄alkoxycarbonyl, C₁-C₄alkylaminocarbonyl or di-(C₁-C₄alkyl)aminocarbonyl; or
stands for C₃-C₆cycloalkyl optionally substituted by halogen, hydroxy, =O, C₁-C₄alkoxy or C₁-C₄alkylamino, di(C₁-C₄alkyl)amino; or
stands for C₁-C₄alkoxy optionally substituted by halogen, C₁-C₄alkoxy; C₁-C₄alkylamino, di(C₁-C₄alkyl)amino; or
stands for phenyl which is optionally substituted by halogen, C₁-C₄alkyl, C₁-C₄haloalkyl, C₁-C₄alkoxy. -CN, -NO₂, C₁-C₄alkylamino, di(C₁-C₄alkyl)amino, C₁-C₄alkylcarbonyl, C₁-C₄alkoxycarbonyl, C₁-C₄alkylaminocarbonyl or di-(C₁-C₄alkyl)aminocarbonyl; or
stands for a 5- or 6-ring membered heteroaryl comprising nitrogen, oxygen or sulfur as ring members and being optionally substituted by halogen, C₁-C₄alkyl, C₁-C₄haloalkyl;
C₁-C₄alkoxy, -CN, -NO₂, C₁-C₄alkylamino, di(C₁-C₄alkyl)amino, C₁-C₄alkylcarbonyl, C₁-C₄alkoxycarbonyl, C₁-C₄alkylaminocarbonyl or dl-(C₁-C₄alkyl)aminocarbonyl.

In the above definitions "halo" or "halogen" includes fluorine, chlorine, bromine and iodine. The alkyl, alkenyl and alkynyl radicals may be straight-chain or branched. This applies also to the alkyl, alkenyl or alkynyl parts of other alkyl-, alkenyl- or alkynyl-containing groups, such as alkoxy, alkylthio, alkylamino and dialkylamino.
Depending upon the number of carbon atoms mentioned, alkyl on its own or as part of another substituent is to be understood as being, for example, methyl, ethyl, propyl, butyl, pentyl, hexyl, heptyl, octyl, nonyl, decyl, undecyl, dodecyl and the isomers thereof, for example isopropyl, isobutyl, tert-butyl or sec-butyl, isopentyl or tert-pentyl.
Cycloalkyl for example is, depending upon the number of carbon atoms mentioned, cyclopropyl, cyclobutyl, cyclopentyl, cyclohexyl, bicyclohexyl, cycloheptyl, bicycloheptyl, cyclooctyl or bicyclooctyl.

Depending upon the number of carbon atoms mentioned, alkanyl as a group or as a structural element of other groups is to be understood as being, for example, ethenyl, allyl, 1-propenyl, buten-2-yl, buten-3-yl, penten-1-yl, penten-3-yl, hexen-1-yl, 4-methyl-3-pentenyl or 4-methyl-3-hexenyl.

Alkynyl as a group or as a structural element of other groups is, for example, ethynyl, propyn-1-yl, propyn-2-yl, butyn-1-yl, butyn-2-yl, 7-methyl 2-butynyl, hexyn-1-yl, 1-ethyl-2-butynyl or octyn-1-yl, depending on the number of carbon atoms present.

A haloalkyl, haloalkenyl, haloalkynyl or halocycloalkyl group may contain one or more (identical or different) halogen atoms, and for example may stand for CHCl₂, CH₂F, CCl₃, CH₂Cl, CHF₂, CF₃, CH₂CH₂Br, C₂Cl₅, CH₂Br, CHClBr, CF₃CH₂, CH₂CH₂Cl, CH₂CH₂F, CH₂CHF₂, CH₂-C=CHCl, CH=CCl₂, CH=CF₂, CH₂C≡CCl, CH₂-C≡C-CF₃, chlorocyclohexyl, dichlorocyclohexyl, etc.

Alkoxy thus includes methoxy, ethoxy, propoxy, isopropoxy, n-butyloxy, s-butyloxy, i-butyloxy or t-butyloxy.

Ar₁ and Ar₂ according to the present invention both present aromatic moieties, belonging to the chemical class of aromatic hydrocarbons designated as aryl.

The definition aryl includes aromatic hydrocarbons ring systems like phenyl, naphthyl, anthracenyl, phenanthrenyl and biphenyl like 1,3-biphenyl and 1,4-biphenyl, with phenyl being preferred. The same definition applies where aryl is part of aryloxy.

Heteroaryl stands for monocyclic aromatic ring systems comprising 1 to 4 heteroatoms selected from N, O and S, where it is understood that the for reasons of complying with the aromatic character of the heteroaryl rings 1 to 4 nitrogen atoms may be present in one ring, but in general not more than one of them may be replaced by oxygen or sulfur. Where condensed ring systems of more than one ring is intended this is especially pointed out, for example by mentioning condensation, including annellation with benzene rings

Typical examples for 5-rings, 6-rings and bicyclic condensed systems are furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyridazinyl, pyrimidinyl, pyrazinyl, triazinyl, tetrazinyl, indolyl, benzothienyl, benzofuryl, isobenzothienyl, isobenzofuryl, benzimidazolyl, benzopyrazolyl, indazolyl, benzotriazolyl, benzothiazolyl, benzoisothiazolyl, benzoxazolyl, benzisoxazolyl, quinolinyl, isoquinolinyl, phthalazinyl, purinyl, naphthridinyl, pteridinyl, quinoxalinyl, quinazolinyl and cinnolinyl. Preferred heterocycles are furyl, thienyl, pyrrolyl, imidazolyl, thiazolyl, oxazolyl, isoxazolyl, oxadiazolyl, thiadiazolyl, triazolyl, pyridyl, pyridazinyl, pyrimidinyl, pyrazinyl, indolyl, benzothienyl, benzofuryl, benzopyrazolyl, benzothiazolyl, benzoxazolyl, benzisoxazolyl, quinolinyl, isoquinolinyl and quinazolinyl.

Depending on the position of the heteroaryl group, the heterocyclic ring may be linked to the basic molecular structure via a ring-carbon atom or via a nitrogen-ring atom.

The aryl and heteroaryl groups according to the invention may be unsubstituted or are optionally substituted. Where substituents are indicated according to this invention, the ring structures may carry one or more identical or different substituents. Normally not more than three substituents are present at the same time. Examples of substituents of aryl or heteroaryl groups are: alkyl, alkenyl, alkynyl, cycloalkyl, alkylamino, dialkylamino, cyano, nitro, amino, hydroxy, cycloalkyl-alkyl, aryl, arylalkyl, heteroaryl, heteroaryl-alkyl, phenyl and phenyl-alkyl, it being possible in turn for all of the preceding groups to carry one or more identical or different halogen atoms; alkoxy; alkenyloxy; alkynyloxy; alkoxyalkyl; haloalkoxy, alkylthio; haloalkylthio; alkylsulfonyl; formyl; alkanoyl; hydroxy; halogen; cyano; nitro; amino; hydroxy, alkylamino; dialkylamino; carboxyl; alkoxycarbonyl; alkenyloxycarbonyl; or alkynyloxycarbonyl.

Typical examples include 1-naphthyl, 2,3-dichlorophenyl, 2,3-difluorophenyl, 2,4,6-trichlorophenyl, 2,4,6-trifluorophenyl, 2,4-dichlorophenyl, 2,4-difluorophenyl, 2,5-dichlorophenyl, 2,5-difluorophenyl, 2,6-dichlorophenyl, 2-chloro-4-ethoxyphenyl, 2-chloro-4-methoxyphenyl, 2-chlorophenyl, 2-ethoxyphenyl, 2-fluoro-4-chlorophenyl, 2-fluoro-4-ethoxyphenyl, 2-fluoro-4-methoxyphenyl, 2-hexyloxyphenyl, 2-methoxy-4-chlorophenyl, 2-methoxyphenyl, 2-methyl-4-chlorophenyl, 2-naphthyl, 2-trifluoromethyl, 3,4,5-trichlorophenyl, 3,4-dibromophenyl, 3,4-dichlorophenyl, 3,4-difluorophenyl, 3,4-dimethoxyphenyl, 3,4-dimethylphenyl, 3,5-dimethyl-4-chlorophenyl, 3'4'-dichloro-4-biphenylyl, 3-bromo-4-methylphenyl, 3-bromophenyl, 3-chloro-4-cyanophenyl, 3-chloro-4-ethoxyphenyl, 3-chloro-4-fluorophenyl, 3-chloro-4-methoxyphenyl, 3-chlorophenyl, 3-ethyl-4-chlorophenyl, 3-fluoro-4-ethoxyphenyl, 3-fluoro-4-methoxyphenyl, 3-fluoro-4-methoxyphenyl, 3-methoxy-4-chlorophenyl, 3-methylphenyl, 4-(1,3,4-oxadiazol-2-yl)phenyl, 4-(1-imidazolyl)-phenyl, 4-(1-methyl-methoximinomethyl)-phenyl, 4-(2,6-dimethoxy-pyrimidin-2-ylthio)-phenyl, 4-(2-cyanopyrid-4-yl)-phenyl, 4-(3-methyl-1,2,4-thiadiazol-4-2-yloxy)phenyl, 4-(3-methyl-1,2,4-thiazol-5-yloxy)-phenyl, 4-(5-ethyl-1,3,4-oxadiazol-2-yl)phenyl, 4-(pyrid-2yloxy)-phenyl, 4'-bromo-4-biphenylyl, 4'-chloro-4-biphenylyl, 4'-cyano-4-biphenylyl, 4'-methyl-4-biphenylyl, 4'-trifluoromethyl-4-biphenylyl, 4-aminocarbonylethoxy-phenyl, 4-aminocarbonylmethyl-phenyl, 4-aminocarbonyl-phenyl, 4-biphenylyl, 4-bromo-3-chlorophenyl, 4-bromophenyl, 4-chloro-3-cyanophenyl, 4-chloro-3-fluorophenyl, 4-chloro-3-methylphenyl, 4-chloro-3-trifluoromethyl-phenyl, 4-chlorophenyl, 4-cyanophenyl, 4-cyclohexylphenyl, 4-ethenylphenyl, 4-ethoxyphenyl, 4-ethylphenyl, 4-ethynyloxyphenyl, 4-ethynylphenyl, 4-fluorophenyl, 4-hexyloxyphenyl, 4-isopropylcarbonylamino-phenyl, 4-isopropylphenyl, 4-isopropoxyphenyl, 4-methoxy-3-methylphenyl, 4-methoxycarbonyl-phenyl, 4-methoxyphenyl, 4-methylphenyl, 4-methylsulfonyl-phenyl, 4-methylthiophenyl, 4-nitrophenyl, 4-N-morpholinocarbonylaminophenyl, 4-N-morpholinocarbonyloxyethoxy-phenyl, 4-phenoxyphenyl, 4-propargyloxyphenyl, 4-propylphenyl, 4-tert.-butylcarbonylamino-phenyl, 4-tert.butylphenyl, 4-trifluoromethoxyphenyl, 4-trifluoromethylphenyl, 5-chloro-thion-2-yl, 5-methyl-fur-2-yl, 5-methyl-thien-2-yl, 6-benzothienyl, 7-benzothienyl, etc.

Where R₁₀ and R'₉ together form a bridge the bridge is normally between vicinal carbon atom of Ar₂. Thus annellated ring structures are formed, which may be substituted with one or two lower alkyl groups, preferably methyl. The bridge includes -(CH₂)₃-, -(CH₂)₄-, -O-(CH₂)₃-, -CO-(CH₂)₃-, -S-(CH₂)₃-, -NH-(CH₂)₃- -O-(CH₂)₂-, -O-(CH₂)₂-O-, -O-CH₂-CH(CH₃)-O-, -O-CH₂-O-, -CO-(CH₂)₂-, -S-(CH₂)₂-, -NH-(CH₂)₂-, -CH₂-O-CH₂-, -CH₂-CO-CH₂-, -CH₂-S-CH₂-, -CH₂-NH-CH₂- , -CO-O-(CH₂)₂-, -CO-NH-(CH₂)-, -NH-CO-(CH₂)₂-, -CH₂-CO-O-CH₂-, -CO-S-(CH₂)₂- , -NH-CO-CH₂-, -O-CO-(CH₂)₂- , -CH₂-CO-O- , -CH₂-O-CO- , -S-CO-(CH₂)₂- , -CO-NH-CH₂- and -CH₂-CO-NH-CH₂- , etc.. Where the acetals or ketals of -CO-R₁₄ are intended the acetals and ketals may appear as -C(C₁-C₄alkoxy)₂-R₁₄ or as cyclic structures wherein the former carbonyl carbon atom carries a dioxoalkylene bridge of the type -O-C₁-C₃alkylene-O- which optionally may be branched, including -O-CH₂-O- , -O-CH(CH₃)-O-, -O-(CH₂)₂-O- , -O-(CH₂)₃-O- , -O-CH₂-CH(CH₃)-O- , and the like.

Where R₁₂ and R₁₃ together with the nitrogen binding the two radicals may form a non-aromatic carbocyclic ring this radical stands for pyrrolidine, piperidine, morpholine or thiomorpholine ring, which may be substituted by one or two methyl groups.

The presence of at least one asymmetric carbon atom in the compounds of formula I means that the compounds may occur in optically isomeric , diastereomeric and enantiomeric forms. As a result of the presence of a possible aliphatic C=C double bond, geometric isomerism may also occur. Formula I is intended to include all those possible isomeric forms and mixtures thereof. Where no specific isomer is specified the mixtures of diastereomers , enantiomers or the racemate are meant, as obtainable from the disclosed synthesis methods. The optical isomers, diastereomers and enantiomers of formula I may be obtained in pure form either by isolation from the mixture by suitable separation methods, which are known in the art, or may be obtained by stereoselective synthesis methods.

Preferred subgroups of compounds of formula I are those wherein
Ar₁ stands for optionally substituted aryl group; or
Ar₁ is optionally substituted phenyl; or
Ar₂ stands for optionally substituted aryl; or
Ar₂ is optionally substituted phenyl; or
Ar₁ and Ar₂ independently of each other stand for optionally substituted phenyl; or the optional substituents R₉ of Ar₁ are preferably selected from the group comprising halogen, C₁-C₄alkyl, C₁-C₄haloalkyl, C₁-C₄alkoxy, C₁-C₄haloalkoxy, C₃-C₆cycloalkyl, -CN and -CO-R₁₄; or
the optional substituents R'₉ of Ar₂ are preferably selected from the group comprising halogen, C₁-C₄alkyl, C₁-C₄haloalkyl, C₁-C₄alkoxy, C₁-C₄haloalkoxy, C₃-C₆cycloalkyl, -CN, -CO-R₁₄ , -NR₁₂R₁₃, -NR₂-CO-R₁₂, -NR₃-CO-OR₁₂ , -NR₂-CO-NR₈R₉, -NR₂-CO=SR₁₂ ; -NR₂-CS-OR₁₂, -NR₂-CS-NR₈R₉, -NR₂=CS=SR₁₂, -S(O)ₚ-C₁-C₄alkyl, -S(O)ₚ-C₁-C₄haloalkyl , -NR₂-SO₂-NR₈R_{9.} nitro, cyano and -CS-NH₂; or
the optional substituents R₉ and R'₉ of Ar₁ and Ar₂ are selected from the group comprising C₁-C₄alkyl, C₁-C₄haloalkyl, C₁-C₄alkoxy, C₁-C₄haloalkoxy and C₆-C₆cycloalkyl; or the optional substituents R₉ and R'₉ of Ar₁ and Ar₂ are selected from the group comprising bromo, chloro, fluoro, iodo, cyano, hydroxy, amino, nitro, methyl, ethyl, propyl, isopropyl, methoxy, ethoxy, propoxy, isopropoxy, allyloxy, propargyloxy, benzyloxy, trifluoromethyl, trifluoromethoxy, 2-cyano-2-methyl-butyloxy, methylsulfonyl, methylsulfinyl, methylthio, cyclopentyl, cyclohexyl,- aminocarbonylmethyl, methoximinoethyl, aminocarbonyl, butylcarbonylamino, tert-butylcarbonylamino, triazol-1-ylmethyl, 1,2,4-triazol-1-ylmethyl, N-morpholinocarbonylamino, aminocarbonyloxy-ethoxy, morpholinocarbonyloxyethoxy and cyanopyridyloxyethoxy; or
the optional substituents R₉ and R'₉ of Ar₁ and Ar₂ are selected from the group comprising bromo, chloro, fluoro, methyl, ethyl, methoxy, ethoxy, trifluoromethyl and trifluoromethoxy; or
the optional substituent R₁₀ on Ar₂ is selected from optionally substituted phenyl, optionally substituted imidazolyl, optionally substituted thiazolyloxy, optionally substituted pyridyloxy, optionally substituted pyridyl, optionally substituted pyrimidinyloxy, optionally substituted pyrimidinyl, optionally substituted oxadiazolyl, optionally substituted triazolyl, optionally substituted pyrazolyl, optionally substituted oxadiazolyloxy, optionally substituted triazolyloxy and optionally substituted pyrazolyloxy; or
the optional substituent R₁₀ on Ar₂ is selected from halogen, C₁-C₄alkyl, C₁-C₄haloalkyl_{;} C₁-C₄alkoxy, -CN, -NO₂ ; -NR₁₂R₁₃, -CO-R₁₄ and the acyclic or cyclic ketals and acetals of -CO-R₁₄; or
the optional substituent R₁₀ on Ar₂ is selected from -CO-R₁₄, -O-CO-R₁₄, optionally substituted phenyl, optionally substituted phenoxy, optionally substituted imidazolyl, optionally substituted imidazolyloxy, optionally substituted thiazolyloxy, optionally substituted thiazolyl, optionally substituted thiadiazolyloxy, optionally substituted thiadiazolyl, optionally substituted pyridyloxy, optionally substituted pyridyl, optionally substituted pyrimidinyloxy, optionally substituted pyrimidinyl, optionally substituted oxadiazolyl, optionally substituted oxadiazolyloxy, optionally substituted triazolyl, optionally substituted pyrazolyl, optionally substituted triazolyloxy and optionally substituted pyrazolyloxy; or
the optional substituent R₁₀ on Ar₂ is selected from -CO-C₁-C₄alkyl, -O-CO-C₁-C₄alkyl and -CO-C₁-C₄alkoxy; or
the optional substituent R₁₀ on Ar₂ is selected from aminocarbonyl, dimethylaminocarbonyl, acetyl, propionyl, acetoxy, methoxycarbonyl, ethoxycarbonyl; benzoyl, methoximinoethyl, 1-imidazolyl, 5-(3-methyl-1,2,4-thiadiazolyloxy), 2-pyridyl, 2-pyridyloxy, 4-pyrimidinyl, 2-(3,5-dichloropyridyloxy), 2-(4,6-dichloropyridyloxy), 2-(4,6-dimethoxypyrimidinylthio), 2-oxadiazolyl, 2-(5-methyl-oxadiazolyl), 2-(5-ethyl-oxadiazolyl), 1-triazolyl, 1-pyrazolyl; 1-(3,4-dimethylpyrazolyl), 4-(2-methylthiazolyl), 2-(1,3,4-oxydialolyl), N-pyrrolidin-2-onyl, and 2-quinoxalinyl, or
R₁, R₂, R₅, R₆, R₇ and R₈ independently of each other stand for hydrogen or methyl; or
R₁ and R₅ are independently of each other C₁-C₄alkyl and R₂ and R₈ are hydrogen; or
R₃ is hydrogen or C₁-C₄alkyl optionally substituted with C₁-C₄alkoxy, C₃-C₄alkenyloxy, or C₃-C₄alkynyloxy; or
R₃ is hydrogen, C₁-C₄alkyl or C₁-C₄alkoxy-C₁-C4alkyl; or
R₄ is hydrogen or C₁-C₄alkyl optionally substituted with halogen. C₁-C₃alkoxy
C₁-C₃alkylamino or di-C₁-C₃alkylamino; or
R₄ is hydrogen, C₁-C₄alkyl or C₁-C₄haloalkyl or
R₄ is C₁-C₄alkyl or C₁-C₄haloalkyl; or
W is -O- ; or
X is a direct bond; or
the suffixes (a) and (b) designate the number 1 ; or
the suffix (c) stands for the number zero.

One preferred subgroup of formula I is wherein Ar₁ and Ar₂ independently of each other stand for optionally substituted phenyl; and the optional substituents R₉ of Ar₁ are preferable selected from the group comprising halogen, C₁-C₄alkyl, C₁-C₄haloalkyl, C₁-C₄alkoxy, C₁-C₄haloalkoxy, C₃-C₆cycloalkyl, -CN and -CO-R₁₄; and the optional substituents R'₉ of Ar₂ are preferably selected from the group comprising halogen, C₁-C₄alkyl, C₁-C₄haloalkyl, C₁-C₄alkoxy, C₁-C₄haloalkoxy, C₃-C₆cycloalkyl, -CN, -CO-R₁₄, -NR₁₂R₁₃, -NR₂-CO-R₁₂, -NR₃-CO-OR₁₂ , -NR₂-CO-NR₈R₉, -NR₂-CO-SR₁₂, =NR₂-CS-OR₁₂ , -NR₂-CS-NR₈R₉, -NR₂-CS-SR_{12;} -S(O)ₚ-C₁-C₄alkyl, -S(O)ₚ-C₁-C₄haloalkyl , -NR₂-SO₂-NR₈R₉, nitro, cyano and -CS-NH₂; and the optional substituent R₁₀ on Ar₂ is selected from optionally substituted phenyl, optionally substituted imidazolyl, optionally substituted thiazolyloxy, Optionally substituted pyridyloxy, optionally substituted pyridyl, optionally substituted pyrimidinyloxy, optionally substituted pyrimidinyl, optionally substituted oxadiazolyl, optionally substituted triazolyl, optionally substituted pyrazolyl; optionally substituted oxadiazolyloxy, optionally substituted triazolyloxy and optionally substituted pyrazolyloxy.

Further preferred subgroups are those wherein
A) Ar₁, and Ar₂ independently stand for optionally substituted aryl groups; and the optional substituents R₉ of Ar₁ are preferably selected from the group comprising halogen, C₁-C₄alkyl, C₁-C₄haloalkyl, C₁-C₄alkoxy, C₁-C₄haloalkoxy, C₃-C₆cycloalkyl, -CN and -CO-R₁₄; and
   the optional substituents R'₉ of Ar₂ are preferably selected from the group comprising halogen, C₁-C₄alkyl, C₁-C₄haloalkyl, C₁-C₄alkoxy, C₁-C₄haloalkoxy, C₃-C₆cycloalkyl, -CN, -CO-R₁₄, -NR₁₂R₁₃, -NR₂-CO-R₁₂, -NR₃-CO-OR₁₂, -NR₂-CO-NR₈R₉, -NR₂-CO-SR₁₂, -NR₂-CS-OR_{12,} -NA₂-CS-NR₈R₉, -NR₂-CS-SR₁₂, -S(O)ₚ-C₁-C₄alkyl, -S(O)ₚ-C₁-C₄haloalkyl, -NR₂-SO₂-NR₈R₉, nitro , cyano and -CS-NH₂; and
   the optional substituent R₁₀ on Ar₂ is selected from halogen, C₁-C₄alkyl, C₁-C₄haloalkyl, C₁-C₄alkoxy, -CN, -NO₂ , -NR₁₂R₁₃, -CO-R₁₄ and the acyclic or cyclic ketals and acetals of -CO-R₁₄ ; -O-CO-R₁₄, optionally substituted phenyl, optionally substituted imidazolyl, optionally substituted thiazolyloxy, optionally substituted pyridyloxy, optionally substituted pyridyl, optionally substituted pyrimidinyloxy, optionally substituted pyrimidinyl, optionally substituted oxadiazolyl, optionally substituted triazolyl, optionally substituted pyrazolyl, optionally substituted oxadiazolyloxy, optionally substituted triazolyloxy and optionally substituted pyrazolyloxy; and
   R₁, R₂, R₅, R₆, R₇ and R₈ independently of each other are hydrogen or methyl; and
   R₃ is hydrogen or C₁-C₄alkyl optionally substituted with C₁-C₄alkoxy, C₃-C₄alkenyloxy, or C₃-C₄alkynyloxy; and
   R₄ is hydrogen or C₁-C₄alkyl optionally substituted with halogen, C₁-C₃alkoxy, C₁-C₃alkylamino or di-C₁-C₃alkylamino; and
   W is -O-; and
   X is a direct bond; and
   the suffixes (a) and (b) designate the number 1 ; and
   the suffix (c) stands for the number zero; or wherein
B) Ar₁ and Ar₂ independently of each other stand for optionally substituted phenyl; and the optional substituents R₉ and R'₉ of Ar₁ and Ar₂ are selected from the group comprising C₁-C₄alkyl, C₁-C₄haloalkyl, C₁-C₄alkoxy, C₁-C₄haloalkoxy and C₃-C₆cycloalkyl; and the optional substituent R₁₀ on Ar₂ is selected from -CO-C₁-C₄alkyl, -CO-C₁-C₄alkoxy, -O-CO-C₁-C₄alkyl, optionally substituted phenyl, optionally substituted phenoxy, optionally substituted imidazolyl, optionally substituted imidazolyloxy, optionally substituted thiazolyloxy, optionally substituted thiazolyl, optionally substituted thiadiazolyloxy, optionally substituted thiadiazolyl, optionally substituted pyridyloxy, optionally substituted pyridyl, optionally substituted pyrimidinyloxy, optionally substituted pyrimidinyl, optionally substituted oxadiazolyl, optionally substituted oxadiazolyloxy, optionally substituted triazolyl, optionally substituted pyrazolyl, optionally substituted triazolyloxy and optionally substituted pyrazolyloxy; and
   R₁ and R₅ are independently C₁-C₄alkyl and R₂ and R₈ are hydrogen; and
   R₃ is hydrogen, C₁-C₄alkyl or C₁-C₄alkoxy-C₁-C₄alkyl; and
   R₄ is C₁-C₄alkyl or C₁-C₄haloalkyl; and
   W is -O- ; and
   X is a direct bond; and
   the suffixes (a) and (b) designate the number 1 ; and
   the suffix (c) stands for the number zero; or wherein
C) Ar₁ and Ar₂ independently of each other stand for optionally substituted phenyl; and the optional substituents R₉ and R'₉ of Ar₁ and Ar₂ are selected from the group comprising bromo, chloro, fluoro, iodo, cyano, hydroxy, amino, nitro, methyl, ethyl, propyl, isopropyl, methoxy, ethoxy, propoxy, isopropoxy, allyloxy, propargyloxy, benzyloxy, trifluoromethyl, trifluoromethoxy, 2-cyano-2-methyl-butyloxy, methylsulfonyl, methylsulfinyl, methylthio, cyclopentyl, cyclohexyl, aminocarbonytmethyl, methoximinoethyl, aminocarbonyl, butylcarbonylamino, tert-butylcarbonylamino, triazol-1-ylmethyl, 1,2,4-1triazol-1-ylmethyl, N-morpholinocarbonylamino, aminocarbonyloxy-ethoxy, morpholinocarbonyloxyethoxy and cyanopyridyloxyethoxy; and
   the optional substituent R₁₀ on Ar₂ is selected from aminocarbonyl, dimethylaminocarbonyl, acetyl, propionyl, acetoxy, methoxycarbonyl, ethoxycarbonyl, benzoyl, methoximinoethyl, 1-imidazolyl, 5-(3-methyl-1,2,4-thiadiazolyloxy), 2-pyridyl, 2-pyridyloxy, 4-pyrimidinyl, 2-(3,5-dichloropyridyloxy), 2-(4,6-dichloropyridyloxy), 2-(4,6-dimethoXypyrimidinylthio); 2-oxadiazolyl, 2-(5-methyl-oxadazolyl), 2-(5-ethyl-oxadiazolyl), 1-triazolyl, 1-pyrazolyl, 1-(3,4-dimethylpyrazolyl), 4-(2-methylthiazolyl), 2-(1,3,4-oxydiazolyl), N-pyrrolidin-2-onyl, and 2-quinoxalinyl, and
   R₁ and R₅ are independently C₁-C₄alkyl and R₂ and R₆ are hydrogen; and
   R₃ is hydrogen, C₁-C₄alkyl or C₁-C₄alkoxy-C₁-C₄alkyl; and
   R₄ is C₁-C₄alkyl or C₁-C₄haloalkyl; and
   W is-O-; and
   X is a direct bond; and
   the suffixes (a) and (b) designate the number 1 ; and
   the suffix (c) stands for the number zero; or wherein
D) An and Ar₂ independently of each other stand for optionally substituted phenyl; and the optional substituents R₉ and R'₉ of Ar₁ and Ar₂ are selected from the group comprising bromo, chloro, fluoro, methyl, ethyl, methoxy, ethoxy, trifluoromethyl arid trifluoromethoxy; and
   the optional substituent R₁₀ on Ar₂ is selected from aminocarbonyl, acetyl, methoxycarbonyl, ethoxycarbonyl, 1-imidazolyl, 5-(3-methyl-1,2,4-thiadiazolyloxy), 2-pyridyl, 2-pyridyloxy, 4-pyrimidinyl, 2-(3,5-dichloropyridyloxy), 2-(4,6-dimethoxypyrimidinylthio), 2-oxadiazolyl, 2-(5-methyl-oxadazolyl), 2-(5-ethyl-oxadiazolyl), 1-triazolyl, 1-pyrazolyl,
   4-(2-methylthiazolyl), 2-(1,3,4-oxydiazolyl), and N-pyrrolidin-2-onyl, and
   R₁ and R₅ are methyl and R₂ and R₆ are hydrogen; and
   R₃ is hydrogen , methyl , ethyl, propyl, ethoxymethyl or methoxymethyl, and
   R₄ is methyl , ethyl, propyl or fluoromethyl; and
   W is -O-; and
   X is a direct bond; and
   the suffixes (a) and (b) designate the number 1 ; and
   the suffix (c) stands for the number zero.

Preferred individual compounds are:
2-[(4-chlorophenoxy)-methyl]-2-benzylsulfonylamino-propionitrile,
2-[(4-chlorophenoxy)-methyl]-2-[(2-chlorophenyl)-methyl]-sulfonylamino-propionitrile,
2-[(4-chlorophenoxy)-methyl]-2-[(2-fluorophenyl)-methyl]-sulfonylamino-propionitrile,
2-[(4-trifluoromethoxyphenoxy)-methyl]-2-benzylsulfonylamino-propionitrile,
2-[(4-chloro-3-methylphenoxy)-methyl]-2-benzylsulfonylamino-propionitrile,
2-[(4-chlorophenoxy)-methyl]-2-benzylsulfonylamino-butryronitrile,
2-[(4-chlorophehoxy)-methyl]-2-benzylsulfonylamino-3-methoxy-propionitrile,
2-[(4-acetylphenoxy)-methyl]-2-benzylsulfonylamino-propionitrile,
2-[(4-methoxyphenoxy)-methyl]-2-benzylsulfonylamino-propionitrile,
2-[(4-acetylphenoxy)-methyl]-2-benzylsulfonylamino-butyronitrile,
2-[(4-cyanophenoxy)-methyl]-2-benzylsulfonylamino-propionitrile,
(-)-2-[(4-cyanophenoxy)-methyl]-2-benzylsulfonylamino-propionitrile,
2-[(4-propionylphenoxy)-methyl]-2-benzylsulfonylamino-propionitrite,
2-[(4-ethoxyphenoxy)-methyl]-2-benzylsulfonylamino-propionitrile,
2-[(4-[1,2,4]triazol-1-yl-phenoxy)-methyl]-2-benzylsulfonylamino-propionitrile;
2-[(4-imidazol-yl-phohoxy)-methyl]-2-benzylsulfonylamino-propionitrile,
2-[(4-cyanophenoxy)-methyl]-2-benzylsulfonylamino-butyronitrile,
2-[(4-[1,3,4]oxadiazol-4-yl-phenoxy)-methyl]-2-benzylsulfonylamino-propionitrile,
2-[(4-methoxyphenoxy)-methyl]-2-benzylsulfonylamino-butyronitrile,
2-[(4-ethoxyphenoxy)-methyl]-2-benzylsulfonylamino-butyronitrile,
(-)2-[(4-ethoxyphenoxy)-methyl]-2-benzylsulfohylamino-butyronitrile,
2-[(4-[1,2,4]triazol-1-yl-phenoxy)-methyl]-2-benzylsulfonylamino-butyronitrile_{;}
2-[(4=methoxycarbonylphenoxy)-methyl]-2-benzylsultonylamino-propionitrile,
2-[(4-propionylphenoxy)-methyl]-2-benzylsulfonylamino-butyronitrile,
2-[(4-chlorophenoxy)-methyl]-2-benzylsulfonylamino-3-fluoro-propionitrile,
2-{[4-(2-methyl-thiazol-4-yl)-phenoxy]-methyl}-2-benzylsulfonylamino-butyronitrile,
2-[(4-pyrazol-1-yl-phenoxy)-methyl]-2-benzylsulfonylamino-butyronitrile,
2-(5-oxo-5,6,7,8-tetrahydronaphth-2-yloxy)-methyl]-2-benzylsulfonylamino-butyronitrile,
2-[(4-chloro-phenoxy)-methyl]-2-benzylsulfonylamino-3-methyl-butyronitrile,
2-[(4-iso-propyl-phenoxy)-methyl]-2-benzylsulfonylamino-butyronitrile,
2-[(4-nitro-phenoxy)-methyl]-2-benzylsulfonylamino-butyronitrile,
2-[(4-cyano-phenoxy)-methyl]-2-benzylsulfonylamino-butyronitrile,
2-[(3-fluoro-4-propionyl-phenoxy)-methyl]-2-benzylsulfonylamino-propionitrile,
(-) -2-[(4-[1,2,4]triazol-1-yl-phenoxy)-methyl]-2-benzylsulfonylamino-butyronitrile, and
(-)-2-[(4-acetylphenoxy)-methyl]-2-benzylsulfonylamino-propionitrile.

Certain sulfonylamido-acetonitriles have been proposed for controlling plant-destructive fungi (for example in EP-A-176327 and EP-A-587110). The biological activity of those compounds is not, however, satisfactory in all aspects and for all needs of the agricultural practices in protecting crop plants.
Surprisingly, with developing the compounds of formula Ia new type of microbiocides has been provided which satisfies to a greater extend the need for an agent for controlling phytopathogenic microorganisms on crop plants having a high level of activity, paired with long lasting effective protection.

The compounds of formula I and the respective starting materials may be obtained according to the processes of Schemes 1 to 7. 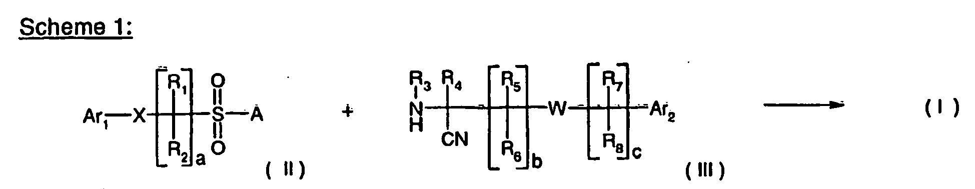 wherein Ar₁, Ar₂, a, b, c, W and R₁, to R₈, are defined as under formula I, and A stands for a leaving group like an anhydride, i.e. -O-SO₂-(CR₁R₂)ₐ-X-Ar₁ or-O-CO-C₁-C₄alkyl, but preferably for halogen, especially bromine or more preferably chlorine.

The compounds of formula I may be prepared by sulfonylation of an amino-acetonitrile of formula III with a sulfonyl-halide / anhydride of formula II wherein A is a leaving group, Suitable solvents for this reaction include ketones, such as acetone and methylethylketone, halogenated hydrocarbons such as chloroform, carbontetrachloride, dichloromethane, dichloro-ethane, aromatic hydrocarbons such as toluene or xylene, ethers such as t-butyl-methyl-ether, di-ethyl-ether, tetrahydrofuran and dioxane. The reaction is performed preferentially in the presence of a base and a catalyst. Typical bases include tertiary amines such as trimethylamine, triethylamine, diisopropylethylamine, dimethyl-aniline, diazabi-cyclooctane and N-methylmorpholine, aromatic amines such pyridine and quinoline as well as inorganic bases such as alkaline bicarbonates or -carbonates. Typical salts are for example sodium and potassium bicarbonate and sodium, potassium or cesium carbonate. Suitable catalysts such as N,N-dialkyl- or cyloalkyl-aminopyridines, like e.g. 4-N,N-dime-thylaminopyridine, may improve the yield.

The substituents R₃ may be introduced into the final active ingredients when starting from the subgroup compounds of formula I wherein R₃ is hydrogen, by reacting them e.g. with an alkylating agent R₃-A' wherein A' designates a leaving group, preferably a halogen atom, e.g. bromo or chloro. Suitable alkylating agents thus include suitably substituted alkylhalides or alkyl-O-sulfonates, e.g. or alkoxy-alkylhalides. On the other hand, when introducing R₃ with the starting compounds of formula III, alkylating of the compounds of the subgroup of formula III, wherein R₃ is hydrogen, may be achieved in a similar way by any commonly known alkylation method. Such alkylation prior to sulfonylation with a compound of formula 11, as alternative to converting R₃ within the final products of formula 1, allows to introduce a wide variety of radicals R₃ while leaving the choice to decide at which stage such optional conversion is preferably performed.

Cyano-amines of formula III may easily be prepared by the so-called Strecker -Synthesis according to Scheme 2 as described e.g. generically in any textbook on organic chemistry, or in a procedures disclosed in the patent literature (EP-A-953565-A; Nihon Noyaku or US 3,529,019, Colgate-Palmolive) starting from the corresponding ketone of formula IV. 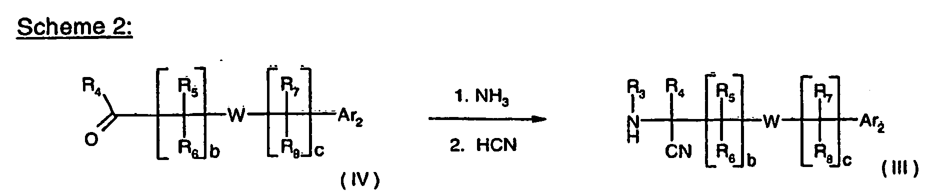 wherein Ar₂, b, c, W and R₅ to R₈ are defined as under formula I and L is a leaving group. The reaction conditions correspond to the standard conditions for preparing amino-acetonitriles by treatment with ammonia and prussic acid.

For the preparation of the ketones of formula IV various methods are known from the literature. Preferably the synthesis is for example conducted in accordance with Scheme 3 by starting from the ketone of formula V, wherein R₄, R₅, R₆ and b are defined as for formula I and L is a leaving group such as e.g. halogen, preferably chlorine, bromine or iodine or a sulfonyloxy group such as e.g. methylsulfonyloxy-, toluylsulfonyloxy- or trifluoromethylsulfonyloxy- group, and reacting it with a compound of formula VI wherein Ar₂, R₇, R₈ and c are defined as for formula I and W' is either an anionic radical species of W such as O⁻, S⁻, S(O)ₘ⁻ combined with an alkaline- or earthalkaline - metal cation as counterion, or is defined as W-H , e.g. as OH, SH, NHR₃. In the latter case the reaction is generally carried out in the presence of a base such as alkaline-, earthalkaline-carbonates or hydrogencarbonates, e.g. sodium or potassium-carbonate, sodium or potassium -hydrogen-carbonate, cesium-carbonate or an agent capable of scavenging the formed acid. 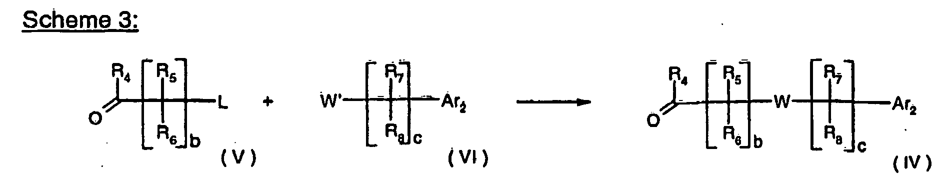 wherein Ar₂, b, c, W and R₄ to R₈, are defined as under formula I and W' is either an anionic radical species of W such as O⁻, S⁻, S(O)ₘ combined with an alkaline- or earthalkaline - metal cation as counterion, or is defined as W-H, e.g. as OH, SH, NHR₃.

As a typical alternative method of preparing the intermediate ketones of formula (IV) Scheme 3A highlights two of the various pathways. 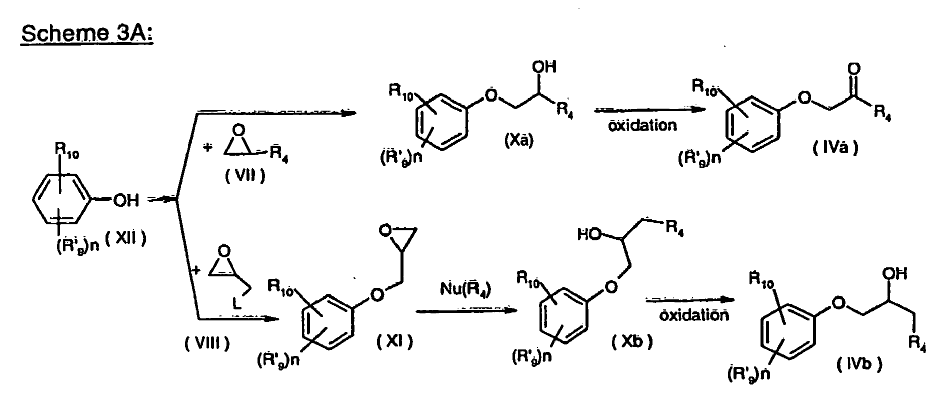 wherein R₄ , R'₉, R₁₀ and n are as defined under formula I and L is a leaving group, While Nu(R₄) designates the nucleophilic form of R₄, such as alkoxides or halides, especially iodides.

For the sake of simplicity in Scheme 3A the optionally substituted Ar₂ is displayed as phenyl, but it is assumed that the entire variation of Ar₂ as defined under formula I can be reacted similarly. According to Scheme 3A the selected intermediates of formula lVa may be prepared by oxidizing the corresponding alcohols of formulae Xa and Xb. Advantageous oxidation procedures include the sulfur-based oxidation agents (in literature referred to as Swem-oxidation, Pfizer-Moffat and others), the metal based oxidation agents, hydrogen peroxide in the presence of metal catalysts such as Na₂WO₄ (c.f. e.g. R.Noyori, Bull.Chem. Soc. Jpn. 1999, 72, 2287-2306) and others more.
The alcohols of formula Xa and Xb are available by ring-opening of an epoxide of formula VII or an epoxide of formula VIII, e.g. epichlorhydrine with a phenol of formula XII and in the latter case reacting the new intermediate epoxide of formula XI again with a nucleophilic derivative of Nu(R₄) such as alkoxides or halides, especially iodides .
The ring opening reaction may be performed in the presence of a catalyst. Suitable catalysts include bases, such as amines like pyridine, tri-ethanolamine and the like, or metal hydroxides and/or carbonates such as lithium hydroxide, cesium carbonate, potassium carbonate, potassium hydroxide, sodium hydroxide or metal hydrides, such as sodium hydride and lithium hydride or cesium fluoride as well as Lewis acids, such as tetramethylammonium chloride. Suitable solvents include alcohols, such as ethanol, isopropanol, tert-butanol and the like, ketones such as acetone and methyl ethyl ketone, and more polar solvents such as N,N-dimethylformamide, dimethylacetamide and nitriles, such as acetonitrile and propionitrile. The reaction temperature can vary within wide limits. It typically lies in the range of room temperature and the boiling point of the reaction mixture.
Preferable solvents for ring opening of the phenoxy-substituted epoxides of formula XI include polyalcohols such as ethyleneglycol, diethyleneglycol and triethyleneglycol, sulfoxides and sulfones such as dimethylsulfoxide and sulfolane as well as other polar solvents. A nucleophiles of special interest in this context is fluoride. Fluoride is typically used in form of potassium hydrogen difluoride (THF₂). The nucleophilicity of the fluoride ion may be enhanced by phase transfer reagents such as quaternary ammonium salts and phosphonium salts as well as complexing agents such as crown ethers. The reaction temperature lies between +100°C and the boiling point of the reaction mixture.
In general, epoxides of the general formulae VII or VIII are commercially available or may be prepared according to published procedures such as reacting a phenol of the general formula XII with epichlorohydrine.

Many methods to prepare sulfonylation agent of formula II are known. General ways of preparing such compounds are e.g. described in Houben Weyl, Vol. E11, p 1067 ff (1985).

Another synthesis to prepare compounds of formula I is described in Scheme 4. 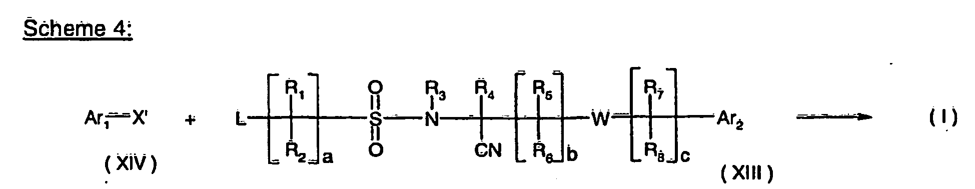 wherein Ar₁, Ar₂, a, b, c, X', W and R₁ to R₈ are defined as under formula I.

Compounds of formula XIII wherein Ar₂, b, c, W and R₃ to R₈ are defined as under formula I and L is a leaving group such as e.g. halogen, preferably chlorine, bromine or iodine or a sulfonyloxy group such a e.g. methylsulfonyloxy, toluylsulfonyloxy- or trifluoromethylsulfonyloxy- group, is coupled with a compound of formula XIV wherein X' is either an anionic radical species of X such as O', S⁻, S(O)ₘ⁻ combined with an alkaline- or earthalkaline-metal cation as counterion or is defined as X-H such as OH, SH, NHR₃. In this case the reaction are generally carried out in the presence of a base such as alkaline-, earthalkaline-carbonates or hydrogencarbonates such e.g. sodium or potassium-carbonate, sodium or potassium -hydrogen-carbonate, cesium-carbonate or an agent capable of scavenging the formed acid.

The starting material of the chemical class of cyano-amines of formula III may also be prepared as described in Scheme 5. 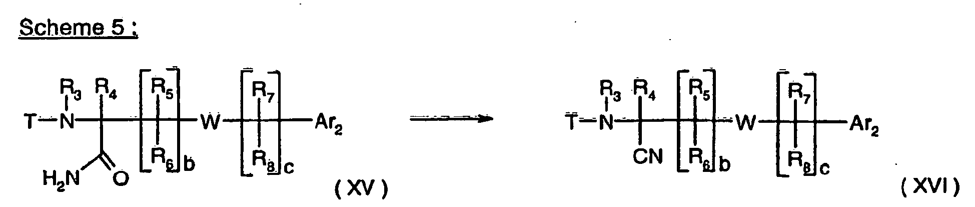 wherein Ar₁, Ar₂, b, c, W and R₃ to R₈ are defined as under formula I and symbol T designates a protecting group such as the well-acknowlegded tert-butyloxycarbonyl- or benzyloxycarbonyl- groups , often referred to as (BOC)- or (Z)- groups, and converting the compund of formula XVI into the desired formula III by cleaving the protective group T off.

Many methods are known in the chemical literature e.g. Synthetic Comm. , 29 (23) ,4235-4239 (1999) or Synthesis (10), 1724-1726 (1999) or Tetrahedron Lett. 29 (18), 2155-2158 (1988) to convert amino acid -amides into amino-nitriles. The reaction conditions for the dihydratisation according to Scheme 5 may be adopted from known examples in the art.
In another preparatory synthesis pathway compounds of formula XXII, which is a subgroup of formula I, may be obtained according to Scheme 6. 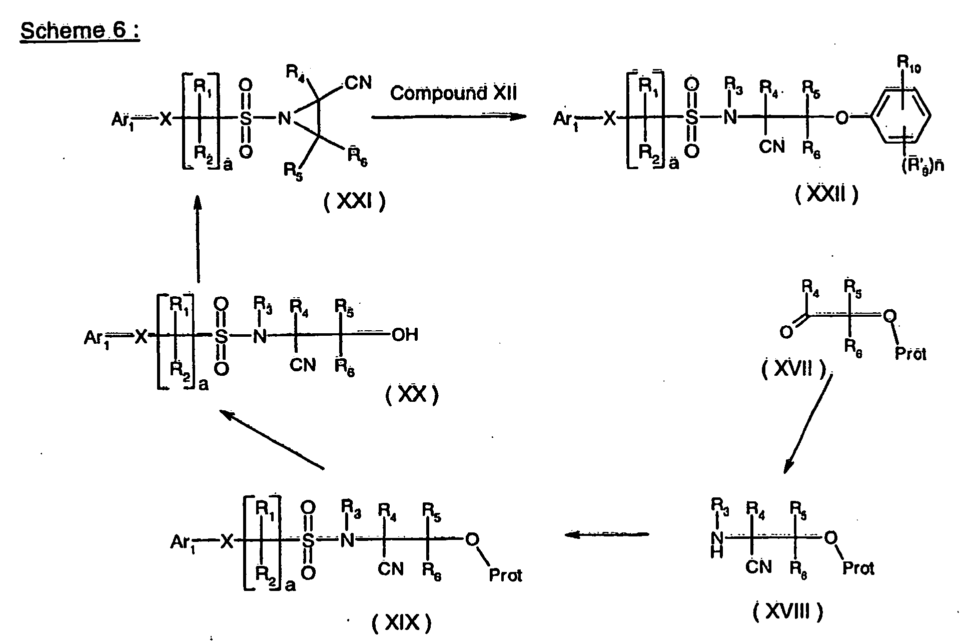 wherein Ar₁, R₁, R₂, R₄, R₅, R₆, R₉, R₁₀ X, n and a are as defined for formula I, R₃ stands for hydrogen and -O-Prot designates an easily cleavable ether group , protecting the hydroxy-function, like the tetrahydropyranyl-oxy group. The reaction pathway of Scheme 6 in the final step shows the ring opening reaction of the aziridine intermediate of formula XXI, which is in analogy to general methods described e.g. Tetrahedron, 52 (40), 13035-13050. The reaction conditions are as e.g. described in the cited reference, using suitable inert solvents , including ketones, such as acetone and methylethylketone, halogenated hydrocarbons such as chloroform, carbontetrachloride, dichloromethane, dichloro-ethane, aromatic hydrocarbons such as toluene or xylene, ethers such as t-butyl-methyl-ether, di-ethyl-ether, tetrahydrofuran and dioxane. The reaction is performed preferentially in the presence of a base. Typical bases include tertiary amines such as trimethylamine, triethylamine, diisopropylethylamine, dimethyl-aniline, diazabi-cyclooctane and N-methylmorpholine, aromatic amines such pyridine and quinoline as well as inorganic bases such as alkaline bicarbonates or -carbonates e.g. example sodium and potassium bicarbonate and sodium, potassium or cesium carbonate. As also shown in Scheme 6 the aziridines of formula XXI may be prepared by a ring closing reaction, starting from alcohols of formula XX, by employing the so-called Mitsunobu reaction. Intermediate alcohols of formula XX are prepared in several step as outlined. Ketones of formula XVII containing a protected hydroxy function in form of the group -O-Prot (Prot stands for the protective radical that temporarily replaces the H atom during a reaction sequence, but thereafter is reinstalled) desigating an easily cleavable ether group, like the tetrahydropyranyl-oxy group, are reacted as described above in a so-called Strecker-Synthesis. The ether groups, i.e. the tetrahydropyranyl-oxy group are easily prepared and cleaved as described e.g in Biorg. Med.Chem.Lett. 11 (2001), 18, 2541-2543.

Enantiomeric mixtures of formula I may be separated into the enantiomers by chromatography on chiral stationary phase or by classical methods of fractionated crystallization of diastereomeric salts of a suitable precursor and subsequent conversion into the desired products. Enantiomers or diastereoisomers may also be prepared by enantioselective or diastereoselective synthesis methods.

Alternatively the pure enantiomeric forms of the compounds of formula I may be obtained by the preparation method of Scheme 1 when using enantiomerically pure starting material of formula III*.
The preparation of the enantiomerically pure amines of formula III* may be achieved by sulfonylation as described in Scheme 7. 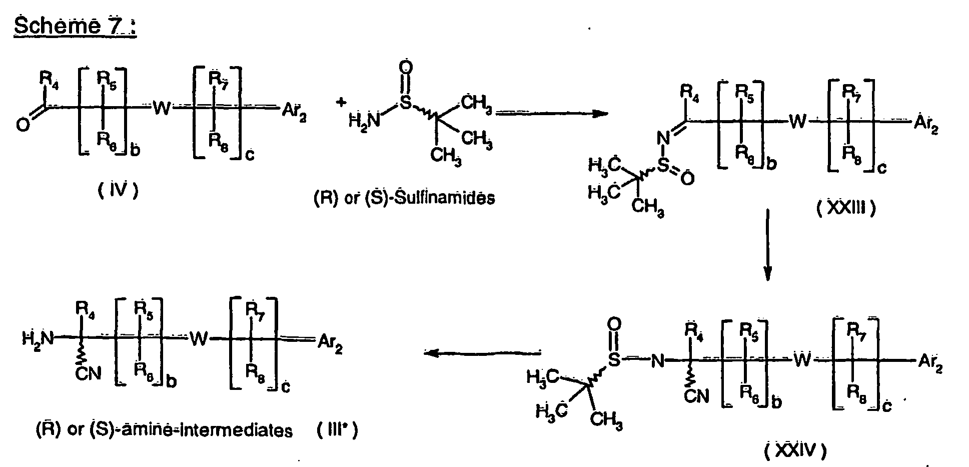 wherein Ar₂, R₄, R₅, R₈, R₇, R₈, W, b and c are as defined for formula I.
In analogy to the methods described in e.g. J.Org. Chem. 1999,64,1278-84, ketones of formula (IV) may be treated with sterically fixed chiral sulfinamides, like th shown (R)-(+)-2-methyl-2-propanesulfinamide or (S) -(-)-2-methyl-2-propanesulfinamide to yield the chiral sulfinimides of formula XXIII.
Introducing the cyanide function by the so-called Strecker-type reaction selectively yields the pure diastereoisomers of formula XXIV. Preferably in this reaction step the sulfimides of formula XXIII are treated with a cyanating reagent, for example a di-alkoxy-alumiriiumcyanide like di-isopropoxy-aluminiumcyanide, in analogy to methods described in e.g. J.Org. Chem.2000, 65,8704-8708. Subsequent cleavage of the sulfinyl group by mineral acids like hydrogen chloride in the last step of the displayed sequence gives the enatomerically pure amines of formula III*.

For this invention it is understood that the preparation method of Scheme 1 encompasses as specific embodiments the preparation of the enantiomerically distinct compounds of formula I which are available when employing the intermediates III* made according to Scheme 7.

The compounds of formula I are oils or solids at room temperature and are distinguished by valuable microbiocidal properties. They can be used in the agricultural sector or related fields preventatively and curatively in the control of plant-destructive microorganisms. The compounds of formula I according to the invention are distinguished at low rates of concentration not only by outstanding microbiocidal, especially fungicidal, activity but also by being especially well tolerated by plants.

Surprisingly, it has now been found that the compounds of formula I have for practical purposes a very advantageous microbiocidal spectrum in the control of phytopathogenic microorganisms, especially fungi. They possess very advantageous curative and preventive properties and are used in the protection of numerous crop plants. With the compounds of formula I it is possible to inhibit or destroy phytopathogenic microorganisms that occur on various crops of useful plants or on parts of such plants (fruit, blossom, leaves, stems, tubers, roots), while parts of the plants which grow latter also remain protected, for example, against phytopathogenic fungi.

The novel compounds of formula I prove to be effective against specific genera of the fungus class Fungi imperfecti (e.g. Cercospora), Basidiomycetes (e.g. Puccinia) and Ascomycetes (e.g. Erysiphe and Venturia) and especially against Oomycetes (e.g. Plasmopara, Peronospora, Pythium and Phytophthora). They therefore represent in plant protection a valuable addition to the compositions for controlling phytopathogenic fungi. The compounds of formula I can also be used as dressings for protecting seed (fruit, tubers, grains) and plant cuttings from fungal infections and against phytopathogenic fungi that occur in the soil.

The invention relates also to compositions comprising compounds of formula I as active ingredient, especially plant-protecting compositions, and to the use thereof in the agricultural sector or related fields.

In addition, the present invention includes the preparation of those compositions, wherein the active ingredient is homogeneously mixed with one or more of the substances or groups of substances described herein. Included is a method of treating plants characterized by the application of the novel compounds of formula I or of the novel compositions.

Target crops to be protected within the scope of this invention comprise, for example, the following species of plants: cereals (wheat, barley, rye, oats, rice, maize, sorghum and related species); beet (sugar beet and fodder beet); pomes, stone fruit and soft fruit (apples, pears, plums, peaches, almonds, cherries, strawberries, raspberries and blackberries); leguminous plants (beans, lentils, peas, soybeans); oil plants (rape, mustard, poppy, olives, sunflowers, coconut, castor oil plants, cocoa beans, groundnuts); cucurbitaceae (marrows, cucumbers, melons); fibre plants (cotton, flax, hemp, jute); citrus fruit (oranges, lemons, grapefruit, mandarins); vegetables (spinach, lettuce, asparagus, cabbages, carrots, onions, tomatoes, potatoes, paprika); lauraceae (avocado, cinnamon, camphor) and plants such as tobacco, nuts, coffee, sugar cane, tea, pepper, vines, hops, bananas and natural rubber plants, and also ornamentals.

The compounds of formula I are normally used in the form of compositions and can be applied to the area or plant to be treated Simultaneously or in succession with other active ingredients. Those other active ingredients may be fertilisers, micronutrient donors or other preparations that influence plant growth. It is also possible to use selective herbicides or insecticides, fungicides, bactericides, nematicides, molluscicides or mixtures of several of those preparations, if desired together with further carriers, surfactants or other application-promoting adjuvants customarily employed in formulation technology.

The compounds of formula I can be mixed with other fungicides, resulting in some cases in unexpected synergistic enhancement of the biological activities. Such mixtures are not limited to two active ingredients (one of formula I and one of the list of other fungicides), but to the contrary many comprise more than one active ingredient of the component of formula I and more than one other fungicide. Mixing components which are particularly suited for this purpose include e.g. azoles, such as azaconazole, bitertanol, bromuconazole, cyproconazole, difenoconazole, diniconazole, epoxiconazole, fenbuconazole, fluquinconazole, flusilazole, flutriafol, hexaconazole, imazalil, S-imazalil, imibenconazole, ipconazole, metconazole, myclobutanil, oxpoconazole, pefurazoate, penconazole, pyrifenox, prochloraz, propiconazole, prothioconazole, simeconazole, tebuconazole, tetraconazole, triadimefon, triadimenol, triflumizole and triticonazole; pyrimidinyl carbinoles, such as ancymidol, fenarimol and nuarimol; 2-amino-pyrimidines, such as bupirimate, dimethirimol and ethirimol; morpholines, such as dodemorph, fenpropidine, fenpropimorph, spiroxamine and tridemorph; anitinopyrimidines, such as cyprodinil, mepanipyrim and pyrimethanil; pyrroles, such as fenpiclonil and fludioxonil; phenylamides, such as benalaxyl, furalaxyl, metalaxyl, R-metalaxyl, ofurace and oxadixyl; benzimidazoles, such as benomyl, carbendazim, debacarb, fuberidazole and thiabendazole; dicarboximides, such as chlozolinate, dichlozoline, iprodione, myclozoline, procymidone and vinclozoline; carboxamides, such as carboxin, fenfuram, flutolanil, furametpyr, mepronil, oxycarboxin and thifluzamide; guanidines, such as guazatine, dodine and iminoctadine; strobilurines, such as azoxystrobin, dimoxystrobin (SSF-129), fluoxastrobin, kresoxim-methyl, metominostrobin, orysastrobin, picoxystrobin, pyraclostrobin and trifloxystrobin; dithiocarbamates, such as ferbam, mancozeb, mane, metiram, propineb, thiram, zineb and ziram; N-halomethylthiotetrahydrophthalimides, such as captafol, captan, dichlofluanid, fluoromides, folpet and tolyfluanid; Copper-compounds, such as Bordeaux mixture, copper hydroxide, copper oxychloride, copper sulfate, cuprous oxide, mancopper and oxine-copper; nitrophenol-derivatives, such as dinocap and nitrothalisopropyl; organo-P-derivatives, such as edifenphos, iprobenphos, isoprothiolane, phosdiphen, pyrazophos and tolclofos-methyl; various others, such as acibenzolar-S-methyl, anilazine, benthiavalicarb, blasticidin-S, boscalid, chinomethtonate, chloroneb, chlorothalonil, IKF-916 (proposed name cyazofamid), cyflufenamid, cymoxanil, dichione, diclomezine, dicloran, diethofencarb, dimethomorph, ethaboxam, fenoxanil, SYP-LI90 (proposed name: flumorph), dithianon, etridiazole, famoxadone, fenamidone, fentin, ferimzone, fluazinam, flusulfamide, fenhexamid, fosetyl-aluminium; hymexazol, iprovalicarb, kasugamycin, methasulfocarb, metrafenone, pencycuron, phthalide, picobenzamid, polyoxins, probenazole, propamocarb, pyroquilon, proquinazid, quinoxyfen, quintozene, silthiofam, sulfur, triazoxide, triadinil, tricyclazole, triforine, validamycin, or zoxamide.

In the above mentioned mixtures, the mixture ratio of the active ingredients is so selected that it reaches optional control of the phytopathogenic microorganism on the host plants. This ratio is in general between 100:1 and 1:100, more preferably between 10:1 and 1:10 of a compound of formula I vis-à-vis the second fungicide. The mixtures may not only comprise one of the listed combinational active ingredients, but may comprise more than one additional active ingredients selected from that specified group, thus forming for example 3-way- or even 4-way-mixtures.

Suitable carriers and surfactants may be solid or liquid and correspond to the substances ordinarily employed in formulation technology, such as e.g. natural or regenerated mineral substances, solvents, dispersants, wetting agents, tackifiers, thickeners, binders or fertilisers. Such carriers and additives are described, for example, in WO 95/30651.

A preferred method of applying a compound of formula I, or an agrochemical composition comprising at least one of those compounds, is application to the foliage (foliar application), the frequency and the rate of application depending upon the risk of infestation by the pathogen in question. The compounds of formula I may also be applied to seed grains (coating) either by impregnating the grains with a liquid formulation of the active ingredient or by coating them with a solid formulation.

The compounds of formula I are used in unmodified form or, preferably, together with the adjuvants conventionally employed in formulation technology, and are for that purpose advantageously formulated in known manner e.g. into emulsifiable concentrates, coatable pastes, directly sprayable or dilutable solutions, dilute emulsions, wettable powders, soluble powders, dusts, granules, and by encapsulation in e.g. polymer substances. As with the nature of the compositions, the methods of application, such as spraying, atomising, dusting, scattering, coating or pouring, are chosen in accordance with the intended objectives and the prevailing circumstances.

Advantageous rates of application are normally from 1 g to 2 kg of active ingredient (a.i.) per hectare (ha), preferably from 10 g to 1 kg a.i./ha, especially from 25 g to 750 g a.i./ha.

When used as seed dressings, rates of from 0.001 g to 5.0 g of active ingredient per kg of seed are advantageously used.

The formulations, i.e, the compositions, preparations or mixtures comprising the compound(s) (active ingredient(s)) of formula I and, where appropriate, a solid or liquid adjuvant, are prepared in known manner; e.g. by homogeneously mixing and/or grinding the active ingredient with extenders, e.g. solvents, solid carriers and, where appropriate, surface-active compounds (surfactants).

Further surfactants customarily used in formulation technology will be known to the person skilled in the art or can be found in the relevant technical literature.

The agrochemical compositions usually comprise 0.01 to 99 % by weight, preferably 0.1 to 95 % by weight, of a compound of formula I, 99.99 to 1 % by weight, preferably 99.9 to 5 % by weight, of a solid or liquid adjuvant, and 0 to 25 % by weight, preferably 0.1 to 25 % by weight, of a surfactant.

Whereas commercial products will preferably be formulated as concentrates, the end user will normally employ dilute formulations.

The compositions may also comprise further ingredients, such as stabilisers, antifoams, viscosity regulators, binders and tackifiers, as well as fertilisers or other active ingredients for obtaining special effects.

The Examples which follow illustrate the invention described above, without limiting the scope thereof in any way. Temperatures are given in degrees Celsius.

### Preparation Examples:

### Example 1: 2-(4-Chlorophenoxy-methyl-2-benzylsulfonylamino-propionitrile

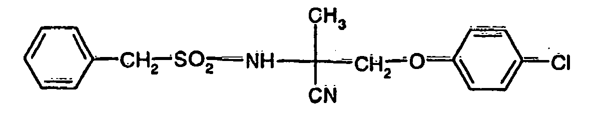

### a) 1-(4-Chlorophenoxy)-propan-2-one

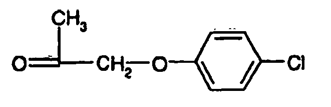

A suspension of 4-chlorophenol (2.6g, 20mmol), chloroacetone (2.8g, 30mmol) and potassium carbonate (3.45g, 25mmol) in acetone (100ml) is heated at reflux for 3 hours. The precipitating inorganic salts are filtered off and the filtrate is evaporated to dryness to give the 1-(4-chlorophenoxy)-propan-2-one.

### b) 2-(4-Chlorophenoxy-methyl)-2-amino-propionitrile

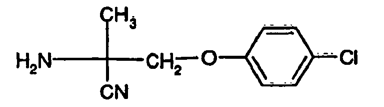

suspension of 1-(4-chloro-phenoxy)-propan-2-one (3.7g, 20mmol), ammonium chloride (1.6g, 29.2mmol) and sodium cyanide (1.15g, 23.3mmol) in a solution of aqueous ammonia (100ml, 28%) is stirred vigorously at room temperature for 20 hours. The aqueous phase is extracted repeatedly with ethyl acetate. The collected organic extracts are dried and the solvent is evaporated under reduced pressure to give the 2-(4-chlorophenoxy-methyl)-2-amino-propionitrile as a solid.

c) A solution of 2-(4-chlorophenoxy-methyl)-2- amino-propionitrile (0.5g, 2.4mmol) and benzylsulfonyl chloride (0.5g, 2.6mmol) in pyridine (5ml) is heated at +80°C with stirring in the presence of DMAP (0.02g) for about 16 hours. The reaction mixture is diluted with ethyl acetate and washed with brine. The organic phase is dried over magnesium sulfate and the product is purified by flash chromatography (eluent: ethyl acetate / hexanes 1:3) to give the 2-(4-chlorophenoxy-methyl)-2-benzylsulfonylamino-propionitrile in form of a colorless solid, m.p. 120-121 °C.

### Example 1b: 2-(4-Chlorophenoxy-methyl)-2-benzylsulfonylamino-propionitrile

### a) 1-(Tetrahydropyran-2-yloxy)-propan-2-one

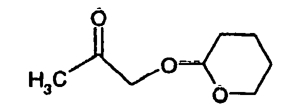

A solution of 1-hydroxy-propan-2-one (95%, 78g, 1.0mol), 3,4-dihydro-2H-pyrane (95%, 88.5g, 1.0mol) and pyridinium p-toluenesulfonate (25.1 g, 0.1 mol) in THF (1.21) is stirred at room temperature for 16 hours. The mixture is diluted with ethyl acetate and repeatedly washed with brine. The organic phase is dried, filtered evaporated to afford 1-(tetrahydropyran-2-yloxy)-propan-2-one (127.8g, 80.8%) as a liquid.

### b) 2-Amino-2-methyl-3-(tetrahydro-pyran-2-yloxy)-propionitrile

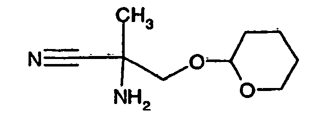

A solution of 1-(tetrahydro-pyran-2-yloxy)-propan-2-one (126.6g, 0.8mol), ammonium chloride (65.8g, 1.23mol) and sodium cyanide (45.5g, 0.92mol) in a solution of aqueous ammonia (920ml, 28%) is stirred vigorously at room temperature for 5 hours. The aqueous phase is extracted repeatedly with ethyl acetate. The collected organic extracts are dried and the solvent is evaporated under reduced pressure to give the 2-amino-2-methyl-3-(tetrahydro-pyran-2-yloxy)-propionitrile (130g, 88.2%) as a liquid,

### c) N-[1-Cyano-1-methyl-2-(tetrahydro-pyran-2-yloxy)-ethyl]-C-benzylsulfonamide

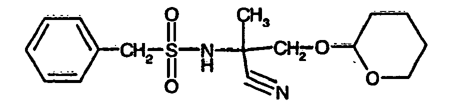

A suspension of 2-Amino-2-methyl)-3-(tetrahydro-pyran-2-yloxy)-propionitrile (74.7g, 0.405mol), benzylsulfonyl chloride (90g, 0.473mol) and diazabicyclooctane (90.9g, 0.81 mol) in THF (1.31) is stirred at room temperature for about 15 hours. The reaction mixture is diluted with ethyl acetate and washed with brine. The organic phase is dried over magnesium sulfate, the solvent is evaporated under reduced pressure to give the N-[1-cyano-1-methyl-2-(tetrahydro-pyran-2-yloxy)-ethyl]-C-benzylsulfonamide (137g_{,} 100%) as a resin.

### d) N-(1-Cyano-2-hydroxy-1-methyl-ethyl)-C-benzylsulfonamide

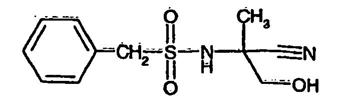

A solution of N-[1-cyano-1-methyl-2-(tetrahydro-pyran-2-yloxy)-ethyl]-C-benzylsulfonamide (135.4g, 0.4mol) and pyridinium p-toluenesulfonate (13g, 0.05mol) in ethanole (0.51) is stirred at 60°C for 4 hours. The solvent is evaporated under reduced pressure. The residue is then poured onto water (0.5l). The solid was collected by filtration, washed several times with water (0.61), an then dried in a vacuum to yield N-(1-cyano-2-hydroxy-1-methyl-ethyl)-C-phenyl-methanesulfonamide (74.2g, 72.9%) as an white solid, m.p.153-155°C. ¹HNMR(CDCl₃) δJ(Hz): 1.73(s,3H,CH₃),3.62-3.66(d,1H,CH),3.92-3.96(d,1H,CH); 4.65(s,2H,CH₂), 6.00(s,1H,OH), 7.59(s,5H,Ar-H), 8.03(s,1H,NH).
MS(ES⁻) m/z 253 (M-H)⁻.

### e) 2-Methyl-1-benzylsulfonyl-aziridine-2-carbonitrile

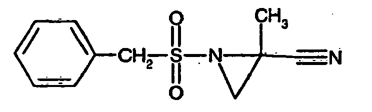

Diethyl azodicarboxylate (0.91 ml, 5.85mmol) was added dropwise below 10°C to a mixture of N-(1-cyano-2-hydroxy-1-methyl-ethyl)-C-benzylsulfonamide (1.1.4g, 4.5mmol) and Triphenylphosphine (1.53g, 5.8mmol) in THF (25ml). The mixture is stirred at room temperature for 2 hours. The solvent was evaporated and the residue is purified by flash chromatography (eluent: ethyl acetate / hexanes 2:3) to yield 2-methyl-1-benzylsulfonyl-aziridine-2-carbonitrile (1.03g, 96.8%) as a colorless solid.
¹HNMA(CDCl₃)δJ(Hz)1.65(s,3H,CH₃),2.35(s, 1H,CH),2.69(1, 1H,CH),4.57(s,2H,CH₃), 7.43(s,5H,Ar-H).

f) Cesiumcarbonate (390mg, 1.2mmol) was added to a solution of 2-methyl-1-benzylsulfonyl-aziridine-2-carbonitrile (236mg, 1.0mmol) in dichloromethane (4ml) at room temperature with stirring for 5 hours, The reaction mixture was diluted with ethyl acetate and washed with brine. The organic phase is dried over magnesium sulfate and the product is purified by flash chromatography (eluent: ethyl acetate / hexanes 2:3) to give the 2-(4-chlorophenoxy-methyl)-2-benzylsulfonylamino-propionitrile (180mg, 49.3%) in form of a colorless solid, m.p. 120-121°C.

### Example 2: 2-(4-Chlorophenoxy-methyl)-2-benzylsulfonylamino-butyronitrile

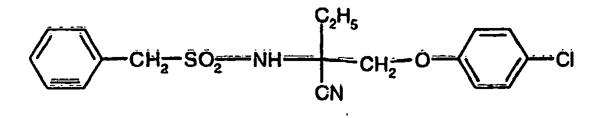

### a)1-(4-Chlorophenoxy)-butane-2-ol

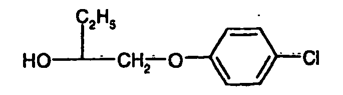

A suspension of chlorophenol (9.0g, 0.07mol) and butyleneoxide (6.9ml, 0.08mmol) in toluene (80ml) is heated at reflux with stirring in the presence of a catalytic amount of cesium fluoride (1.5g) for 20 hours. The reaction mixture is washed with an aqueous solution of sodium hydroxide and dried. The volatiles are evaporated under reduced pressure to give the 1-(4-chloro-phenoxy)-butane-2-ol as a colorless solid.

### b) 1-(4-Chlorophenoxy)-butan-2-one

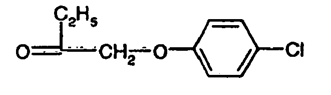

Oxalylchloride (1.0ml, 0.011 mmol) is added to a solution ofdimethylsulfoxide (DMSO) (1.7ml, 0.022mol) in methylenechloride (20ml) at-60°C. 1-(4-Chlorophenoxy)-butan-2-ol (2.0g, 0.01 mol) is added in one portion. After 15 minutes triethylamine (7ml, 0.05mol) is added and the reaction temperature is allowed to warm up and reach 0°C. The mixtures is diluted with diethylether and washed repeatedly with brine. The organic phase is dried, filtered evaporated to give the 1-(4-chlorophenoxy)-butane-2-one.
¹H-NMR (CDCl₃): 7.26 (d, 2H); 6.82 (d, 2H); 4.53 (s, 2H); 2.60 (q, 2H); 1.12 (t, 3H).

### c) 2-(4-Chlorophenoxy)-methyl)-2-amino-butyronitrile

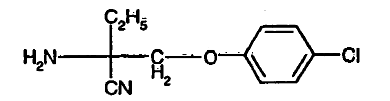

A suspension of 1-(4-chloro-phenoxy)-butan-2-one (3.0g, 15mmol), ammonium chloride (1.3g, 25mmol) and sodium cyanide (1.0g, 20mmol) in a solution of aqueous ammonia (100ml, 28%) is stirred vigorously at room temperature for 20 hours. The aqueous phase is extracted repeatedly with ethyl acetate. The combined organic phases are dried over sodium sulfate and the solvent is evaporated under reduced pressure to give the 2-(4-chlorophenoxy)-methyl)-2-amino-butyronitrile as an oil having sufficient purity for being directly employable for the following reaction step.

d) A solution of 2-(4-chlorophenoxy)-methyl)-2-amino-butyronitrile (0.5g, 2.2mmol), benzylsulfonyl chloride (0.50g, 2.6mmol) and diazabicyclooctane (1.0g, 4mmol) in anhydrous tetrahydrofuran (20ml) is stirred for about 16 hours. The reaction mixture is diluted with ethyl acetate and washed With brine. The organic phase is dried over magnesium sulfate and the residue raw product is purified by flash chromatography (eluent: ethyl acetate / hexanes 1:3) to give the 2-(4-chlorophenoxy-methyl)-2-benzylsulfonylamino-butyronitrile in form of a colorless oil. ¹H-NMR (CDCl₃): 7.54-7.50 (m, 2H); 7.46-7.40(m,3H); 7.30 (d,2H); 6.84 (d, 2H); 4.70 (s, 1H); 4.49 (s, 2H); 4.22 (dxd, 2H); 2.26-2.04 (m, 2H); 1.12 (t, 3H).

### Example_3: 2-(4-Chlorophenoxy-methyl)-2-(4-chlorophenyl-methylsulfonyl)-propionitrile

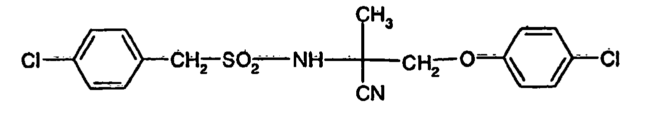

A solution of 2-amino-2-(4-chlorophenoxymethyl)-propionitrile (0.5g, 0.24mmol), 4-chlorophenyl-methylsulfonyl chloride (0.46g, 2.4mmol) and diazabicyclooctane (0.6g, 2.4mmol) in anhydrous tetrahydrofuran (20ml) is stirred for about 16 hours. The reaction mixtures is diluted with ethyl acetate and washed with brine. The organic phase is dried over magnesium sulfate and the raw product received as the residue is purified by flash chromato-graphy (eluent: ethyl acetate / hexanes 1:3) to give the 2-(4-chlorophenoxy-methyl)-2-(4-chlorophenyl-methylsulfonyl)-propionitrile in form of colorless crystals, m.p. 119-120°C.

In analogous manner the compounds of following Table 1 are obtained.

**Table 1:**

| | | | | | | | | | | |
|---|---|---|---|---|---|---|---|---|---|---|
| 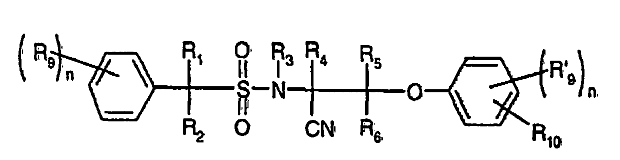 | | | | | | | | | | |

| No. | R₁ | R₂ | R₃ | R₄ | R₅ | R₆ | (R₉)ₙ | (R'₉)ₙ | R₁₀ | m.p.[°C] |
|---|---|---|---|---|---|---|---|---|---|---|
| 001 | H | H | H | CH₃ | H | H | H | 2-Cl | H | solid |
| 002 | H | H | H | CH₃ | H | H | 4-F | 2-Cl | H | |
| 003 | CH₃ | H | H | CH₃ | H | H | H | 2-Cl | H | |
| 004 | H | H | H | CH₂CH₃ | H | H | H | 2-Cl | H | |
| 005 | H | H | H | CH₂CH₂CH₃ | H | H | H | 2-Cl | H | |
| 006 | H | H | CH₂ -OCH₂CH₃ | CH₃ | H | H | H | 2-Cl | H | |
| 007 | H | H | CH₂-OCH₂CH₃ | CH₂CH₃ | H | H | H | 2-Cl | H | |
| 008 | H | H | H | CH₃ | H | H | H | 2,3-Cl₂ | H | solid |
| 009 | H | H | H | CH₂CH₃ | H | H | H | 2,3-Cl₂ | H | |
| 010 | H | H | H | CH₂(CH₃)₂ | H | H | H | 2,3-Cl₂ | H | |
| 011 | H | H | H | CH₂F | H | H | H | 2,3-Cl₂ | H | |
| 012 | H | H | CH₂-OCH₂CH₃ | CH₃ | H | H | H | 2,3-Cl₂ | H | |
| 013 | H | H | CH₂-OCH₂CH₃ | CH₂CH₃ | H | H | H | 2,3-Cl₂ | H | |
| 014 | H | H | H | CH₃ | H | H | H | 3,4-Cl₂ | H | |
| 015 | H | H | H | CH₂CH₃ | H | H | H | 3,4-Cl₂ | H | |
| 016 | H | H | H | CH₂(CH₃)₂ | H | H | H | 3,4-Cl₂ | H | |
| 017 | H | H | H | CH₂F | H | H | H | 3,4-Cl₂ | H | |
| 018 | H | H | CH₂-OCH₂CH₃ | CH₃ | H | H | H | 3,4-Cl₂ | H | |
| 019 | H | H | CH₂-OCH₂CH₃ | CH₂CH₃ | H | H | H | 3,4-Cl₂ | H | |
| 020 | H | H | H | CH₃ | H | H | H | 2,4-Cl₂ | H | solid |
| 021 | H | H | H | CH₂CH₃ | H | H | H | 2,4-Cl₂ | H | |
| 022 | H | H | H | CH₂(CH₃)₂ | H | H | H | 2,4-Cl₂ | H | |
| 023 | H | H | H | CH₂F | H | H | H | 2,4-Cl₂ | H | |
| 024 | H | H | H | CH₂CH₃ | H | H | CH₃ | 2,4-Cl₂ | H | |
| 025 | H | H | H | CH₂CH₃ | H | H | H | 2,4-Cl₂ | H | |
| 026 | H | H | CH₂-OCH₂CH₃ | CH₃ | H | H | 4-F | 2,4-Cl₂ | H | |
| 027 | H | H | CH₂-OCH₂CH₃ | CH₂CH₃ | H | H | H | 2,4-Cl₂ | H | |
| 028 | H | H | H | CH₃ | H | H | H | 4-CF₃ | H | 142-143 |
| 029 | H | H | H | CH₃ | H | H | 4-F | 4-CF₃ | H | |
| 030 | CH₃ | H | H | CH₃ | H | H | H | 4-CF₃ | H | |
| 031 | H | H | H | CH₂CH₃ | H | H | H | 4-CF₃ | H | |
| 032 | H | H | H | CH₂CH₂CH₃ | H | H | H | 4-CF₃ | H | |
| 033 | CH₃ | CH₃ | H | CH₃ | H | H | H | 4-CF₃ | H | |
| 034 | H | H | H | CH₃ | H | H | H | 4-CH(CH₃)₂ | H | |
| 035 | H | H | H | CH₃ | H | H | 4-F | 4-CH(CH₃)₂ | H | |
| 036 | CH₃ | H | H | CH₃ | H | H | H | 4-CH(CH₃)₂ | H | |
| 037 | H | H | H | CH₂CH₃ | H | H | H | 4-CH(CH₃)₂ | H | |
| 038 | H | H | H | CH₂CH₂CH₃ | H | H | H | 4-CH(CH₃)₂ | H | |
| 039 | CH₃ | CH₃ | H | CH₃ | H | H | H | 4-CH(CH₃)₂ | H | |
| 040 | H | H | H | CH₃ | H | H | H | 4-Cl | H | 120-121 |
| 041 | H | H | H | CH₃ | H | H | 2-CH₃ | 4-Cl | H | oil |
| 042 | H | H | CH₂-OCH₂CH₃ | CH₃ | H | H | H | 4-Cl | H | |
| 043 | H | H | CH₂-OCH₂CH₃ | CH₂CH₃ | H | H | H | 4-Cl | H | |
| 044 | H. | H | H | CH₃ | H | H | 2-Cl | 4-Cl | H | 116-117 |
| 045 | H | H | H | CH₃ | H | H | 4-F | 4-Cl | H | 105-106 |
| 046 | H | H | H | CH₃ | Hi | H | 4-SO₂CH₃ | 4-Cl | H | 134-137 |
| 047 | H | H | H | CH₃ | H | H | 3-Cl | 4-Cl | H | 110-111 |
| 048 | H | H | H | CH₃ | H | H | 2-CF₃ | 4-Cl | H | 98-100 |
| 049 | H | H | H | CH₃ | H | H | 2,6-Cl₂ | 4-Cl | H | oil |
| 050 | H | H | H | CH₃ | H | H | 2-F | 4-Cl | H | 96-97 |
| 051 | H | H | H | CH₃ | H | H | 4-Cl | 4-Cl | H. | 119-120 |
| 052 | H | H | H | CH₂CH₃ | H | H | H | 4-Cl | H | oil |
| 053 | H | H | CH₃ | CH₃ | H | H | H | 4-Cl | H | oil |
| 054 | H | H | H | CH₂OC-(CH₃)₃ | H | H | H | 4-Cl | H! | oil |
| 055 | H | H | H | CH₂OCH₂CH-(CH₃)₂ | H | H | H | 4-Cl | H | oil |
| 056 | H | H | H | CH₂OCH₂CH=CH₂ | H | H | H | 4-Cl | H | 77-78 |
| 057 | H | H | H | CH₂OCH₃ | H | H | H | 4-Cl | H | oil |
| 058 | H | H | H | (CH₂)₂CH₃ | H | H | H | 4-Cl | H | oil |
| 059 | H | H | H | CH₂F | H | H | H | 4-Cl | Hi | oil |
| 060 | H | H | H | H | H | H | H | 2,4-Cl₂-6(N=CHCH=CH)-5 | | solid |
| 061 | H | H | H | CH(CH₃)₂ | H | H | H | 4-Cl | H | resinous |
| 062 | H | H | H | C(CH₃)₃ | H | H | H | 4-Cl | H | solid |
| 063 | H | H | H | CH₂CH₃ | CH₃ | H | H | 4-Cl | H | |
| 064 | H | H | H | CH₂CH₃ | CH₃ | CH₃ | H | 4-Cl | H | |
| 065 | H | H | H | CH₃ | CH₃ | H | H | 4-Cl | H | |
| 066 | H | H | H | CH₂CH₃ | CH₃ | CH₂CH₃ | H | 4-Cl | H | |
| 067 | H | H | H | CH₃ | CH₃ | CH₃ | H | 4-Cl | H | |
| 068 | H | H | H | CH₃ | H | H | H | 3-OCH₃,4-Cl | H | 68 |
| 069 | H | H | H | CH₃ | H | H | H | 2-OCH₃,4-Cl | H | 108-109 |
| 070 | H | H | H | CH₂CH₃ | H | H | H | 2-OCH₃,4-Cl | H | |
| 071 | H | H | H | CH₃ | H | H | H | 4-OCF₃ / | H | 124-125 |
| 072 | H | H | H | CH₃ | H | H | 4-F | 4-OCF₃ | H | |
| 073 | CH₃ | H | H | CH₃ | H | H | H | 4-OCF₃ | H | |
| 074 | H | H | H | CH₂CH₃ | H | H | H | 4-OCF₃ | H | |
| 075 | H | H | H | CH₂CH₂CH₃ | H | H | H | 4-OCF₃ | H | |
| 076 | H | H | H | CH₂CH₃ | CH₃ | H | H | 4-OCF₃ | H | |
| 077 | H | H | H | CH₂CH₃ | CH₃ | CH₃ | H | 4-OCF₃ | H | |
| 078 | CH₃ | H | H | CH₂CH₃ | H | H | H | 4-OCF₃ | H | |
| 079 | H | H | H | CH₃ | H | H | H | 2-F;4-Cl | H | oil |
| 080 | H | H | H | CH₃ | H | H | H | 2-CH₃;4-Cl | H | oil |
| 081 | H | H | H | CH₃ | H | H | H | 4-F | H | 100-103 |
| 082 | H | H | H | CH₃ | H | H | 4-F | 4-F | H | |
| 083 | CH₃ | H | H | CH₃ | H | H | H | 4-F | H | |
| 084 | H | H | H | CH₂CH₃ | H | H | H | 4-F | H | |
| 085 | H | H | H | CH₂CH₂CH₃ | H | H | H | 4-F | H | |
| 086 | H | H | H | CH₂CH₃ | CH₃ | H | H | 4-F | H | |
| 087 | H | H | H | CH₂CH₃ | CH₃ | CH₃ | H | 4-F | H | |
| 088 | CH₃ | CH₃ | H | CH₃ | CH₃ | CH₃ | H | 4-F | H | |
| 089 | H | H | H | CH₃ | H | H | H | 2,4,6-Cl₃ | H | 128-130 |
| 090 | H | H | H | CH₃ | H | H | H | 3-F;4-Cl | H | 123-124 |
| 091 | H | H | H | CH₃ | H | H | H | 3-CH₃; 4-Cl | H | 109-110 |
| 092 | H | H | H | CH₃ | H | H | H | H | 4-C₆H₅ | 135-136 |
| 093 | H | H | H | CH₃ | H | H | H | 4-CH₃ | H | 89-90 |
| 094 | H | H | H | CH₃ | H | H | 4-F | 4-CH₃ | H | |
| 095 | CH₃ | H | H | CH₃ | H | H | H | 4-CH₃ | H | |
| 096 | H | H | H | CH₂CH₃ | H | H | H | 4-CH₃ | H | |
| 097 | H | H | H | CH₂CH₂CH₃ | H | H | H | 4-CH₃ | H | |
| 098 | H | H | H | CH₂CH₃ | CH₃ | H | H | 4-CH₃ | H | |
| 099 | H | H | H | CH₃ | H | H | H | 4-C₆H₁₁(cycl) | H | 145-146 |
| 100 | H | H | H | CH₃ | H | H | H | 3-CH₃ | H | 88-89 |
| 101 | H | H | H | CH₃ | H | H | H | 3,5-(CH₃)₂;4-Cl | H | 118-119 |
| 102 | H | H | H | CH₃ | H | H | H | 3-CH₂CH₃,4-Cl | H | 115-116 |
| 103 | H | H | H | CH₃ | CH₃ | CH₃ | H | 3-CH₂CH₃,4-Cl | H | |
| 104 | H | H | H | CH₃ | H | H | H | 4-OCH₃ | H | oil |
| 105 | H | H | H | CH₂CH₃ | H | H | H | 4-OCH₃ | H | 127-128 |
| 106 | H | H | CH₂-OCH₂CH₃ | CH₃ | H | H | H | 4-OCH₃ | H | |
| 107 | H | H | CH₂-OCH₂CH₃ | CH₂CH₃ | H | H | H | 4-OCH₃ | H | |
| 108 | H | H | H | CH₂CH₃ | H | H | 4-F | 4-OCH₃ | H | |
| 1109 | H | H | H | CH₃ | H | H | 4-F | 4-OCH₃ | H | |
| 110 | CH₃ | H | H | CH₂CH₃ | H | H | 4-F | 4-OCH₃ | H | |
| 111 | CH₃ | H | H | CH₃ | H | H | 4-F | 4-OCH₃ | H | |
| 112 | CH₃ | H | H | CH₂CH₃ | H | H | H | 4-OCH₃ | H | |
| 113 | CH₃ | H | H | CH₃ | H | H | H | 4-OCH₃ | H | |
| 114 | CH₃ | H | H | CH₂CH₃ | H | H | H | 4-OCH₃ | H | |
| 115 | CH₃ | H | H | CH₃ | H | H | H | 4-OCH₃ | H | |
| 116 | CH₃ | CH₃ | H | CH₂CH₃ | CH₃ | H | H | 4-OCH₃ | H | |
| 117 | CH₃ | CH₃ | H | CH₃ | CH₃ | H | H | 4-OCH₃ | H | |
| 118 | H | H | H | CH₂CH₃ | H | H | 4-Cl | 4-OCH₃ | H | |
| 119 | H | H | H | CH₃ | H | H | 4-Cl | 4-OCH₃ | H | |
| 120 | H | H | H | CH₂CH₃ | H | H | 2-F | 4-OCH₃ | H | |
| 121 | H | H | H | CH₃ | H | H | 2.F | 4-OCH₃ | H | |
| 122 | H | H | H | CH₂CH₃ | H | H | 2,4-F₂ | 4-OCH₃ | H | |
| 123 | H | H | H | CH₃ | H | H | 2,4-F₂ | 4-OCH₃ | H | |
| 124 | H | H | H | CH₂CH₃ | H | H | 2,4-Cl₂ | 4-OCH₃ | H | |
| 125 | H | H | H | CH₃ | H | H | 2,4-Cl₂ | 4-OCH₃ | H | |
| 126 | H | H | H | CH₂CH₃ | H | H | 3,4-F₂ | 4-OCH₃ | H | |
| 127 | H | H | H | CH₃ | H | H | 3,4-F₂ | 4-OCH₃ | H | |
| 128 | H | H | H | CH₂CH₃ | H | H | 3,4-Cl₂ | 4-OCH₃ | H | |
| 129 | H | H | H | CH₃ | H | H | 3,4-Cl₂ | 4-OCH₃ | H | |
| 130 | H | H | H | CH₂CH₃ | H | H | 4-CH₃ | 4-OCH₃ | H | |
| 131 | H | H | H | CH₃ | H | H | 4-CH₃ | 4-OCH₃ | H | |
| 132 | H | H | H | CH₂CH₃ | CH₃ | CH₃ | H | 4-OCH₃ | H | |
| 133 | H | H | H | CH₃ | CH₃ | CH₃ | H | 4-OCH₃ | H | |
| 134 | H | H | H | CH₂CH₃ | CH₃ | CH₂CH₃ | H | 4-OCH₃ | H | |
| 135 | H | H | H | CH₂CH₃ | H | H | H | 3-F;4-OCH₃ | H | |
| 136 | H | H | H | CH₂CH₃ | H | H | H | 3-Cl;4-OCH₃ | H | |
| 137 | H | H | H | CH₂CH₃ | H | H | H | 2-F;4-OCH₃ | H | |
| 138 | H | H | H | CH₂CH₃ | H | H | H | 2-Cl;4-OCH₃ | H | |
| 139 | H | H | H | CH₃ | H | H | H | 3-F;4-OCH₃ | H | |
| 140 | H | H | H | CH₃ | H | H | H | 3-Cl;4-OCH₃ | H | |
| 141 | H | H | H | CH₃ | H | H | H | 2-F;4-OCH₃ | H | |
| 142 | H | H | H | CH₃ | H | H | H | 2-Cl;4-OCH₃ | H | |
| 143 | H | H | H | CH₂CH₃ | CH₂CH₃ | CH₂CH₃ | H | 4-OCH₃ | H | |
| 144 | H | H | H | CH₃ | H | H | H | H | 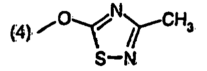 | oil |
| 145 | H | H | CH₂-OCH₂CH₃ | CH₃ | H | H | H | H | 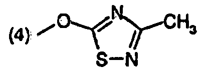 | |
| 146 | H | H | CH₂-OCH₂CH₃ | CH₂CH₃ | H | H | H | H | 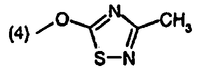 | |
| 147 | H | H | H | CH₃ | H | H | 4-F | H | 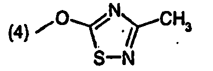 | |
| 148 | CH₃ | H | H | CH₃ | H | H | H | H | 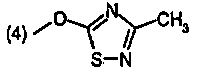 | |
| 149 | H | H | H | CH₂CH₃ | H | H | H | H | 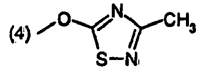 | |
| 150 | H | H | H | CH₂CH₂CH₃ | H | H | H | H | 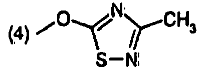 | |
| 151 | H | H | H | CH₂CH₃ | CH₃ | CH₂CH₃ | H | H | 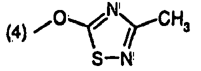 | |
| 152 | H | H | H | CH₂CH₃ | CH₃ | H | H | H | 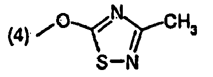 | |
| 153 | H | H | H | CH₂CH₃ | CH₃ | CH₃ | H | H | 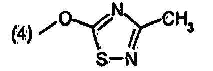 | |
| 154 | H | H' | H | CH₂CH₃ | H | H | H | 2-Cl | 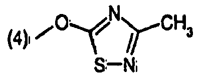 | |
| 155 | H | h | H | CH₃ | H | H | H | 4-CH₂CONH₂ | H | 113-116 |
| 156 | H | H | H | CH₂CH₃ | H | H | | 4-CH₂CONH₂ | H | |
| 157 | H | H | H | CH₃ | H | H | H | H | 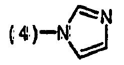 | 128-131 |
| 158 | H | H | CH₂-OCH₂CH₃ | CH₃ | H | H | H | H | 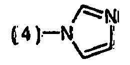 | |
| 159 | H | H | CH₂-OCH₂CH₃ | CH₂CH₃ | H | H | H | H | 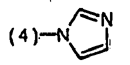 | |
| 160 | H | H | H | CH₂CH₃ | H | H | 4-F | H | 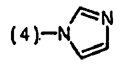 | |
| 161 | H | H | H | CH₃ | H | H | 4-F | H | 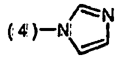 | |
| 162 | CH₃ | H | H | CH₃ | H | H | H | H | 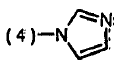 | |
| 163 | CH₃ | H | H | CH₂CH₃ | H | H | H | H | 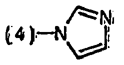 | |
| 164 | H | H | H | CH₂CH₃ | H | H | 4-Cl | H | 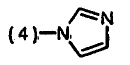 | |
| 165 | H | H | H | CH₃ | H | H | 4-Cl | H | 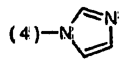 | |
| 166 | H | H | H | CH₂CH₃ | H | H | 2-F | H | 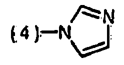 | |
| 167 | H | H | H | CH₃ | H | H | 2-F | H | 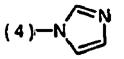 | |
| 168 | H | H | H | CH₂CH₃ | H | H | 2,4-F₂ | H | 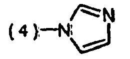 | |
| 169 | H | H | H | CH₃ | N | H | 2,4-F₂ | H | 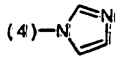 | |
| 170 | H | H | H | CH₂CH₃ | H | H | 4-CH₃ | H | 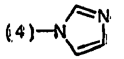 | |
| 171 | H | H | H | CH₃ | H | H | 4-CH₃ | H | 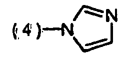 | |
| 172 | H | H | H | CH₂CH₃ | H | H | H | 3-F | 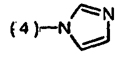 | |
| 173 | H | H | H | CH₂CH₃ | H | H | H | 3-Cl | 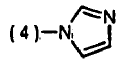 | |
| 174 | H | H | H | CH₂CH₃ | H | H | H | 2-F | 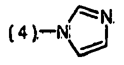 | |
| 175 | H | H | H | CH₂CH₃ | H | H | H | 2-Cl | 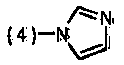 | |
| 176 | H | H | H | CH₃ | H | H | H | 3-F | 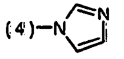 | |
| 177 | H | H | H | CH₃ | H | H | H | 3-Cl | 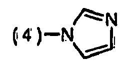 | |
| 178 | H | H | H | CH₃ | H | H | H | 2-F | 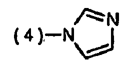 | |
| 179 | H | H | H | CH₃ | H | H | H | 2-Cl | 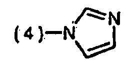 | |
| 180 | H | H | H | (CH₂)₂CH₃ | H | H | H | H | 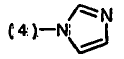 | |
| 181 | H | H | H | CH₂(CH₃)₂ | | H | H | H | 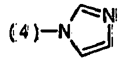 | |
| 182 | H | H | H | CH₂CH₃ | CH₃ | CH₂CH₃ | H | H | 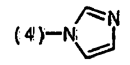 | |
| 183 | H | H | H | CH₂CH₃ | CH₂ | H | H | H | 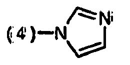 | |
| 184 | H | H | H | CH₂CH₃ | CH₃ | CH₃ | H | H | 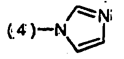 | |
| 185 | | | H | CH₂CH₃ | H | H | H | H | 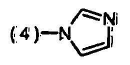 | |
| 186 | H | H | H | CH₃ | H | H | H | H | 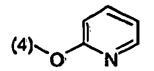 | 169-170 |
| 187 | H | H | H | CH₃ | H | H | H | 4-O(CH₂)₅CH₃ | H | 93-94 |
| 188 | H | H | H | CH₃ | H | H | H | H | 4-C(CH₃)=NOCH₃ | 106-107 |
| 189 | H | H | H | CH₂CH₃ | H | H | H | H | +C(CH₃)=NOCH₃ | |
| 190 | H | H | H | ' CH₃ | H | H | H | H | 4-CONH₂ | 174-176 |
| 191 | H | H | H | CH₃ | H | H | 4-F | H | 4-CONH₂ | |
| 192 | CH₃ | H | H | CH₃ | H | H | H | H | 4-CONH₂ | |
| 193 | H | H | H | CH₂CH₃ | H | H | H | H | 4-CONH₂ | |
| 194 | H: | H | H | CH₂CH₂CH₃ | H | H | H | H | 4-CONH₂ | |
| 195 | H | H | H | CH₂CH₃ | CH₃ | H | H | H | 4-CONH₂ | |
| 196 | CH₃ | CH₃ | H | CH₃ | H | H | H | H | 4-CONH₂ | |
| 197 | H | H | H | CH₃ | H | H | H | H | 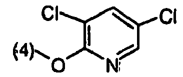 | oil |
| 198 | H | H | H | CH₃ | H | H | H | 4-(C(=NOCH₃)CH₂-CH₂-CH₂-CH₂)-3 | | 100 |
| 199 | H | H | H | CH₃ | H | H | H | 4-NHCOC(CH₃)₃ | H | 165-166 |
| 200 | H | H | H | CH₂CH₃ | H | H | H | 4-NHCOC(CH₃)₃ | H | |
| 201 | H | H | H | CH₃ | H | H | H | 4-SCH₃ | H | 102 |
| 202 | H | H | H | CH₃ | H | H | 4-F | 4-SCH₃ | H | |
| 203 | CH₃ | H | H | CH₃ | H | H | H | 4-SCH₃ | H | |
| 204 | H | H | H | CH₂CH₃ | H | H | H | 4-SCH₃ | H | |
| 205 | H | H | H | CH₂CH₂CH₃ | H | H | H | 4-SCH₃ | H | |
| 206 | H | H | H | CH₂CH₃ | CH₃ | H | H | 4-SCH₃ | H | |
| 207 | H | H | H | CH₃ | CH₃ | H | H | 4-SCH₃ | H | |
| 208 | H | H | H | CH₃ | H | H | H | 4-OCH(CH₃)CONH₂ | H | oil |
| 209 | H | H | H | CH₃ | H | H | H | H | 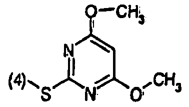 | 142 |
| 210 | H | H | H | CH₃ | H | H | H | H | 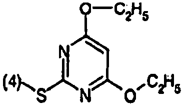 | 89-90 |
| 211 | H | H | H | CH₃ | H | H | H | 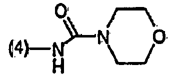 | H | 180-181 |
| 212 | H | H | H | CH₃ | H | H | 4-F | 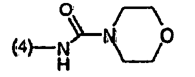 | H | |
| 213 | CH₃ | H | H | CH₃ | H | H | H | 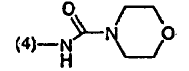 | H | |
| 214 | H | H | H | CH₂CH₃ | H | H | H | 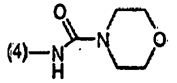 | H | |
| 215 | H | H | H | CH₂CH₂CH₃ | H | H | H | 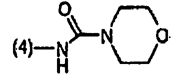 | H | |
| 216 | H | H | H | CH₂CH₃ | CH₃ | CH₂CH₃ | H | 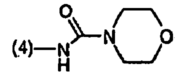 | H | |
| 217 | H | H | H | CH₂CH₃ | CH₃ | H | H | 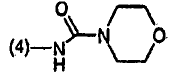 | H | |
| 218 | H | H | H | CH₂CH₃ | CH₃ | CH₃ | H | 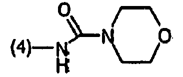 | H | |
| 219 | H | H | H | CH₃ | H | H | 4-Cl | 4-SO₂CH₃ | H | |
| 220 | H | H | H | CH₃ | H | H | H | 4-SO₂CH₃ | H | 152-153 |
| 221 | H | H | H | CH₃ | H | H | 4-F | 4-SO₂CH₃ | H | |
| 222 | CH₃ | H | H | CH₃ | H | H | H | 4-SO₂CH₃ | H | |
| 223 | H | H | H | CH₂CH₃ | H | H | H | 4-SO₂CH₃ | H | |
| 224 | H | H | H | CH₂CH₂CH₃ | H | H | H | 4-SO₂CH₃ | H | |
| 225 | H | H | H | CH₂CH₃ | CH₃ | H | H | 4-SO₂CH₃ | H | |
| 226 | H | H | H | CH₃ | H | H | H | 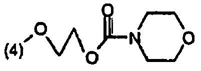 | H | 119-120 |
| 227 | H | H | H | CH₃ | H | H | H | 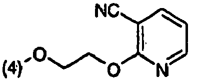 | H | oil |
| 228 | H | H | H | CH₂CH₃ | H | H | H | 4-CN | H | oil |
| 229 | H | H | H | CH₃ | H | H | H | 4-CN | H | 153-154 |
| 230 | H | H | CH₂-OCH₂CH₃ | CH₃ | H | H | H | 4-CN | H | |
| 231 | H | H | CH₂-OCH₂CH₃ | CH₂CH₃ | H | H | H | 4-CN | H | oil |
| 232 | H | H | H | CH₂CH₃ | H' | H | 4-F | 4-CN | H | |
| 233 | H | H | H | CH₃ | H | H | 4-F | 4-CN | H | |
| 234 | CH₃ | H | H | CH₂CH₃ | H | H | 4-F | 4-CN | H | |
| 235 | CH₃ | H | H: | CH₃ | H | H | 4-F | 4-CN | H | |
| 236 | CH₃ | H | H | CH₂CH₃ | H | H | H | 4-CN | H | |
| 237 | CH₃ | H | H | CH₃ | H | H | H | 4-CN | H | |
| 238 | CH₃ | H | H | CH₂CH₃ | H | H | H | 4-CN | H | |
| 239 | CH₃ | H | H | CH₃ | H | H | H | 4-CN | H | |
| 240 | CH₃ | CH₃ | H | CH₂CH₃ | CH₃ | H | H | 4-CN | H | |
| 241 | CH₃ | CH₃ | H | CH₃ | CH₃ | H | H | 4-CN | H | |
| 242 | H | H | H | CH₂CH₃ | H | H | 4-Cl | 4-CN | H | |
| 243 | H | H | H | CH₃ | H | H | 4-Cl | 4-CN | H | |
| 244 | H | H | H | CH₂CH₃ | H | H | 2-F | 4-CN | H | |
| 245 | H | H | H | CH₃ | H | H | 2-F | 4-CN | H | |
| 246 | H | H | H | CH₂CH₃ | H | H | 2,4-F₂ | 4-CN | H | |
| 247 | H | H | H | CH₃ | H | H | 2,4-F₂ | 4-CN | H | |
| 248 | H | H | H | CH₂CH₃ | H | H | 2,4-Cl₂ | 4-CN | H | |
| 249 | H | H | H | CH₃ | H | H | 2,4-Cl₂ | 4-CN | H | |
| 250 | H | H | H | CH₂CH₃ | H | H | 3,4-F₂ | 4-CN | H | |
| 251 | H | H | H | CH₃ | H | H | 3,4-F₂ | 4-CN | H | |
| 252 | H | H | H | CH₂CH₃ | H | H | 3,4-Cl₂ | 4-CN | H | |
| 253 | H | H | H | CH₃ | H | H | 3,4-Cl₂ | 4-CN | H | |
| 254 | H | H | H | CH₂CH₃ | H | H | 4-CH₃ | 4-CN | H | |
| 255 | H | H | H | CH₃ | H | H | 4-CH₃ | 4-CN | H | |
| 256 | H | H | H | CH₂CH₃ | CH₃ | CH₃ | H | 4-CN | H | |
| 257 | H | H | H | CH₃ | CH₃ | CH₃ | H | 4-CN | H | |
| 258 | H | H | H | CH₂CH₃ | CH₃; | CH₂CH₃ | H | 4-CN | H | |
| 259 | H | H | H | CH₂CH₃ | H | H | H | 3-F;4-CN | H | |
| 260 | H | H | H | CH₂CH₃ | H | H | H | 3-Cl;4-CN | H | |
| 261 | H | H | H | CH₂CH₃ | H | H | H | 2-F;4-CN | H | |
| 262 | H | H | H | CH₂CH₃ | H | H | H | 2-Cl;4-CN | H | |
| | H | H | H | CH₃ | H | H | H | 3-F;4-CN | H | |
| 264 | H | H | H | CH₃ | H | H | H | 3-Cl;4-CN | H | |
| 265 | H | H | H | CH₃ | H | H | H | 2-F;4-CN | H | |
| 266 | H | H | H | CH₃ | H | H | H | 2-Cl;4-CN | H | |
| 267 | H | H | H | (CH₂)₂CH₃ | H | H | H | 4-CN | H | |
| 268 | H | H | H | CH₂(CH₃)₂ | H | H | H | 4-CN | H | |
| 269 | H | H | H | CH₂F | H | H | H | 4-CN | H | |
| 270 | H | H | H | CH₃ | H | H | H | 2-CH₃;4-CN | H | |
| 271 | H | H | H | CH₃ | H | H | H | H | 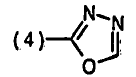 | 137-138 |
| 273 | H | H | CH₂-OCH₂CH₃ | CH₃ | H | H | H | H | 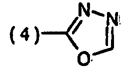 | |
| 274 | H | H | CH₂-OCH₂CH₃ | CH₂CH₃ | H | H | H | H | 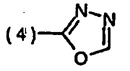 | |
| 275 | H | H | H | CH₃ | H | H | 4-F | H | 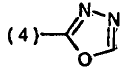 | |
| 276 | CH₃ | H | H | CH₃ | H | H | H | H | 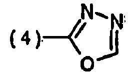 | |
| 277 | H | H | H | CH₂CH₃ | H | H | H | H | 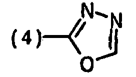 | 88-89 |
| 278 | H | H | H | CH₂CH₂CH₃ | H | H | H | H | 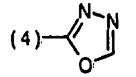 | |
| 279 | H | H | H | CH₂CH₃ | CH₃ | CH₂CH₃ | H | H | 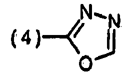 | |
| 280 | H | H | H | CH₂CH₃ | CH₃ | H | H | H | 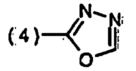 | |
| 281 | H | H | H | CH₂CH₃ | CH₃ | CH₃ | H | H | 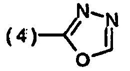 | |
| 282 | H | H | H | CH₃ | H | H | 4-Cl | H | 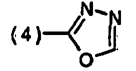 | |
| 283 | H | H | H | CH₃ | H | H | H | 4-SOCH₃ | H | 160-162 |
| 284 | H | H | H | CH₂CH₃ | H | H | H | H | 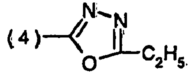 | 88-91 |
| 285 | H | H | CH₂-OCH₂CH₃ | CH₃ | H | H | H | H | 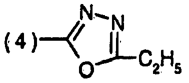 | |
| 286 | H | H | CH₂-OCH₂CH₃ | CH₂CH₃ | H | H | H | H | 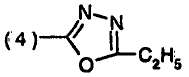 | |
| 287 | CH₃ | H | CH₂-OCH₂CH₃ | CH₂CH₃ | H | H | H | H | 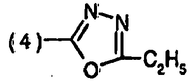 | |
| 288 | H | H | H | CH₂CH₃ | H | H | H | 4-OCH₂CH₃ | H | 85-86 |
| 289 | H | H | H | CH₃ | H | H | H | 4-OCH₂CH₃ | H | 84-85 |
| 290 | H | H | CH₂-OCH₂CH₃ | CH₃ | H | H | H | 4-OCH₂CH₃ | H | |
| 291 | H | H | CH₂-OCH₂CH₃ | CH₂CH₃ | H | H | H | 4-OCH₂CH₃ | H | |
| 292 | H | H | H | CH₂CH₃ | H | H | 4-F | 4-OCH₂CH₃ | H | |
| 293 | H | H | H | CH₃ | H | H | 4-F | 4-OCH₂CH₃ | H | |
| 294 | CH₃ | H | H | CH₂CH₃ | H | H | 4-F | 4-OCH₂CH₃ | H | |
| 295 | CH₃ | H | H | CH₃ | H | H | 4-F | 4-OCH_{2C}H₃ | H | |
| 296 | CH₃ | H | H | CH₂CH₃ | H | H | H | 4-OCH₂CH₃ | H | |
| 297 | CH₃ | H | H | CH₃ | H | H | H | 4-OCH₂CH₃ | H | |
| 298 | CH₃ | H | H | CH₂CH₃ | H | H | H | 4-OCH₂CH₃ | H | |
| 299 | CH₃ | H | H | CH₃ | H | H | H | 4-OCH₂CH₃ | H | |
| 300 | CH₃ | CH₃ | H | CH₂CH₃ | CH₃ | H | H | 4-OCH₂CH₃ | H | |
| 301 | CH₃ | CH₃ | H | CH₃ | CH₃ | H | H | 4-OCH₂CH₃ | H | |
| 302 | H | H | H | CH₂CH₃ | H | H | 4-Cl | 4-OCH₂CH₃ | H | |
| 303 | H | H | H | CH₃ | H | H | 4-Cl | 4-OCH₂CH₃ | H | |
| 304 | H | H | H | CH₂CH₃ | H | H | 2-F | 4-OCH₂CH₃ | H | |
| 305 | H | H | H | CH₃ | H | H | 2-F | 4-OCH₂CH₃ | H | |
| 306 | H | H | H | CH₂CH₃ | H | H | 2,4-F₂ | 4-OCH₂CH₃ | H | |
| 307 | H | H | H | CH₃ | H | H | 2,4-F₂ | 4-OCH₂CH₃ | H | |
| 308 | H | H | H | CH₂CH₃ | H | H | 2,4-Cl₂ | 4-OCH₂CH₃ | H | |
| 309 | H | H | H | CH₃ | H | H | 2,4-Cl₂ | 4-OCH₂CH₃ | H | |
| 310 | H | H | H | CH₂CH₃ | H | H | 3,4-F₂ | 4-OCH₂CH₃ | H | |
| 311 | H | H | H | CH₃ | H | H | 3,4-F₂ | 4-OCH₂CH₃ | H | |
| 312 | H | H | H | CH₂CH₃ | H | H | 3,4-Cl₂ | 4-OCH₂CH₃ | H | |
| 313 | H | H | H | CH₃ | H | H | 3,4-Cl₂ | 4-OCH₂CH₃ | H | |
| 314 | H | H | H | CH₂CH₃ | H | H | 4-CH₃ | 4-OCH₂CH₃ | H | |
| 315 | H | H | H | CH₃ | H | H | 4-CH₃ | 4-OCH₂CH₃ | H | |
| 316 | H | H | H | CH₂CH₃ | CH₃ | CH₃ | H | 4-OCH₂CH₃ | H | |
| 317 | H | H | H | CH₃ | CH₃ | CH₃ | H | 4-OCH₂CH₃ | H | |
| 318 | H | H | H | CH₂CH₃ | CH₃ | CH₂CH₃ | H | 4-OCH₂CH₃ | H | |
| 319 | H | H | H | CH₂CH₃ | H | H | H: | 3-F;4.OCH₂CH₃ | H | |
| 320 | H | H | H | CH₂CH₃ | H | H | H | 3-Cl;4-OCH₂CH₃ | H | |
| 321 | H | H | H | CH₂CH₃ | H' | H | H | 2-F;4-OCH₂CH₃ | H | |
| 322 | H | H | H | CH₂CH₃ | H | H | H | 2-Cl;4-OCH₂CH₃ | H | |
| 323 | H | H | H | CH₃ | H | H | H | 3-F;4-OCH₂CH₃ | H | |
| 324 | H | H | H | CH₃ | H | H | H | 3-Cl;4-OCH₂CH₃ | H | |
| 325 | H | H | H | CH₃ | H | H | H | 2-F;4-OCH₂CH₃ | H | |
| 326 | H | H | H | CH₃ | H | H | H | 2-Cl;4-OCH₂CH₃ | H | |
| 327 | H | H | H | (CH₂)₂CH₃ | H | H | H | 4-OCH₂CH₃ | H | |
| 328 | H | H | H | CH₂(CH₃)₂ | H | H | H | 4-OCH₂CH₃ | H | |
| 329 | H | H | H | CH₂F | H | H | H | 4-OCH₂CH₃ | H | |
| 330 | H | H | H | CH₃ | CH₂CH₃ | CH₂CH₃ | H | 4-OCH₂CH₃ | H | |
| 331 | H | H | H | CH₂CH₃ | H | H | H | H | 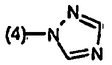 | oil |
| 332 | H | H | H | CH₃ | H | H | H | H | 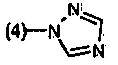 | 103-105 |
| 333 | H | H | CH₂-OCH₂CH₃ | CH₃ | H | H | H | | 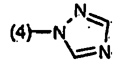 | |
| 334 | H | H | CH₂-OCH₂CH₃ | CH₂CH₃ | H | H | H | H | 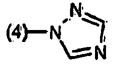 | |
| 335 | H | H | H | CH₂CH₃ | H | H | 4-F | H | 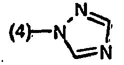 | |
| 336 | H | H | H | CH₃ | H | H | 4-F | H | 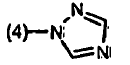 | |
| 337 | CH₃ | H | H | CH₂CH₃ | H | H | 4-F | H | 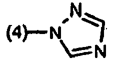 | |
| 338 | CH₃ | H | H | CH₃ | H | H | 4-F | H | 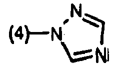 | |
| 339 | CH₃ | H | H | CH₂CH₃ | H | H | H | H | 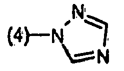 | |
| 340 | CH₃ | H | H | CH₃ | H | H | H | H | 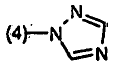 | |
| 341 | CH₃ | H | H | CH₂CH₃ | H | H | H | H | 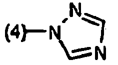 | |
| 342 | CH₃ | H | H | CH₃ | H | H | H | H | 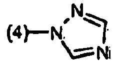 | |
| 343 | CH₃ | CH₃ | H | CH₂CH₃ | CH₃ | H | H | H | 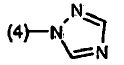 | |
| 344 | CH₃ | CH₃ | H | CH₃ | CH₃ | H | H | H | 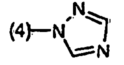 | |
| 345 | H | | H | CH₂CH₃ | H | H | 4-Cl | H | 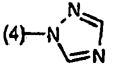 | |
| 346 | H | H | H | CH₃ | H | H | 4-Cl | H | 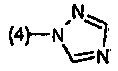 | |
| 347 | H | H | H | CH₂CH₃ | H | H | 2-F | H | 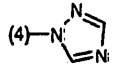 | |
| 348 | H | H | H | CH₃ | H | H | 2-F | H | 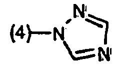 | |
| 349 | H | H | H' | CH₂CH₃ | H | H | 2,4-F₂ | H | 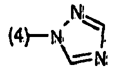 | |
| 350 | H | H | H | CH₃ | H | H | 2,4-F₂ | H | 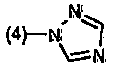 | |
| 351 | H | H | H | CH₂CH₃ | H | H | 2,4-Cl₂ | H | 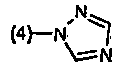 | |
| 352 | H | H | H | CH₃ | H | H | 2,4-Cl₂ | H | 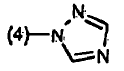 | |
| 353 | H | H | H | CH₂CH₃ | H | H | 3,4-F₂ | H | 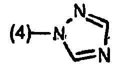 | |
| 354 | H | H | H | CH₃ | H | H | 3,4-F₂ | H | 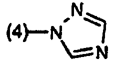 | |
| 355 | H | H | H | CH₂CH₃ | H | H | 3,4-Cl₂ | H | 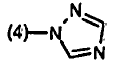 | |
| 356 | H | H | H | CH₃ | H | H | 3,4-Cl₂ | H | 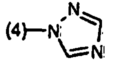 | |
| 357 | H | H | H | CH₂CH₃ | H | H | 4-CH₃ | H | 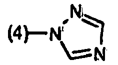 | |
| 358 | H | H | H | CH₃ | H | H | 4-CH₃ | H | 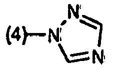 | |
| 359 | H | H | H | CH₂CH₃ | CH₃ | CH₃ | H | H | 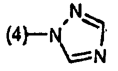 | |
| 360 | H | H | H | CH₃ | CH₃ | CH₃ | H | H | 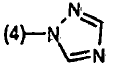 | |
| 361 | H | H | H | CH₂CH₃ | CH₃ | CH₂CH₃ | H | H | 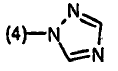 | |
| 362 | H | H | H | CH₂CH₃ | H | H | H | 3-F | 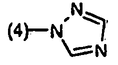 | |
| 363 | H | H | H | CH₂CH₃ | H | H | H | 3-Cl | 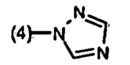 | |
| 364 | H | H | H | CH₂CH₃ | H | H | H | 2-F | 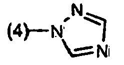 | |
| 365 | H | H | H | CH₂CH₃ | H | H | H | 2-Cl | 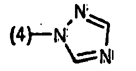 | |
| 366 | H | H | H | CH₃ | H | H | H | 3-F | 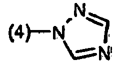 | |
| 367 | H | H | H | CH₃ | H | H | H | 3-Cl | 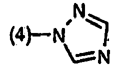 | |
| 368 | H | H | H | CH₃ | H | H | H | 2-F | 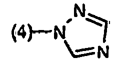 | |
| 369 | H | H | H | CH₃ | H | H | H | 2-Cl | 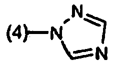 | |
| 370 | H | H | H | (CH₂)₂CH₃ | H | H | H | H | 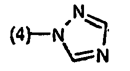 | |
| 371 | H | H | H | CH₂(CH₃)₂ | H | H | H | H | 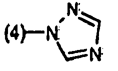 | |
| 372 | H | H | H | CH₂F | H | H | H | H | 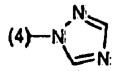 | |
| 373 | H | H | CH₂-OCH₃ | CH₃ | H | H | H | H | 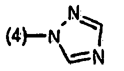 | |
| 374 | H | H | CH₂-OCH₃ | CH₂CH₃ | H | H | H | H | 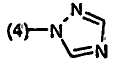 | |
| 375 | H | H | H | CH₃ | H | H | H | H | 4-COOCH₃ | 13-134 |
| 376 | H | H | CH₂-OCH₂CH₃ | CH₃ | H | H | H | H | 4-COOCH₃ | |
| 377 | H | H | CH₂-OCH₂CH₃ | CH₂CH₃ | H | H | H | H | 4-COOCH₃ | |
| 378 | H | H | H | CH₃ | H | H | H | H | 4-COC₆H₅ | solid |
| 379 | H | H | H | CH₂CH₃ | H | H | H | H | 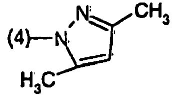 | oil |
| 380 | H | H | H | CH₃ | H | H | H | H | 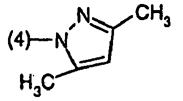 | |
| 381 | H | H | H | CH₂CH₃ | H | H | H | H | 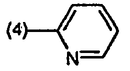 | 124-125 |
| 382 | H | H | H | CH₃ | H | H | 4-F | H | 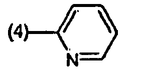 | |
| 383 | CH₃ | H | H | CH₃ | H | H | H | H | 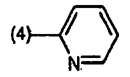 | |
| 384 | H | H | H | CH₃ | H | H | H | H | 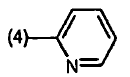 | |
| 385 | H | H | H | CH₂CH₂CH₃ | H | H | H | H | 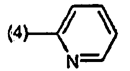 | |
| 386 | H | H | H | CH₂CH₃ | CH₃ | CH₂CH₃ | H | H | 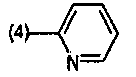 | |
| 387 | H | H | H | CH₂CH₃ | CH₃ | H | H | H | 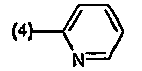 | |
| 388 | H | H | H | CH₂CH₃ | CH₃ | CH₃ | H | H | 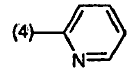 | |
| 389 | CH₃ | H | H | CH₂CH₃ | CH₃ | CH₃ | H | H | 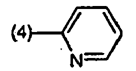 | |
| 390 | H | H | H | CH₂CH₃ | H | H | H | H | 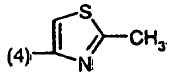 | |
| 391 | H | H | H | CH₂CH₃ | H | H | H | H | 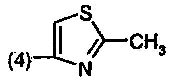 | |
| 392 | H | H | H | CH₃ | H | H | H | H | 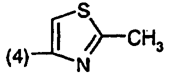 | |
| 393 | H | H | CH₂-OCH₂CH₃ | CH₃ | H | H | H | H | 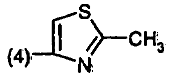 | |
| 394 | H | H | CH₂-OCH₂CH₃ | CH₂CH₃ | H | H | H | H | 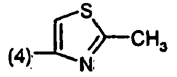 | |
| 395 | H | H | H | CH₂CH₃ | H | H | 4-F | H | 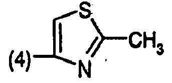 | |
| 396 | H | H | H | CH₃ | H | H | 4-F | H | 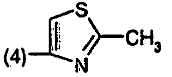 | |
| 397 | CH₃ | H | H | CH₃ | H | H | H | H | 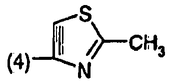 | |
| 398 | CH₃ | H | H | CH₂CH₃ | H | H | H | H | 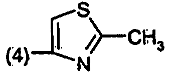 | |
| 399 | H | H | H | CH₂CH₃ | H | H | 4-Cl | H | 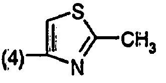 | |
| 400 | H | H | H | CH₃ | H | H | 4-Cl | H | 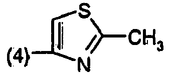 | |
| 401 | H | H | H | CH₂CH₃ | H | H | 2-F | H | 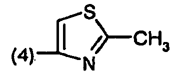 | |
| 402 | H | H | H | CH₃ | H | H | 2-F | H | 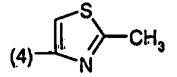 | |
| 403 | H | H | H | CH₂CH₃ | H | H | 2,4-F₂ | H | 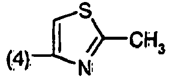 | |
| 404 | H' | H' | H | CH₃ | H | H | 2,4-F₂ | H | 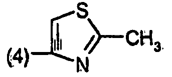 | |
| 405 | H | H | H | CH₂CH₃ | H | H | 4-CH₃ | H | 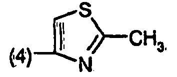 | |
| 406 | H | H | H | CH₃ | H | H | 4-CH₃ | H | 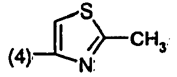 | |
| 407 | H | H | H | CH₂CH₃ | H | H | H | 3-F | 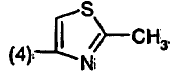 | |
| 408 | H | H | H | CH₂CH₃ | H | H | H | 3-Cl | 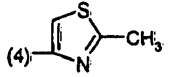 | |
| 409 | H | H | H | CH₂CH₃ | H | H | H | 2-F | 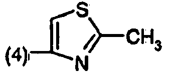 | |
| 410 | H | H | H | CH₂CH₃ | H | H | H | 2-Cl | 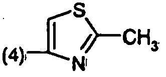 | |
| 411 | H | H | H | CH₃ | H | H | H | 3-F | 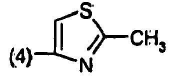 | |
| 412 | H | H | H | CH₃ | H | H | H | 3-Cl | 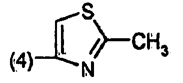 | |
| 413 | H: | H | H | CH₃ | H | H | H | 2-F | 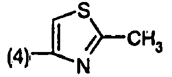 | |
| 414 | H | H | H | CH₃ | H | H | H | 2-Cl | 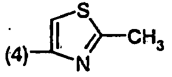 | |
| 415 | H | H | H | (CH₂)₂CH₃ | H | H | H | H | 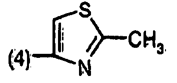 | |
| 416 | H | H | H | CH₂(CH₃)₂ | H | H | H | H | 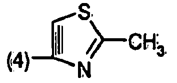 | |
| 417 | H | H | H | CH₂CH₃ | CH₃ | CH₂CH₃ | H | H | 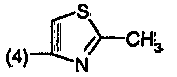 | |
| 418 | H | H | H | CH₂CH₃ | CH₃ | H | H | H | 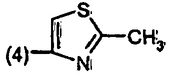 | |
| 419 | H | H | H | CH₂CH₃ | CH₃ | CH₃ | H | H | 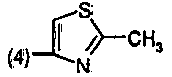 | |
| 420 | H | H | H | CH₂CH₃ | CH₂CH₃ | CH₂CH₃ | H | H | 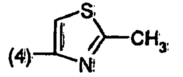 | |
| 421 | H | H | H | CH₂CH₃ | H | H | H | H | 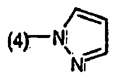 | solid |
| 422 | H | H | H | CH₃ | H | H | H | H | 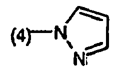 | solid |
| 423 | H | H | CH₂-OCH₂CH₃ | CH₃ | H | H | H | H | 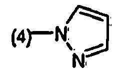 | |
| 424 | H | H | CH₂-OCH₂CH₃ | CH₂CH₃ | H | H | H | H | 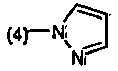 | |
| 425 | H | H | H | CH₂CH₃ | H | H | 4-F | H | 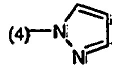 | |
| 426 | H | H | H | CH₃ | H | H | 4-F | H | 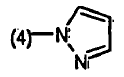 | |
| 427 | CH₃ | H | H | CH₃ | H | H | H | H | 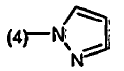 | |
| 428 | CH₃ | H | H | CH₂CH₃ | H | H | H | H | 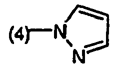 | |
| 429 | H | H | H | CH₂CH₃ | H | H | 4-Cl | H | 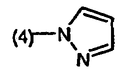 | |
| 430 | H | H | H | CH₃ | H | H | 4-Cl | H | 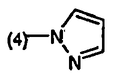 | |
| 431 | H | H | H | CH₂CH₃ | H | H | 2-F | H | 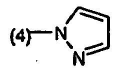 | |
| 432 | H | H | H | CH₃ | H | H | 2-F | H | 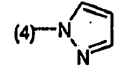 | |
| 433 | H | H | H | CH₂CH₃ | H | H | 2,4-F₂ | H | 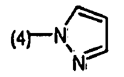 | |
| 434 | H | H | H | CH₃ | H | H | 2,4-F₂ | H I | 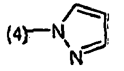 | |
| 435 | H | H | H | CH₂CH₃ | H | H | 4-CH₃ | H | 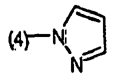 | |
| 436 | H | H | H | CH₃ | H | H | 4-CH₃ | H | 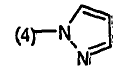 | |
| 437 | H | H | H | CH₂CH₃ | H | H | H | 3-F | 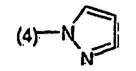 | |
| 438 | H | H | H | CH₂CH₃ | H | H | H | 3-Cl | 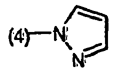 | |
| 439 | H | H | H | CH₂CH₃ | H | H | H | 2-F | 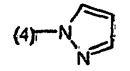 | |
| 440 | H | H | H | CH₂CH₃ | H | H | H | 2-Cl | 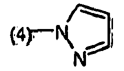 | |
| 441 | H | H | H | CH₃ | H | H | H | 3-F | 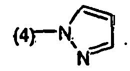 | |
| 442 | H | H | H | CH₃ | H | H | H | 3-Cl | 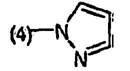 | |
| 443 | H | H | H | CH₃ | H | H | H | 2-F | 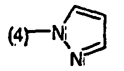 | |
| 444 | H | H | H | CH₃ | H | H | H | 2-Cl | 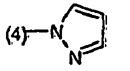 | |
| 445 | H | H | H | (CH₂)₂CH₃ | H | H | H | H | 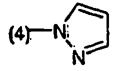 | |
| 446 | H | H | H | CH₂(CH₃)₂ | H | H | H | H | 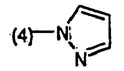 | |
| 447 | H | H | H | CH₂CH₃ | CH₃ | CH₂CH₃ | H | H | 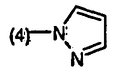 | |
| 448 | H | H | H | CH₂CH₃ | CH₃ | H | H | H | 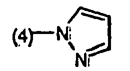 | |
| 449 | H | H | H | CH₂CH₃ | CH₃ | CH₃ | H | H | 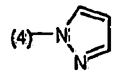 | |
| 450 | CH₃ | CH₃ | H | CH₂CH₃ | H | H | H | H | 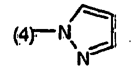 | |
| 451 | H | H | H | CH₃ | H | H | H | 4-(COCH₂-CH₂-CH₂-CH₂)-3 | | 159-160 |
| 452 | H | H | H | CH₂CH₃ | H | H | H | H | 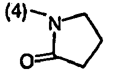 | 136-137 |
| 453 | H | H | H | CH₂CH₃ | H | H | H | 4-(COCH₂-CH₂-CH₂-CH₂)-3 | | 117-119 |
| 454 | H | H | H | CH₂CH₃ | H | H | H | H | 4-CON(CH₃)₂ | 148-149 |
| 455 | H | H | H | CH₂CH₃ | H | H | H | H | 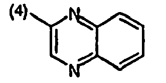 | 112-113 |
| 456 | H | H | H | CH₃ | ! H | H | H | 4-OCOCH₃ | H | 143-145 |
| 457 | H | H | H | CH₃ | H | H | H | 4-OH | H | 120-121 |
| 458 | H | H | CH₃ | CH₃ | H | H | H | 4-OH | H | resinous |
| 459 | H | H | H | CH₃ | H | H | 4-F | 4-OH | H | |
| 460 | CH₃ | H | H | CH₃ | H | H | H | 4-OH | H | |
| 461 | H | H | H | CH2CH₃ | H | H | H | 4-OH | H | |
| 462 | H | H | H | CH₂CH₂CH₃ | H | H | H | 4-OH | H | |
| 463 | H | H | H | CH₂CH₃ | CH₃ | CH₂CH₃ | H | 4-OH | H | |
| 464 | H | H | H | CH₂CH₃ | CH₃ | H | H | 4-OH | H | |
| 465 | H | H | H | CH₂CH₃ | CH₃ | CH₃ | H | 4-OH | H | |
| 466 | CH₃ | CH₃ | H | CH₂CH₃ | H | H | H | 4-OH | H | |
| 467 | H | H | H | CH₂CH₃ | H | H | H | 4-(C(CH₃)=C-C(O)-O)-3 | | 190-191 |
| 468 | H | H | H | CH₂CH₃ | H | H | H | 4-(CH=CH-C(O)-O)-3 | | 131-132 |
| 469 | H | H | H | CH₂CH₃ | H | H | H | 4-OCH(CH₃)₂ | H | 98-99 |
| 470 | H | H | H | CH₃ | H | H | 4-F | ; 4-OCH(CH₃)₂ | H | |
| 471 | CH₃ | H | H | CH₃ | H | H | H | 4-OCH(CH₃)₂ | H | |
| 472 | H | H | H | CH₃ | H | H | H | 4-OCH(CH₃)₂ | H | |
| 473 | H | H | H | CH₂CH₂CH₃ | H | H | H | 4-OCH(CH₃)₂ | H | |
| 474 | H | H | H | CH₂CH₃ | CH₃ | CH₂CH₃ | H | 4-OCH(CH₃)₂ | H | |
| 475 | H | H | H | CH₂CH₃ | CH₃ | H | H | 4-OCH(CH₃)₂ | H | |
| 476 | H | H | H | CH₂CH₃ | CH₃ | CH₃ | H | 4-OCH(CH₃)₂ | H | |
| 477 | CH₃ | CH₃ | H | CH₃ | H | H | H | 4-OCH(CH₃)₂ | H | |
| 478 | H | H | H | CH₂CH₃ | H | H | H | 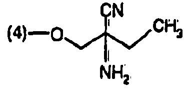 | H | 108-109 |
| 479 | H | H | H | CH₂CH₃ | H | H | H | 4-NO₂ | H | glass |
| 480 | H | H | H | CH₂CH₃ | H | H | H | 4-NH₂ | H | 153-154 |
| 481 | H | H | H | CH₃ | H | H | H | 4-NH₂ | H | |
| 482 | H | H | H | CH₃ | H | H | 4-F | 4-NH₂ | H | |
| 483 | CH₃ | H | H | CH₃ | H | H | H | 4-NH₂ | H | |
| 484 | H | H | H | CH₂CH₂CH₃ | H | H | H | 4-NH₂ | H | |
| 485 | H | H | H | CH₂CH₃ | CH₃ | CH₂CH₃ | H | 4-NH₂ | H | |
| 486 | H | H | H | CH₂CH₃ | CH₃ | H | H | 4-NH₂ | H | |
| 487 | H | H | H | CH₂CH₃ | CH₃ | CH₃ | H | 4-NH₂ | H | |
| 488 | CH₃ | H | H | CH₂CH₃ | H | H | H | 4-NH₂ | H | |
| 489 | H | H | H | CH₂CH₃ | H | H | H | 4-I | H | resinous |
| 490 | H | H | H | CH₃ | H | H | H | 4-I | H | |
| 491 | H | H | H | CH₃ | H | H | 4-F | 4-I | H | |
| 492 | CH₃ | H | H | CH₃ | H | H | H | 4-I | H | |
| 493 | H | H | H | CH(CH₃)₂ | H | H | H. | 4-I | H | |
| 494 | H | H | H | CH₂CH₂CH₃ | H | H | H | 4-I | H | |
| 495 | H | H | H | CH₂CH₃ | CH₃ | CH₂CH₃ | H | 4-I | H | |
| 496 | H | H | H | CH₂CH₃ | CH₃ | H | H | 4-I | H | |
| 497 | H | H | H | CH₂CH₃ | CH₃ | CH₃ | H | 4-I | H | |
| 498 | H | H | H | CH₂F | H | H | H | 4-I | H | |
| 499 | H | H | H | CH₂CH₃ | H | H | H | 4-Br | H | |
| 500 | H | H | H | CH₃ | H | H | H | 4-Br | H | |
| 501 | H | H | H | CH₃ | H | H | 4-F | 4-Br | H | |
| 502 | CH₃ | H | H | CH₃ | H | H | H | 4-Br | H | |
| 503 | H | H | H | CH(CH₃)₂ | H | H | H | 4-Br | H | |
| 504 | H | H | H | CH₂CH₂CH₃ | H | H | H | 4-Br | H | |
| 505 | H | H | H | CH₂CH₃ | CH₃ | CH₂CH₃ | H | 4-Br | H | |
| 506 | H | H | H | CH₂CH₃ | CH₃ | H | H | 4-Br | H | |
| 507 | H | H | H | CH₂CH₃ | CH₃ | CH₃ | H | 4-Br | H | |
| 508 | H | H | H | CH₂F | H | H | H | 4-Br | H | |
| 509 | H | H | H | CH₃ | H | H | H | 4-CH(CH₃)₂ | H | resinous |
| 510 | H | H | H | CH₂CH₃ | H | H | H | 4-OCH₂C₆H₅ | H | resinous |
| 511 | H | H | H | CH₃ | H | H | H | 4-OCH₂C₆H₅ | H | |
| 512 | H | H | H | CH₃ | H | H | H | 3-F | 4-COCH₂CH₃ | 103-104 |
| 513 | H | H | H | CH₂CH₃ | H | H | H | 3-F | 4-COCH₂CH₃ | 114-118 |
| 514 | H | H | H | CH₂CH₃ | H | H | H | H | 4-COCH₂CH₃ | oil |
| 515 | H | H | CH₂-OCH₂CH₃ | CH₃ | H | H | H | H | 4-COCH₂CH₃ | |
| 516 | H | H | CH₂-OCH₂CH₃ | CH₂CH₃ | H | H | H | H | 4-COCH₂CH₃ | |
| 517 | H | H | H | CH₃ | H | H | H | H | 4-COCH2CH3 | 124-126 |
| 518 | H | H | H | CH₂CH₃ | H | H | 4-F | H | 4-COCH₂CH₃ | |
| 519 | H | H | H | CH₃ | H | H | 4-F | H | 4-COCH₂CH₃ | |
| 520 | CH₃ | H | H | CH₂CH₃ | H | H | 4-F | H | 4-COCH₂CH₃ | |
| 521 | CH₃ | H | H | CH₃ | H | H | 4-F | H | 4-COCH₂CH₃ | |
| 522 | CH₃ | H | H | CH₂CH₃ | H | H | H | H | 4-COCH₂CH₃ | |
| 523 | CH₃ | H | H | CH₃ | H | H | H | H | 4-COCH₂CH₃ | |
| 524 | CH₃ | H | H | CH₂CH₃ | H | H | H | H | 4-COCH₂CH₃ | |
| 525 | CH₃ | H | H | CH₃ | H | H | H | H | 4-COCH₂CH₃ | |
| 526 | CH₃ | CH₃ | H | CH₂CH₃ | CH₃. | H | H | H | 4-COCH₂CH₃ | |
| 527 | CH₃ | CH₃ | H | CH₃ | CH₃ | H | H | H | 4-COCH₂CH₃ | |
| 528 | H | H | H | CH₂CH₃ | H | H | 4-Cl | H | 4-COCH₂CH₃ | |
| 529 | H | H | H | CH₃ | H | H | 4-Cl | H | 4-COCH₂CH₃ | |
| 530 | H | H | H | CH₂CH₃ | H | H | 2-F | H | 4-COCH₂CH₃ | |
| 531 | H | H | H | CH₃ | H | H | 2-F | H | 4-COCH₂CH₃ | |
| 532 | H | H | H | CH₂CH₃ | H | H | 2,4-F₂ | H | 4-COCH₂CH₃ | |
| 533 | H | H | H | CH₃ | H | H | 2,4-F₂ | H | 4-COCH₂CH₃ | |
| 534 | H | H | H | CH₂CH₃ | H | H | 2,4-Cl₂ | H | 4-COCH₂CH₃ | |
| 535 | H | H | H | CH₃ | H | H | 2,4-Cl₂ | H | 4-COCH₂CH₃ | |
| 536 | H | H | H | CH₂CH₃ | H | H | 3,4-F₂ | H | 4-COCH₂CH₃ | |
| 537 | H | H | H | CH₃ | H | H | 3,4-F₂ | H | 4-COCH₂CH₃ | |
| 538 | H | H | H | CH₂CH₃ | H | H | 3,4-Cl₂ | H | 4-COCH₂CH₃ | |
| 539 | H | H | H | CH₃ | H | H | 3,4-Cl₂ | H | 4-COCH₂CH₃ | |
| 540 | H | H | H | CH₂CH₃ | H | H | 4-CH₃ | H | 4-COCH₂CH₃ | |
| 541 | H | H | H | CH₃ | H | H | 4-CH₃ | H | 4-COCH₂CH₃ | |
| 542 | H | H | H | CH₂CH₃ | CH₃ | CH₃ | H | H | 4-COCH₂CH₃ | |
| 543 | H | H | H | CH₃ | CH₃ | CH₃ | H | H | 4-COCH₂CH₃ | |
| 544 | H | H | H | CH₂CH₃ | CH₃ | CH₂CH₃ | H | H | 4-COCH₂CH₃ | |
| 545 | H | H | H | CH2CH₃ | H | H | H | 3-F | 4-COCH₂CH₃ | |
| 546 | H | H | H | CH₂CH₃ | H | H | H | 3-Cl | 4-COCH₂CH₃ | |
| 547 | H | H | H | CH₂CH₃ | H | H | H | 2-F | 4-COCH₂CH₃ | |
| 548 | H | H | H | CH₂CH₃ | H | H | H | 2-Cl | 4-COCH₂CH₃ | |
| 549 | H | H | H | CH₃ | | H | H | 3-F | 4-COCH₂CH₃ | |
| 550 | H | H | H | CH₃ | H | H | H | 3-Cl | 4-COCH₂CH₃ | |
| 551 | H | H | H | CH₃ | H | H | H | 2-F | 4-COCH₂CH₃ | |
| 552 | H | H | H | CH₃ | H | H | H | 2-Cl | 4-COCH₂CH₃ | |
| 553 | H | H | H | (CH₂)₂CH₃ | H | H | H | H | 4-COCH₂CH₃ | |
| 554 | H | H | H | CH₂(CH₃)₂ | H | H | H | H | 4-COCH₂CH₃ | |
| 555 | H | H | H | CH₂F | H | H | H | H | 4-COCH₂CH₃ | |
| 556 | H | H | H | CH₃ | H | H | H | H | 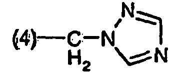 | |
| 557 | H | H | H | CH₂CH₃ | H | H | H | H | 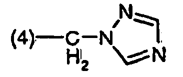 | solid |
| 558 | H | H | H | CH₂CH₃ | H | H | H | H | 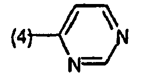 | solid |
| 559 | H | H | H | CH₃ | H | H | 4-F | H | 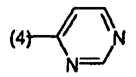 | |
| 560 | CH₃ | H | H | CH₃ | H | H | H | H | 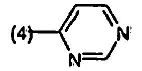 | |
| 561 | H | H | H | CH₃ | H | H | H | H | 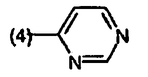 | |
| 562 | H | H | H | CH₂CH₂CH₃ | H | H | H | H | 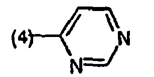 | |
| 563 | H | H | H | CH₂ CH₂CH₂CH₃ | H | H | H | H | 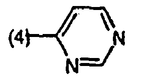 | |
| 564 | H | H | H | CH₂CH₃ | H | H | H | H | 4-COCH3 | oil |
| 565 | H | H | H | CH₃ | CH₃ | H | H | H | 4-COCH₃ | oil |
| 566 | H | H | CH₂-OCH₂CH₃ | CH₃ | H | H | H | H | 4-COCH₃ | |
| 567 | H | H | CH₂-OCH₂CH₃ | CH₂CH₃ | H | H | H | H | 4-COCH₃ | |
| 568 | H | H | H | CH₃ | H | H | H | H | 4-COCH₃ | 143-144 |
| 569 | H | H | H | CH₂CH₃ | H | H | 4-F | H | 4-COCH₃ | |
| 570 | H | H | H | CH₃ | H | H | 4-F | H | 4-COCH₃ | |
| 571 | CH₃ | H | H | CH₂CH₃ | H | H | 4-F | H | 4-COCH₃ | |
| 572 | CH₃ | H | H | CH₃ | H | H | 4-F | H | 4-COCH₃ | |
| 573 | CH₃ | H | H | CH₂CH₃ | H | H | H | H | 4-COCH₃ | |
| 574 | CH₃ | H | H | CH₃ | H | H | H | H | 4-COCH₃ | |
| 575 | CH₃ | H | H | CH₂CH₃ | H | H | H | H | 4-COCH₃ | |
| 576 | CH₃ | H | H | CH₃ | H | H | H | H | 4-COCH₃ | |
| 577 | CH₃ | CH₃ | H | CH₂CH₃ | CH₃ | H | H | H | 4-COCH₃ | |
| 578 | CH₃ | CH₃ | H | CH₃ | CH₃ | H | H | H | 4-COCH₃ | |
| 579 | H | H | H | CH₂CH₃ | H | H | 4-Cl | H | 4-COCH₃ | |
| 580 | H | H | H | CH₃ | H | H | 4-Cl | H | 4-COCH₃ | |
| 581 | H | H | H | CH₂CH₃ | H | H | 2-F | H | 4-COCH₃ | |
| 582 | H | H | H | CH₃ | H | H | 2-F | H | 4-COCH₃ | |
| 583 | H | H | H | CH₂CH₃ | H | H | 2,4-F₂ | H | 4-COCH₃ | |
| 584 | H | H | H | CH₃ | H | H | 2,4-F₂ | H | 4-COCH₃ | |
| 585 | H | H | H | CH₂CH₃ | H | H | 2,4-Cl₂ | H | 4-COCH₃ | |
| 586 | H | H | H | CH₃ | H | H | 2,4-Cl₂ | H | 4-COCH₃ | |
| 587 | H | H | H | CH₂CH₃ | H | H | 3,4-F₂ | H | 4-COCH₃ | |
| 588 | H | H | H | CH₃ | H | H | 3,4-F₂ | H | 4-COCH₃ | |
| 589 | H | H | H | CH₂CH₃ | H | H | 3,4-Cl2. | H | 4-COCH₃ | |
| 590 | H | H | H | CH₃ | H | H | 3,4-Cl₂ | H | 4-COCH₃ | |
| 591 | H | H | H | CH₂CH₃ | H | H | 4-CH₃ | H | 4-COCH₃ | |
| 592 | H | H | H | CH₃ | H | H | 4-CH₃ | H | 4-COCH₃ | |
| 593 | H | H | H | CH₂CH₃ | CH₃ | CH₃ | H | H | 4-COCH₃ | |
| 594 | H | H | H | CH₃ | CH2 | CH₃ | H | H | 4-COCH₃ | |
| 595 | H | H | H | CH₂CH₃ | CH₃ | CH₂CH₃ | H | H | 4-COCH₃ | |
| 596 | H | H | H | CH₂CH₃ | H | H | H | 3-F | 4-COCH₃ | |
| 597 | H | H | H | CH₂CH₃ | H | H | H | 3-Cl | 4-COCH₃ | |
| 598 | H | H | H | CH₂CH₃ | H | H | H | 2-F | 4-COCH₃ | |
| 599 | H | H | H | CH₂CH₃ | H | H | H | 2-Cl | 4-COCH₃ | |
| 600 | H | H | H | CH₃ | H | H | H | 3-F | 4-COCH₃ | |
| 601 | H | H | H | CH₃ | H | H | H | 3-Cl | 4-COCH₃ | |
| 602 | H | H | H | CH₃ | H | H | H | 2-F | 4-COCH₃ | |
| 603 | H | H | H | CH₃ | H | H | H | 2-Cl | 4-COCH₃ | |
| 604 | H | H | H | (CH₂)₂CH3 | H | H | H | H | 4-COCH₃ | |
| 605 | H | H | H | CH₂(CH₃)₂ | H | H | H | H | 4-COCH₃ | |
| 606 | H | H | H | CH₂F | H | H | H | H | 4-COCH₃ | |
| 607 | H | H | H | CH₃ | H | H | H | H | 4-COCH₃ | 152-153 n_{D}²⁰ = +10.7°(CHCl₃ c1.34° |
| 608 | H | H | CH₂-OCH₂CH₃ | CH₃ | H | H | H | H | -4-COCH3 | |
| 609 | H | H | CH₂ -OCH₂CH₃ | CH₂CH₃ | H | H | H | H | -4-COCH₃ | |
| 610 | H | H | H | CH₃ | H | H | H | H | -4-COCH₃ | 152-153 nD²⁰=10.7° (CHCl₃ c1,34) |
| 611 | H | H | CH₂OCH₂CH₃ CH₂-OCH₂CH₂ | CH₃ CH₃ | H | H | H | H | -4-COCH₃ | |
| 612 | H | H | CH₂-OCH₂CH3 | CH₂CH₃ | H | H | H | H | -4-COCH₃ | |
| 613 | H | H | H | CH₃ | CH₂CH₃ ; | CH₂CH₃ | H | H | 4-COCH₃ | |
| 614 | H | H | H | CH₃ | H | H | 4-F | 3,4-(OCH₃)₂ | H | |
| 615 | CH₃ | H | H | CH3 | H | H | H | 3,4-(OCH₃)₂ | H | |
| 616 | H | H | H | CH₃ | H | H | 4-F | 3,4-(OCH₃)₂ | H | |
| 617 | H | H | H | CH₂CH₃ | H | H | H | 3,4-(OCH₃)₂ | H | |
| 618 | H | H | H | CH₂CH₂CH₃ | H | H | H | 3,4-(OCH₃)₂ | H | |
| 619 | H | H | H | CH₂CH₃ | CH₃ | H | H | 3,4-(OCH₃)₂ | H | |
| 620 | H | H | H | CH₂CH₃ | CH₃ | CH₃ | H | 3,4-(OCH₃)₂ | H | |
| 621 | H | H | H | CH₃ CH₃ | H | H | 4-F 4F | 3-OCH₃; 4-OCH₂CH=CH₂ | H | |
| 622 | CH₃ | H | H | CH₃ | H | H | H | 3-OCH₃; 4-OCH₂CH=CH₂ | H | |
| 623 | H | H | H | CH₃ | H | H | 4-F | 3-OCH₃; 4-OCH₂CH=CH₂ | H | |
| 624 | H | H | H | CH₂CH₃ | H | H | H | 3-OCH₃; 4-OCH₂CH=CH₂ | H | |
| 625 | H | H | H | CH₂CH₂CH₃ | H | H | H | 3-OCH₃; 4-OCH₂CH=CH₂ | H | |
| 626 | H | H | H | CH₂CH₃ | CH₃ | H | H | 3-OCH₃; 4-OCH₂CH=CH₂ | H | , |
| 627 | H | H | H | CH₂CH₃ | CH₃ | CH₃ | H | 3-OCH₃; 4-OCH₂CH=CH₂ | H | |
| 628 | H | H | H | CH₃ | CH₂ | H | H | 3-OCH₃; 4-OCH₂CH=CH₂ | H | |
| 629 | H | H | H | CH₂CH₃ | CH₃ | CH₂CH₃ | H | 3-OCH₃; 4-OCH₂CH=CH₂ | H | |
| 630 | H | H | H | CH₃ | H | H | 4-F | 3-OCH_{3;} 4-OCH₂C-CH | H | |
| 631 | CH₃ | H | H | CH₃ | H | H | H | 3-OCH_{3;} 4-OCH₂C₂CH | H | |
| 632 | H | H | H | CH₃ | H | H | 4-F | 3-OCH_{3;} 4-OCH₂C=CH | H | |
| 633 | H | H | H | CH₂CH₃ | H | II H | H | 3-OCH3; 4-OCH₂C=CH | H | |
| 634 | H | H | H | CH₂CH₂CH₃. | H | H | H | 3-OCH₃; 4-OCH₂=CH | H | |
| 635 | H | H | H | CH₂CH₃ | CH₃ | H | H | 3-OCH_{3;} 4-OCH_{2C}=CH | H | |
| 636 | H | H | H | CH₂CH₃ | CH₃ | CH₃ | H | 3-OCH₃, 4-OCH₂C=CH | H | |
| 637 | H | H | H | CH₃ | CH₃ | H | H | 3-OCH_{3;}, 4-OCH₂C=CH | H | |
| 638. | H | H | H | CH₂CH₃ | CH₃; | CH₂QH₃ | H | 3-OCH₃; 4-OCH₂C=CH | H | |
| 639 | H | H | H | CH₃ | H | H' | 4-F | 4-OCH₂C=CH | H | |
| 640 | CH₃ | H | H | CH₃ | H | H | H; | 4-OCH₂C=H | H | |
| 641 | H | H | H | CH₃ | H | H | 4-F | 4-OCH₂C=CH | H | |
| 642 | H | H | H | CH₂CH₃ | H | H | H | 4-OCH₂C=CH | H | |
| 643 | H | H | H | CH2CH₂CH₃ | H | H | H | 4-OCH_{2C}=CH | H | |
| 644 | H | H | H | CH2CH₃ | CH₃ | H | H | 4-OCH₂C=CH | H | |
| 645 | H | H | H | CH₂CH₃. | CH₃ | CH2 | H | 4-OCH₂C=CH | H | |
| 646 | H | H | H | CH₃ | CH₃ | H | H | 4-OCH₂C=CH | H | |
| 647 | H | H | H | CH2CH₃ | CH₃ | CH₂CH₃ | H | 4-OCH₂C=CH | H | |
| 648 | H | H | H | CH₃ | H | H | 4-F | 4-OCH₂CH=CH₂ | H | |
| 649 | CH₃ | H | H | CH3. | H | H | H | 4-OCH₂CH=CH₂ | H | |
| 650 | H | H | H | CH₃ | H | H | 4-F | 4-OCH₂CH=CH₂ | H | |
| 651 | H | H | H | CH2CH₃ | H | H | H | +OCH₂CH=CH₂ | H | |
| 652 | H | H | H | CH₂CH₂CH₃ | H | H | H | +OCH₂CH=CH₂ | H | |
| 653 | H | H | H | CH₂CH₃ | CH₃ | H | H | 4-OCH₂CH=CH₂ | H | |
| 654 | H | H | H | CH₂CH₃ | CH₃ | CH₃ | H | 4-OCH₂CH=CH₂ , | H | |
| 655 | H | H | H | CH₃ | CH₃ | H | H | 4-OCH₂CH=CH₂ | H | |
| 656 | H | H | H | CH₂,CH₃. | CH₃ | CH₂CH₃ | H | 4-OCH₂CH=CH₂ | H | |
| 657 | H | H | H | CH₂cH₃ | H | H | H | 2-Cl | 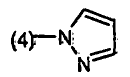 | (-) isomer |
| 658 | H | H | H | CH₃ | H | H | H | 3-F | 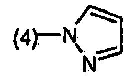 | (+)-isomer |
| 659 | H | H | H | CH₂CH₃ | H | H | H | H | 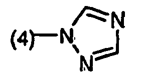 | (-) isomer |
| 660 | H | H | H | CH₂CH₃ | H | H | H | H | 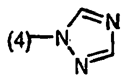 | (+)-isomer |
| 661 | H | H | H | CH₃ | H | H | H | H | 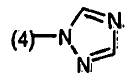 | (+)-isomer |
| 662 | H | H | H | CH₃ | H | H | H | H | 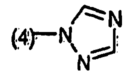 | (-) isomer |
| 663 | H | H | H | CH₂CH₃ | H | H | H | 4-OCH₂CH₃. | H | (-) isomer |
| 664 | H | H | H | CH₂CH₃ | H | H | H | 4-OCH₂CH₃ | H | (+)-isomer |
| 665 | | H | H | CH₃ | H | H | H | 4-OCH₂CH₃ | H | (-)-isomer |
| 666 | H | H | H | CH₃ | H | M | H | 4-OCH₂CH₃ | H | (+)-isomer |
| 667 | H | H | H | CH₂CH₃ | H | H | H | H | 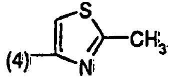 | (-)-isomer |
| 668 | H | H | H | CH₃ | H | H | H | H | 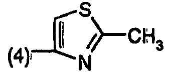 | (-)-isomer |
| 669 | H | H | H | CH₃ | H | H | H | H | 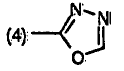 | (-)-isomer |
| 670 | H | H | H | CH₂CH₃. | H | H | H | 4-CN | H | (-)-isomer |
| 671 | H | H | H | CH₃ | H | H | H | 4-CN | H | (-)-isomer |
| 672 | H | H | H | CH₂CH₃ | H | H | H | 4-CN | H | (+)-isomer |
| 673 | H | H | H | CH₃ | H | H | H | 4-CN | H | (+)-isomer |
| 674 | H | H | H | CH₂CH₃ | H | H | H | 4-Cl | H | (-)-isomer |
| 675 | H | H | H | CH₂CH₃ | H | H | H | 4-Cl | H | (+)-isomer |
| 676 | H | H | H | CH₃ | H | H | H | H | 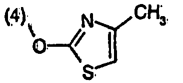 | |
| 677 | H | H | H | CH₂CH₃ | H | H | H | H | 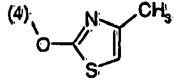 | |
| 678. | H | H | H | CH₃ | H | H | H | H | 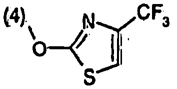 | |
| 679 | H | H | H | CH₂CH₃ | H | H | H | H | 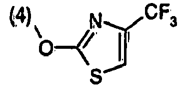 | |
| 680 | H | H | H | CH₃ | H | H | H | H | 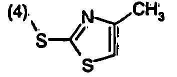 | |
| 681 | H | H | H | CH₂CH₃ | H | H | H | H | 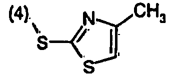 | |
| 682 | H | H | H | CH₃ | H | H | H | H | 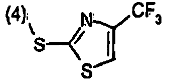 | |
| 683 | H | H | H | CH₂CH₃ | H | H | H | H | 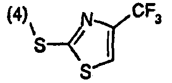 | |
| 684 | H | H | H | CH₃ | H | H | H | H | 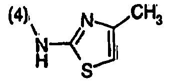 | |
| 68 | H | H | H | CH₂CH₃ | H | H | H | H | 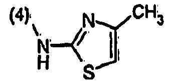 | |
| 686 | H | H | H | CH₃ | H | H | H | H | 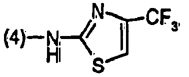 | |
| 687 | H | H | H | CH₂CH₃ | H | H | H | H | 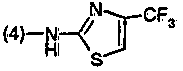 | |
| 688 | H | H | H | CH₃ | H | H | H | H | 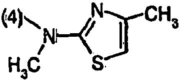 | |
| 689 | H | H | H | CH₂CH₃ | H | H | H | H | 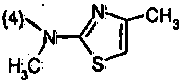 | |
| 690 | H | H | H | CH₃ | H | H | H | H | 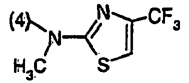 | |
| 691 | H | H | H | CH₂CH₃ | H | H | H | H | 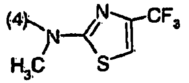 | |
| 692 | H | H | H | CH₃ | Hi | H | H | H | 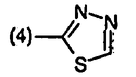 | |
| 693 | H | H | H | CH₂CH₃ | H | H | H | H | 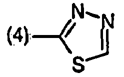 | |
| 694 | H | H | H | CH₃ | H | H | H | H | 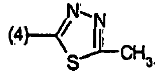 | |
| 695 | H | H | H | CH₂CH₃ | H | H | H | H | 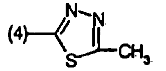 | |
| 696 | H | H | H | CH₃ | H | H | H | H | 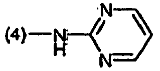 | |
| 697 | H | H | H | CH₂CH₃ | H | H | H | H | 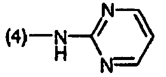 | |
| 698 | H | H | H | CH₃ | H | H | H | H | 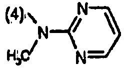 | |
| 699 | H | H | H | CH₂CH₃ | H | H | H | H | 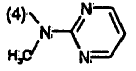 | |
| 700 | H | H | H | CH₃ | H | H | H | H | 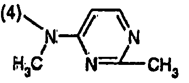 | |
| 701 | H | H | H | CH₂CH₃ | H | H | H | H | 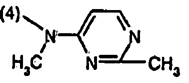 | |
| 702 | H | H | H | CH₃ | H | H | H | H | 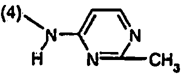 | |
| 703 | H | H | H | CH₂CH₃ | H | H | H | H | 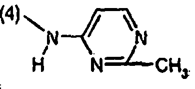 | |
| 704 | H | H | H | CH₃ | H | H | H | H | 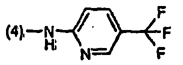 | |
| 705 | H | H | H | CH₂CH₃ | H | H | H | H | 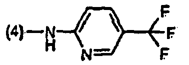 | |
| 706 | H | H | H | CH₃ | H | H | H | H | 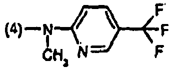 | |
| 707 | H | H | H | CH₂CH₃ | H | H | H | H | 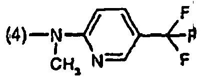 | |
| 708 | H | H | H | CH₃ | H | H | H | H | 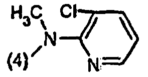 | |
| 709 | H | H | H | CH₂CH₃ | H | H | H | H | 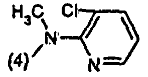 | |
| 710 | H | H | H | CH₃ | H | H | H | H | 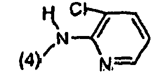 | |
| 711 | H | H | H | CH₂CH₃ | H | H | H | H | 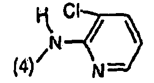 | |

Formulations may be prepared analogously to those described in, for example, WO 95/30651.

### Biological Examples

### D-1: Action against Plasmopara viticola on vines

### a) Residual-protective action

Vine seedlings are sprayed at the 4- to 5-leaf stage with a spray mixture (0.02 % active ingredient) prepared from a wettable powder formulation of the test compound. After 24 hours, the treated plants are infected with a sporangia suspension of the fungus. Fungus infestation is evaluated after incubation for 6 days at 95-100 % relative humidity and +20°C.

### b) Residual-curative action

Vine seedlings are infected at the 4- to 5-leaf stage with a sporangia suspension of the fungus. After incubation for 24 hours in a humidity chamber at 95-100 % relative humidity and +20°C, the infected plants are dried and sprayed with a spray mixture (0.02 % active ingredient) prepared from a wettable powder formulation of the test compound. After the spray coating has dried, the treated plants are placed in the humidity chamber again. Fungus infestation is evaluated 6 days after infection.
Compounds of Tables 1 exhibit a good fungicidal action against Plasmopara viticola on vines. Compounds No. 040, 052, 059, 071, 091, 101, 102, 104, 105, 157, 186, 197, 220, 228, 229, 271, 288, 289, 331, 332, 378, 381, 391, 421, 422, 452, 453, 469, 479, 489, 499, 512, 513, 514, 517, 464, 568, 607, 659 and 662 at 200 ppm inhibit fungal infestations in both tests D-1a) and D-1b) by 80-100%. At the same time untreated plants showed pathogen attack of 60 - 100 %.

### D-2: Action against Phytophthora on tomato plants

### a) Residual-protective action

After a cultivation period of 3 weeks, tomato plants are sprayed with a spray mixture (0.02 % active ingredient) prepared from a wettable powder formulation of the test compound. After 48 hours, the treated plants are infected with a sporangia suspension of the fungus. Fungus infestation is evaluated after incubation of the infected plants for 5 days at 90-100% relative humidity and +20°C.

### b) Systemic action

After a cultivation period of 3 weeks, tomato plants are watered with a spray mixture (0.02 % active ingredient based on the volume of the soil) prepared from a wettable powder formulation of the test compound. Care is taken that the spray mixture does not come into contact with the parts of the plants that are above the ground. After 96 hours, the treated plants are infected with a sporangia suspension of the fungus. Fungus infestation is evaluated after incubation of the infected plants for 4 days at 90-100 % relative humidity and +20°C. Compounds of Tables 1 exhibit a good fungicidal action against Plasmopara viticola on vines. Compounds No. 040, 052, 059, 071, 091, 101, 102, 104, 105, 157, 186, 197, 220, 228, 229, 271, 288, 289, 331, 332, 378, 381, 391, 421, 422, 452, 453, 469, 479, 489, 499, 512, 513, 514, 517, 464, 568, 607, 659 and 662 at 200 ppm inhibit fungal infestations in both tests D-1a) and D-1b) by 80 - 100 %. At the same time untreated plants showed pathogen attack of 60 - 100 %.

### D-3 : Action against Phytophthora on potato plants

### a) Residual-protective action

2-3 week old potato plants (Bintje variety) are sprayed with a spray mixture (0.02 % active ingredient) prepared from a wettable powder formulation of the test compound. After 48 hours, the treated plants are infected with a sporangia suspension of the fungus. Fungus infestation is evaluated after incubation of the infected plants for 4 days at 90-100 % relative humidity and +20°C.

### b) Systemic action

2-3 week old potato plants (Bintje variety) are watered with a spray mixture (0.02 % active ingredient based on the volume of the soil) prepared from a wettable powder formulation of the test compound. Care is taken that the spray mixture does not come into contact with the parts of the plants that are above the ground. After 48 hours, the treated plants are infected with a sporangia suspension of the fungus. Fungus infestation is evaluated after incubation of the infected plants for 4 days at 90-100 % relative humidity and +20°C. Fungal infestation is effectively controlled with compounds of Table 1.
Compounds 040, 052, 105, 157, 228, 229, 271, 288, 289, 332, 421, 422, 469, 479, 489, 514, 517, 564, 568, 607, 659 and 662 at 200 ppm inhibit fungal infestations in both tests D-3a) and D-3b) by 60 - 100 %. At the same time untreated plants showed a pathogen attack of 60 - 100 %.

## Claims

1. A compound of the general formula 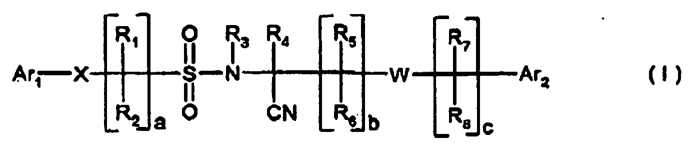 including the optical isomers thereof and mixtures of such Isomers, wherein Ar₁ and Ar₂ independently of each other stand for an optionally substituted aryl,
R₁ and R₂ stand independently of each other for hydrogen, optionally substituted C₁-C₅alkyl, optionally substituted C₂-C₅alkenyl, C₂-C₅alkynyl;
R₃ designates hydrogen, C₃-C₅alkenyl, C₃-C₅alkynyl or optionally substituted C₁-C₅alkyl;
R₄ is optionally substituted C₁-C₅alkyl, optionally substituted C₂-C₅alkenyl, C₂-C₅alkynyl;
R₅ and R₆ are independently of each other hydrogen or optionally substituted C₁-C₅alkyl, optionally substituted C₂-C₅alkenyl, C₂-C₅alkynyl;
R₇ and R₈ are Independently of each other hydrogen or optionally substituted C₁-C₅alkyl.;
W designates a bridge selected from -O-;
X designates a direct bond;
a and b independently of each other stand for a number 1, 2 or 3; and
c is zero,
wherein
Ar₁ stands for an aryl group which is optionally substituted with n radicals independently selected from R₉;
Ar₂ stands for an aryl group which is optionally substituted with n radicals independently selected from R'₉ and from one radical R₁₀;

2. A compound according to claim 1
wherein Ar₁ and Ar₂ independently of each other stand for phenyl; optionally substitutes by halogen, C₁-C₄alkyl, C₁-C₄haloalkyl, C₁-C₄alkoxy, C₁-C₄haloalkoxy, C₃-C₆cycloalkyl, -CN and -CO-R₁₄; and Ar₂ is optionally substituted by halogen, C₁-C₄alkyl, C₁-C₄haloalkyl, C₁-C₄alkoxy, C₁-C₄haloalkoxy, C₃-C₆cycloalkyl, -CN, -CO-R₁₄, -NR₁₂R₁₃, -NR₂-CO-R₁₂, -NR₃-CO-OR₁₂, -NR₂-CO-NR₈R₉, -NR₂CO-SR₁₂ , -NR₂-CS-OR₁₂, - NR₂-CS-NR₈R₉, -NR₂CS-SR₁₂, -S(O)ₚ-C₁-C₄alkyl, -S(O)ₚ-C₁-C₄haloalkyl, nitro, cyano and -CS-NH₂; and the optional substituent R₁₀ on Ar₂ is selected from optionally substituted phenyl, optionally substituted imidazolyl, optionally substituted thiazolyloxy, optionally substituted pyridyloxy, optionally substituted pyridyl, optionally substituted pyrimidinyloxy, optionally substituted pyrimidinyl, optionally substituted oxadiazolyl, optionally substituted triazolyl, optionally substituted pyrazolyl, optionally substituted oxadiazolyloxy, optionally substituted triazolyloxy and optionally substituted pyrazolyloxy.

3. A compound of formula I according to claim 1 wherein
Ar₁ and Ar₂ Independently stand for optional substituted aryl groups; and
the optional substituents R₉ of Ar₁ are preferably selected from the group comprising halogen, C₁-C₄alkyl, C₁-C₄haloalkyl, C₁-C₄alkoxy, C₁-C₄haloalkoxy, C₃-C₈cycloalkyl, -CN and -CO-R₁₄; and
the optional substituents R'₈ of Ar₂ are preferably selected from the group comprising halogen, C₁-C₄alkyl, C₁-C₄haloalkyl, C₁-C₄alkoxy, C₁-C₄haloalkoxy, C₃-C₆cycloalkyl, -CN, -CO-R₁₄, -NR₁₂R₁₃, -NR₂CO-R₁₂, -NR₃CO-OR₁₂, -NR₂-CO-NR₈R₉, -NR₂-CO-SR₁₂, -NR₂-CS-OR₁₂, -NR₂-CS-SR₁₂, -S(O)ₚ-C₁-C₄alkyl, -S(O)ₚ-C₁-C₄haloalkyl, -NR₂-SO₂-NR₈R₉, nitro , cyano and -CS-NH₂; and
the optional substituent R₁₀ on Ar₂ is selected from halogen, C₁-C₄alkyl, C₁-C₄haloalkyl, C₁-C₄alkoxy, -CN, -NO₂, -NR₁₂R₁₃, -CO-R₁₄ and the acyclic or cyclic ketals and acetals of -CO-R₁₄ ; -O-CO-R₁₄, optionally substituted phenyl, optionally substituted imidazolyl, optionally substituted thiazolyloxy, optionally substituted pyridyloxy, optionally substituted pyridyl, optionally substituted pyrimidinyloxy, optionally substituted, pyrimidinyl, optionally substituted oxadiazolyl, optionally substituted triazolyl, optionally substituted pyrazolyl, optionally substituted oxadiazolyloxy, optionally substituted triazolyloxy and optionally substituted pyrazolyloxy; and
R₁, R₂, R₅, R₆, R₇ and R₈ independently of each other are hydrogen or methyl; and R₃ is hydrogen or C₁-C₄alkyl optionally substituted with C₁-C₄alkoxy, C₃-C₄alkenyloxy, or C₃-C₄alkynyloxy; and
R₄ is hydrogen or C₁-C₄alkyl optionally substituted with halogen, C₁-C₃alkoxy
C₁-C₃alkylamlno or di-C₁-C₃alkylamino; and
the suffixes (a) and (b) designate the number 1.

4. A compound of formula I according to claim 1 wherein
Ar₁ and Ar₂ independently of each other stand for optionally substituted phenyl; and
the optional substituents R₈ and R'₉ of Ar₁ and Ar₂ are selected from the group comprising C₁-C₄alkyl, C₁-C₄haloalkyl, C₁-C₄alkoxy, C₁-C₄haloalkoxy and C₃-C₆cycloalkyl; and the optional substituent R₁₀ on Ar₂ is selected from -CO-C₁-C₄alkyl, -CO-C₁-C₄alkoxy, -O-CO-C₁-C₄alkyl, optionally substituted phenyl, optionally substituted phenoxy, optionally substituted imidazolyl, optionally substituted imidazolyloxy, optionally substituted thiazolyloxy, optionally substituted thiazolyl, optionally substituted thiadiazolyloxy, optionally substituted thiadiazolyl, optionally substituted pyridyloxy, optionally substituted pyridyl, optionally substituted pyrimidinyloxy, optionally substituted pyrimidinyl, optionally substituted oxadiazolyl, optionally substituted oxadiazotyloxy, optionally substituted triazolyl, optionally substituted pyrazolyl, optionally substituted triazolyloxy and optionally substituted pyrazolyloxy; and
R₁ and are independently C₁-C₄alkyl and R₂ and R₆ are hydrogen; and
R₃ is hydrogen, C₁-C₄alkyl or C₁-C₄alkoxy-C₁-C₄alkyl; and
R₄ is C₁-C₄alkyl or C₁-C₄haloalkyl; and
the suffixes (a) and (b) designate the number 1.

5. A compound of formula I according to claim 1 wherein
Ar₁ and Ar₂ independently of each other stand for optionally substituted phenyl; and the optional substituents R₈ and R'₉ of Ar₁ and Ar₂ are selected from the group comprising bromo, chloro, fluoro, iodo, cyano, hydroxy, amino, nitro, methyl, ethyl, propyl, isopropyl, methoxy, ethoxy, propoxy, isopropoxy, allyloxy, propargyloxy, benzyloxy, trifluoromethyl, trifluoromethoxy, 2-cyano-2-methyl-butyloxy, methylsulfonyl, methylsulfinyl, methylthio, cyclopentyl, cyclohexyl, aminocarbonylmethyl, methoximinoethyl, aminocarbonyl, butylcarbonylamino, tert-butylcarbonylamino, triazol-1-ylmethyl, 1,2,4-triazol-1-ylmethyl, N-morpholinocarbonylamino, aminocarbonyloxy-ethoxy, morpholinocarbonyloxyethoxy and cyanopyridyloxyethoxy; and
the optional substituent R₁₀ on Ar₂ is selected from aminocarbonyl, dimethylaminocarbonyl, acetyl, propionyl, acetoxy, methoxycarbonyl, ethoxycarbonyl, benzoyl, methoximinoethyl, 1-imidazolyl, 5-(3-methyl-1,2,4-thiadiazolyloxy), 2-pyridyl, 2-pyridyloxy, 4-pyrimidinyl, 2-(3,5-dichloropyrldyloxy), 2-(4,6-dichloropyridyloxy),
2-(4,6-dimethoxypyrimidinylthio), 2-oxadiazotyl, 2-(5-methyl-oxadiazolyl), 2-(5-ethyl-oxadiazolyl), 1-triazolyl, 1-pyrazolyl, 1-(3,4₋dimethylpyrazolyl), 4-(2-methylthiazolyl), 2-(1,3,4-oxydiazolyl), N-pyrrolidin-2-onyl, and 2-quinoxalinyl, and
R₁ and R₅ are Independently C₁-C₄alkyl and R₂ and R₆ are hydrogen; and
R₃ is hydrogen, C₁-C₄alkyl or C₁-C₄alkoxy C₁-C₄alkyl; and
R₄ is C₁-C₄alkyl or C₁-C₄haloalkyl; and
the suffixes (a) and (b) designate the number 1.

6. A compound according to claim 1, wherein
Ar₁ and Ar₂ Independently of each other stand for optionally substituted phenyl; and
the optional substituents R₉ and R'₉ of Ar₁ and Ar₂ are selected from the group comprising bromo, chloro, fluoro, methyl, ethyl, methoxy, ethoxy, trifluoromethyl and trifluoromethoxy; and
the optional substituent R₁₀ on Ar₂ is selected from aminocarbonyl, acetyl, methoxycarbonyl, ethoxycarbonyl, 1-imidazolyl, 5-(3-methyl-1,2,4-thiadiazolyloxy), 2-pyridyl, 2-pyridyloxy, 4-pyrimidinyl, 2-(3,5-dichloropyridyloxy),
2-(4,6-dimethoxypyrimidinyithio), 2-oxadiazolyl, 2-(5-methyl-oxadazolyl), 2-(5-ethyl-oxadiazolyle), 1-triazolyl, 1-pyrazolyl, 4-(2-methylthiazolyl), 2-(1,3,4-oxydiazolyl), and N-pyrrolidin-2-onyl, and
R₁ and R₅ are methyl and R₂ and R₆ are hydrogen; and
R₃ is hydrogen, methyl, ethyl, propyl, ethoxymethyl or methoxymethyl, and
R₄ is methyl, ethyl, propyl or fluoromethyl; and
the suffixes (a) and (b) designate the number 1.

7. A compound of formula I according to claim 1 selected from the group comprising
2-[(4-chlorophenoxy)-methyl]-2-benzylsulfonylemino-propionitrile,
2-[(4-chlorophenoxy)-methyl]-2-[(2-1hlorophenyl)-methyl]-sulfonylamino-propionitrite,
2-[(4-chlorophenoxy)-methyl]-2-[(2-fluorophenyl)-methyl)-sulfonylamino-propionitrite,
2-[(4-trifluoromethoxyphenoxy)-methyl]-2-benzylsulfonylamino-propionitrile,
2-[(4-chloro-3-methylphenoxy)-methyl]-2-benzylsulfonylamino-propionitrile,
2-[(4-chlorophenoxy)-methyl]-2-benzylsulfonylamino-butryronitrile,
2{(4-hlorophenoxy)-methyl]-2-benzylsulfonylamino-3-methoxy-propionitrile,
2-[(4-acetylphenoxy)-methyl]-2-benzylsulfonylamino-propionitrile,
2-[(4-methoxyphenoxy)-methyl)-2-benzylsulfonylamino-propionitrile,
2-[(4-acetylphenoxy)-methyl]-2-benzylsulfonylamino-butyronitrile,
2-[(4-cyanophenoxy)-methyl]-2-benzylsulfonylamino-propionitrile,
(-)-2-[(4-cyanophenoxy)-methyl]-2-benzylsulfonylamlno-propionitrile,
2-[(4-propionylphenoxy)-methyl]-2-benzylsulfonylamino-propionitrile,
2-[(4-ethoxyphenoxy)-methyl]-2-benzylsulfonylamino-propionitrile,
2-[(4-[1,2,4]triazol-1-yl-phenoxy)-methyl]-2-benzylsulfonylamino-propionitrile,
2-[(4-imidazol-1-yl-phenoxy)-methyl]-2-benzylsulfonylamino-propionitrile,
2-[(4-cyanophenoxy)-methyl]-2-benzylsulfonylamino-butyronitrile,
2-[(4-[1,3,4]oxadiazol-4-yl-phenoxy)-methyl]-2-benzylsulfonylamino-propionitrile,
2-[(4-methoxyphenoxy)-methyl]-2-benzylsulfonylamino-butyronitrile,
2-[(4-ethoxyphenoxy)-methyl]-2-benzylsulfonylamino-butyronitrile,
(-)2-[(4-ethoxyphenoxy)-methyl]-2-benzylsulfonylamino-butyronitrile,
2-[(4-[1,2,4]triazol-1-yl-phenoxy)-methyl]-2-benzylsulfonylamino-butyronitrile,
2-[(4-methoxycarbonylphenoxy)-methyl]-2-benzylsulfonylamino-propionitrile,
2-[(4-propionylphenoxy)-methyl]-2-benzylsulfonylamino-butyronitrile,
2-[(4-chlorophenoxy)-methyl]-2-benzylsulfonylamino-3-fluoro-propionitrile,
2-{[4-(2-methyl-thiazol-4-yl)-phenoxy]-methyl}-2-benzylsulfonylamino-butyronitrile,
2-[(4-pyrazol-1-yl-phenoxy)-methyl]-2-benzylsulfonylamino-butyronitrile,
2-(5-oxo-5,6,7,8,-tetrahydronaphth-2-yloxy)-methyl]-2-benzylsulfonylamino-butyronitrile,
2-[(4-chloro-phenoxy)-methyl]-2-benzylsulfonylamino-3-methyl-butyronitrile,
2-[(4-iso-propyl-phenoxy)-methyl]-2-benzylsulfonylamino-butyronitrile,
2-[(4-nitro-phenoxy)-methyl]-2-benzylsulfonylamino-butyronitrile,
2-[(4-cyano-phenoxy)-methyl]-2-benzylsulfonylamino-butyronitrile,
2-[(3-fluoro-4-propionyl-phenoxy)-methyl]-2-benzylsulfonylamino-propionitrile,
(-) -2-[(4-[1,2,4]triazol-1-yl-phenoxy)-methy]-2-benzylsulfonylamino-butyronitrile, and
(-)-2-[(4-acetylphenoxy)-methyl]-2-benzylsuffonylamino-propionitrile.

8. A process for the preparation of a compound of formula I according to claim 1, which comprises
a) reacting the the sulfonylating agent of formula II 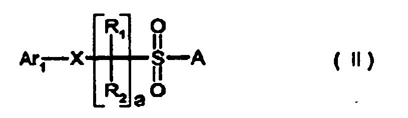 wherein Ar₁, a, X and R₁ to R₂, are defined as under formule I, and A stands for a leaving group like an anhydride, i.e. -O-SO₂-(CR₁R₂)ₐ-X-Ar₁ or -O-CO-C₁-C₄alkyl, but preferably for halogen, especially bromine or more preferably chlorine, with an amino-acetonitrile of formula III 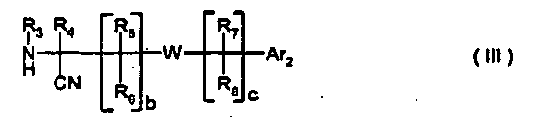 wherein Ar₂, b, c, W and R₃ to R₆, are defined as under formula I, or
b) coupling the compound of formula XIII 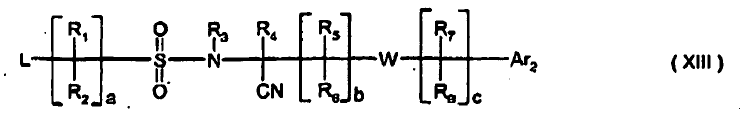 wheren Ar₁, Ar₂, a, b, c, W and R₁ to R₈ are defined as under formula I and L is a leaving group such as e.g. halogen, preferably chlorine, bromine or iodine or a sulfonyloxy group such as e.g. methylsulfonyloxy-, toluylsulfonyloxy- or trifluoromethylsulfonyloxy- group, with a compound of formula XIV
Ar₁—X' (XIV)
wherein Ar₁ **is** defined as under formula I and X' is either an anionic radical species of X such as O', S⁻, S(O)ₘ⁻ combined with an alkaline- or earthalkaline- metal cation as counterion or is defined as X-H such as OH, SH, NHR₃ if at the same time the reaction is generally carried out in the presence of a base such as alkaline-, earthalkaline-carbonates or hydrogencarbonates such e.g. sodium or potassium-carbonate, sodium or potassium -hydrogen-carbonate, cesium-carbonate or an agent capable of scavenging the formed acid.

9. A composition for controlling and protecting against phytopathogenic microorganisms, comprising a compound of formula I according to claim 1 as active ingredient together with a suitable carrier.

10. The use of a compound of formula I according to claim 1 in protecting plants against infestation by phytopathogenic microorganisms.

11. A method of controlling and preventing an infestation of crop plants by phytopathogenic microorganisms, which comprises the application of a compound of formula I according to claim 1 as active Ingredient to the plant, to parts of plants or to the locus thereof.

12. A method according to claim 11, wherein the phytopathogenic microorganisms are fungal organisms.

## Patentansprüche

1. Verbindung der allgemeinen Formel 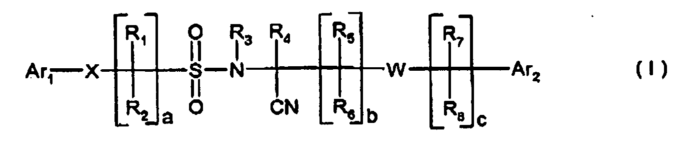 einschließlich der optischen Isomeren davon und Gemische von solchen Isomeren, worin
Ar₁ und Ar₂ unabhängig voneinander für ein gegebenenfalls substituiertes Aryl stehen,
R₁ und R₂ unabhängig voneinander für Wasserstoff, gegebenenfalls substituiertes C₁-C₅-Alkyl, gegebenenfalls substituiertes C₂-C₅-Alkenyl, C₂-C₅-Alkinyl stehen;
R₃ Wasserstoff, C₃-C₅-Alkenyl, C₃-C₅-Alkinyl oder gegebenenfalls substituiertes C₁-C₅-Alkyl bedeutet;
R₄ gegebenenfalls substituiertes C₁-C₅-Alkyl, gegebenenfalls substituiertes C₂-C₅-Alkenyl, C₂-C₅-Alkinyl darstellt;
R₅ und R₆ unabhängig voneinander Wasserstoff oder gegebenenfalls substituiertes C₁-C₅-Alkyl, gegebenenfalls substituiertes C₂-C₅-Alkenyl, C₂-C₅-Alkinyl darstellen;
R₇ und R₈ unabhängig voneinander Wasserstoff oder gegebenenfalls substituiertes C₁-C₅-Alkyl darstellen;
W eine Brücke, ausgewählt aus -O-, bedeutet;
X eine direkte Bindung bedeutet;
a und b unabhängig voneinander für eine Zahl 1, 2 oder 3 stehen; und
c null ist,
wobei
Ar₁ für eine Aryl-Gruppe steht, die gegebenenfalls substituiert ist mit n Resten, unabhängig ausgewählt aus R₉;
Ar₂ für eine Aryl-Gruppe steht, die gegebenenfalls substituiert ist mit n Resten, unabhängig ausgewählt aus R'₉ und aus einem Rest R₁₀.

2. Verbindung nach Anspruch 1,
worin Ar₁ und Ar₂ unabhängig voneinander stehen für Phenyl; gegebenenfalls substituiert mit Halogen, C₁-C₄-Alkyl, C₁-C₄-Halogenalkyl, C₁-C₄-Alkoxy, C₁-C₄-Halogenalkoxy, C₃-C₆-Cycloalkyl , -CN und -CO-R₁₄; und Ar₂, gegebenenfalls substituiert ist mit Halogen, C₁-C₄-Alkyl, C₁-C₄-Halogenalkyl, C₁-C₄-Alkoxy, C₁-C₄-Halogen-alkoxy, C₃-C₆-Cycloalkyl , -CN, -CO-R₁₄ , -NR₁₂R₁₃, -NR₂-CO-R₁₂, NR₃-CO-OR₁₂ , -NR₂-CO-NR₈R₉, -NR₂CO-SR₁₂, -NR₂-CS-OR₁₂, -NR₂-CS-NR₈R₉, -NR₂-CS-SR₁₂, -S (O)ₚ C₁-C₄-Alkyl, -S (O)ₚ C₁-C₄-Halogenalkyl, Nitro, Cyano und -CS-NH₂; und der wahlweise Substituent R₁₀ an Ar₂ ausgewählt ist aus gegebenenfalls substituiertem Phenyl, gegebenenfalls substituiertem Imidazolyl, gegebenenfalls substituiertem Thiazolyloxy, gegebenenfalls substituiertem Pyridyloxy, gegebenenfalls substituiertem Pyridyl, gegebenenfalls substituiertem Pyrimidinyloxy, gegebenenfalls substituiertem Pyrimidinyl, gegebenenfalls substituiertem Oxadiazolyl, gegebenenfalls substituiertem Triazolyl, gegebenenfalls substituiertem Pyrazolyl, gegebenenfalls substituiertem Oxadiazolyloxy, gegebenenfalls substituiertem Triazolyloxy und gegebenenfalls substituiertem Pyrazolyloxy.

3. Verbindung der Formel I nach Anspruch 1, worin
Ar₁ und Ar₂ unabhängig voneinander für gegebenenfalls substituierte Aryl-Gruppen stehen; und die wahlweisen Substituenten R₉ von Ar₁ vorzugsweise ausgewählt sind aus der Gruppe, umfassend Halogen, C₁-C₄-Alkyl, C₁-C₄-Halogenalkyl, C₁-C₄-Alkoxy, C₁-C₄-Halo-genalkoxy, C₃-C₆-Cycloalkyl, -CN und -CO-R₁₄; und die wahlweisen Substituenten R'₉ von Ar₂ vorzugsweise ausgewählt sind aus der Gruppe, umfassend Halogen, C₁-C₄-Alkyl, C₁-C₄-Halogenalkyl, C₁-C₄-Alkoxy, C₁-C₄-Halogenalkoxy, C₃-C₆-Cycloalkyl, -CN, -CO-R₁₄ , -NR₁₂R₁₃, -NR₂-CO-R₁₂, -NR₃-CO-OR₁₂ , -NR₂-CO-NR₈R₉, -NR₂-CO-SR₁₂, -NR₂-CS-OR₁₂, -NR₂-CS-SR₁₂, -S (O) ₚ-C₁-C₄-Alkyl, -S (O) ₚ-C₁-C₄-Halogenalkyl, -NR₂-SO₂-NR₈R₉, Nitro, Cyano und -CS-NH₂; und der wahlweise Substituent R₁₀ an Ar₂ ausgewählt ist aus Halogen, C₁-C₄-Alkyl, C₁-C₄-Halogenalkyl, C₁-C₄-Alkoxy, -CN, -NO₂, -NR₁₂R₁₃, -CO-R₁₄, und den acyclischen oder cyclischen Ketalen und Acetalen von -CO-R₁₄; -O-CO-R₁₄, gegebenenfalls substituiertem Phenyl, gegebenenfalls substituiertem Imidazolyl, gegebenenfalls substituiertem Thiazolyloxy, gegebenenfalls substituiertem Pyridyloxy, gegebenenfalls substituiertem Pyridyl, gegebenenfalls substituiertem Pyrimidinyloxy, gegebenenfalls substituiertem Pyrimidinyl, gegebenenfalls substituiertem Oxadiazolyl, gegebenenfalls substituiertem Triazolyl, gegebenenfalls substituiertem Pyrazolyl, gegebenenfalls substituiertem Oxadiazolyloxy, gegebenenfalls substituiertem Triazolyloxy und gegebenenfalls substituiertem Pyrazolyloxy, und
R₁, R₂, R₅, R₆, R₇ und R₈ unabhängig voneinander Wasserstoff oder Methyl darstellen; und
R₃ Wasserstoff oder C₁-C₄-Alkyl, gegebenenfalls substituiert mit C₁-C₄-Alkoxy, C₃-C₄-Alkenyloxy, oder C₃-C₄-Alkinyloxy, darstellt; und
R₄ Wasserstoff oder C₁-C₄-Alkyl, gegebenenfalls substituiert mit Halogen, C₁-C₃-Alkoxy, C₁-C₃-Alkylamino oder Di-C₁-C₃-alkylamino, darstellt; und
die Suffixe (a) und (b) die Zahl 1 bedeuten.

4. Verbindung der Formel I nach Anspruch 1, worin
Ar₁ und Ar₂ unabhängig voneinander für gegebenenfalls substituiertes Phenyl stehen; und
die wahlweisen Substituenten R₉ und R'₉ von Ar₁ und Ar₂ ausgewählt sind aus der Gruppe, umfassend C₁-C₄-Alkyl, C₁-C₄-Halogenalkyl, C₁-C₄-Alkoxy, C₁-C₄-Halogenalkoxy, und C₃-C₆-Cycloalkyl; und
der wahlweise Substituent R₁₀ an Ar₂ ausgewählt ist aus -CO-C₁-C₄-Alkyl, -CO-C₁-C₄-Alkoxy, -O-CO-C₁-C₄-Alkyl, gegebenenfalls substituiertem Phenyl, gegebenenfalls substituiertem Phenoxy, gegebenenfalls substituiertem Imidazolyl, gegebenenfalls substituiertem Imidazolyloxy, gegebenenfalls substituiertem Thiazolyloxy, gegebenenfalls substituiertem Thiazolyl, gegebenenfalls substituiertem Thiadiazolyloxy, gegebenenfalls substituiertem Thiadiazolyl, gegebenenfalls substituiertem Pyridyloxy, gegebenenfalls substituiertem Pyridyl, gegebenenfalls substituiertem Pyrimidinyloxy, gegebenenfalls substituiertem Pyrimidinyl, gegebenenfalls substituiertem Oxadiazolyl, gegebenenfalls substituiertem Oxadiazolyloxy, gegebenenfalls substituiertem Triazolyl, gegebenenfalls substituiertem Pyrazolyl, gegebenenfalls substituiertem Triazolyloxy und gegebenenfalls substituiertem Pyrazolyloxy; und
R₁ und R₅ unabhängig C₁-C₄-Alkyl darstellen und R₂ und R₆ Wasserstoff darstellen; und
R₃ Wasserstoff, C₁-C₄-Alkyl oder C₁-C₄-Alkoxy-C₁-C₄-alkyl darstellt; und
R₄ C₁-C₄-Alkyl oder C₁-C₄-Halogenalkyl darstellt; und
die Suffixe (a) und (b) die Zahl 1 bedeuten.

5. Verbindung der Formel I nach Anspruch 1, worin
Ar₁ und Ar₂ unabhängig voneinander für gegebenenfalls substituiertes Phenyl stehen; und die wahlweisen Substituenten R₉ und R'₉ von Ar₁ und Ar₂ ausgewählt sind aus der Gruppe, umfassend Brom, Chlor, Fluor, Jod, Cyano, Hydroxy, Amino, Nitro, Methyl, Ethyl, Propyl, Isopropyl, Methoxy, Ethoxy, Propoxy, Isopropoxy, Allyloxy, Propargyloxy, Benzyloxy, Trifluormethyl, Trifluormethoxy, 2-Cyano-2-methyl-butyloxy, Methylsulfonyl, Methylsulfinyl, Methylthio, Cyclopentyl, Cyclohexyl, Aminocarbonylmethyl, Methoximinoethyl, Aminocarbonyl, Butylcarbonylamino, tert-Butylcarbonylamino, Triazol-1-ylmethyl, 1,2,4-Triazol-1-yl-methyl, N-Morpholinocarbonylamino, Aminocarbonyloxy-ethoxy, Morpholinocarbonyl-oxyethoxy und Cyanopyridyl-oxyethoxy; und
der wahlweise Substituent R₁₀ an Ar₂ ausgewählt ist aus Aminocarbonyl, Dimethylaminocarbonyl, Acetyl, Propionyl, Acetoxy, Methoxycarbonyl, Ethoxycarbonyl, Benzoyl, Methoximinoethyl, 1-Imidazolyl, 5-(3-Methyl-1,2,4-thiadiazolyloxy), 2-Pyridyl, 2-Pyridyloxy, 4-Pyrimidinyl, 2-(3,5-Dichlorpyridyloxy), 2-(4,6-Dichlorpyridyloxy), 2-(4,6-Dimethoxy-pyrimidinylthio), 2-Oxadiazolyl, 2-(5-Methyl-oxadiazolyl), 2-(5-Ethyl-oxadiazolyl), 1-Triazolyl, 1-Pyrazolyl, 1-(3,4-Dimethylpyrazolyl), 4-(2-Methylthiazolyl}, 2-(1,3,4-Oxydiazolyl), N-Pyrrolidin-2-on-yl, und 2-Chinoxalinyl, und
R₁ und R₅ unabhängig C₁-C₄-Alkyl darstellen und R₂ und R₆ Wasserstoff darstellen; und
R₃ Wasserstoff, C₁-C₄-Alkyl oder C₁-C₄-Alkoxy-C₁-C₄-alkyl darstellt; und
R₄ C₁-C₄-Alkyl oder C₁-C₄-Halogenalkyl darstellt; und
die Suffixe (a) und (b) die Zahl 1 bedeuten.

6. Verbindung nach Anspruch 1, worin
Ar₁ und Ar₂ unabhängig voneinander für gegebenenfalls substituiertes Phenyl stehen; und
die wahlweisen Substituenten R₉ und R'₉ von Ar₁ und Ar₂ ausgewählt sind aus der Gruppe, umfassend Brom, Chlor, Fluor, Methyl, Ethyl, Methoxy, Ethoxy, Trifluormethyl und Trifluormethoxy; und
der wahlweise Substituent R₁₀ an Ar₂ ausgewählt ist aus Aminocarbonyl, Acetyl, Methoxycarbonyl, Ethoxycarbonyl, 1-Imidazolyl, 5-(3-Methyl-1,2,4-thiadiazolyloxy), 2-Pyridyl, 2-Pyridyloxy, 4-Pyrimidinyl, 2-(3,5-Dichlorpyridyloxy), 2-(4,6-Dimethoxypyrimidinylthio), 2-Oxadiazolyl, 2-(5-Methyl-oxadiazolyl), 2-(5-Ethyl-oxadiazolyl) , 1-Triazolyl, 1-Pyrazolyl, 4-(2-Methylthiazolyl), 2-(1,3,4-Oxydiazolyl) und N-Pyrrolidin-2-on-yl, und
R₁ und R₅ Methyl darstellen und R₂ und R₆ Wasserstoff darstellen; und
R₃ Wasserstoff, Methyl, Ethyl, Propyl, Ethoxymethyl oder Methoxymethyl darstellt; und
R₄ Methyl, Ethyl, Propyl, oder Fluormethyl darstellt; und
die Suffixe (a) und (b) die Zahl 1 bedeuten.

7. Verbindung der Formel I nach Anspruch 1, ausgewählt aus der Gruppe, umfassend
2-[(4-Chlorphenoxy)-methyl]-2-benzylsulfonylamino-propionitril,
2-[(4-Chlorphenoxy)-methyl]-2-[2-chlorphenyl)-methyl]-sulfonylamino-propionitril,
2-[(4-Chlorphenoxy)-methyl]-2-[(2-chlorphenyl)-methyl]-sulfonylamino-propionitril,
2-[(4-Trifluormethoxy-phenoxy)-methyl]-2-benzylsulfonylamino-propionitril,
2-[(4-Chlor-3-methylphenoxy)-methyl]-2-benzylsulfonylamino-propionitril,
2-[(4-Chlorphenoxy)-methyl]-2-benzylsulfonylamino-butyronitril,
2-[(4-Chlorphenoxy)-methyl]-2-benzylsulfonylamino-3-methoxy-propiontril,
2-[(4-Acetylphenoxy)-methyl]-2-benzylsulfonylamino-propionitril,
2-[(4-Methoxyphenoxy)-methyl]-2-benzylsulfonylamino-propionitril,
2-[(4-Acetylphenoxy)-methyl]-2-benzylsulfonylamino-butyronitril,
2-[(4-Cyanophenoxy)-methyl]-2-benzylsulfonylamino-propionitril,
(-)-2-[(4-Cyanophenoxy)-methyl]-2-benzylsulfonylamino-propionitril,
2-[(4-Propionyl-phenoxy)-methyl]-2-benzylsulfonylamino-propionitril,
2-[(4-Ethoxyphenoxy)-methyl]-2-benzylsulfonylamino-propionitril,
2-[(4-[1,2,4]-Triazol-1-yl-phenoxy)-methyl]-2-benzylsulfonylaminopropionitril,
2-[(4-Imidazol-l-yl-phenoxy)-methyl] -2-benzylsulfonylamino-propionitril,
2-[(4-Cyano-phenoxy)-methyl]-2-benzylsulfonylamino-butyronitril,
2-[(4-[1,3,4]-Oxadiazol-4-yl-phenoxy)-methyl]-2-benzylsulfonylamino-propionitril,
2-[(4-Methoxyphenoxy)-methyl]-2-benzylsulfonylamino-butyronitril,
2-[(4-Ethoxyphenoxy)-methyl]-2-benzylsulfonylamino-butyronitril,
(-)-2-[(4-Ethoxyphenoxy)-methyl]-2-benzylsulfonylamino-butyronitril,
2-[(4-[1,2,4]-Triazol-1-yl-phenoxy)-methyl]-2-benzylsulfonylaminobutyronitril,
2-[(4-Methoxycarbonyl-phenoxy)-methyl]-2-benzylsulfonylamino-propionitril,
2-[(4-Propionylphenoxy)-methyl]-2-benzylsulfonylamino-butyronitril,
2-[(4-Chlorphenoxy)-methyl]-2-benzylsulfonylamino-3-fluor-propionitril,
2-{[4-(2-Methyl-thiazol-4-yl)-phenoxy]-methyl)-2-benzylsulfonylamino-butyronitril,
2-[(4-Pyrazol-1-yl-phenoxy)-methyl]-2-benzylsulfonylamino-butyronitril,
2-(5-Oxo-5, 6, 7, 8-tetrahydronaphth-2-yl-oxy)-methyl]-2-benzylsulfonylamino-butyronitril,
2-[(4-Chlor-phenoxy)-methyl]-2-benzylsulfonylamino-3-methyl-butyronitril,
2-[(4-Iso-propyl-phenoxy)-methyl]-2-benzylsulfonylamino-butyronitril,
2-[(4-Nitro-phenoxy)-methyl]-2-benzylsulfonylamino-butyronitril,
2-[(4-Cyano-phenoxy)-methyl]-2-benzylsulfonylamino-butyronitril,
2-[(3-Fluor-4-propionyl-phenoxy)-methyl]-2-benzylsulfonylaminopropionitril,
(-)-2-[(4-[1,2,4]-Triazol-1-yl-phenoxy)-methyl]-2-benzylsulfonylamino-butyronitril, und
(-)-2-[(4-Acetylphenoxy)-methyl]-2-benzylsulfonylamino-propionitril.

8. Verfahren zur Herstellung einer Verbindung der Formel I nach Anspruch 1, das umfasst
a) Umsetzen des Sulfonylierungsmittels der Formel II 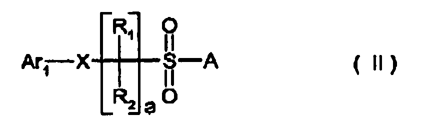 worin Ar₁, a, X und R₁ bis R₂ wie unter Formel I definiert sind, und A für eine Abgangsgruppe, wie ein Anhydrid, steht, d.h. -O-SO₂-(CR₁R₂)ₐ-X-Ar₁ oder -O-CO-C₁-C₄-Alkyl, jedoch vorzugsweise für Halogen, insbesondere Brom oder bevorzugter Chlor, mit einem Amino-acetonitril der Formel III 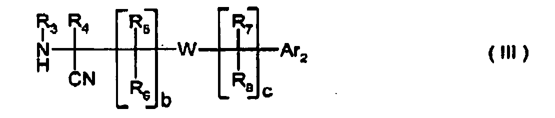 worin Ar₂, b, c, W und R₃ bis R₈ wie unter Formel I definiert sind, oder
b) Kuppeln der Verbindung der Formel XIII 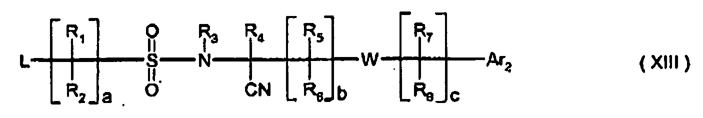
worin Ar₁, Ar₂, a, b, c, W und R₁ bis R₈ wie unter Formel I definiert sind, und L eine Abgangsgruppe, wie zum Beispiel Halogen, vorzugsweise Chlor, Brom oder Jod, oder eine Sulfonyloxy-Gruppe, wie zum Beispiel Methyl-sulfonyloxy-, Toluyl-sulfonyloxy- oder Trifluormethyl-sulfonyloxy-Gruppe darstellt, mit einer Verbindung der Formel XIV
Ar₁-X' (XIV)
worin Ar₁ wie unter Formel I definiert ist und X' entweder eine anionische Rest-Spezies von X wie O⁻, S⁻, S (O)ₘ⁻, kombiniert mit einem Alkali- oder Erdalkali-metall-Kation als Gegenion, darstellt oder als X-H, wie OH, SH, NHR₃, definiert ist, wenn gleichzeitig die Reaktion im Allgemeinen in Gegenwart von einer Base, wie Alkalimetall-, Erdalkalimetall-carbonaten oder -hydrogencarbonaten, wie zum Beispiel Natrium- oder Kaliumcarbonat, Natrium- oder Kalium-hydrogen-carbonat, Cäsium-carbonat oder einem Mittel, das die gebildete Säure einfangen kann, ausgeführt wird.

9. Zusammensetzung zum Bekämpfen von und Schützen gegen phytopathogene(n) Mikroorganismen, umfassend eine Verbindung der Formel I nach Anspruch 1 als Wirkbestandteil zusammen mit einem geeigneten Träger.

10. Verwendung einer Verbindung der Formel I nach Anspruch 1 beim Schutz von Pflanzen gegen den Befall durch phytopathogene Mikroorganismen.

11. Verfahren zur Bekämpfung und Prävention eines Befalls von Kulturpflanzen durch phytopathogene Mikroorganismen, das die Applikation von einer Verbindung der Formel I nach Anspruch 1 als Wirkbestandteil auf die Pflanze, auf Teile der Pflanzen oder deren Standort umfasst.

12. Verfahren nach Anspruch 11, wobei die phytopathogenen Mikroorganismen Pilz-Organismen darstellen.

## Revendications

1. Composé de formule générale 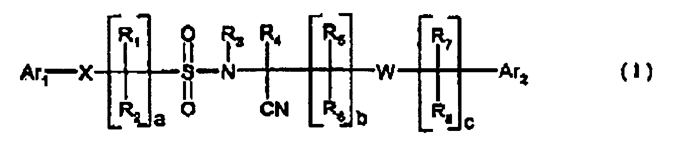 comprenant ses isomères optiques et des mélanges de tels isomères, dans lequel
Ar₁ et Ar₂ représentent indépendamment l'un de l'autre un groupe aryle facultativement substitué,
R₁ et R₂ représentent indépendamment l'un de l'autre un atome d'hydrogène, un groupe alkyle en C₁ à C₅ facultativement substitué, alcényle en C₂ à C₅ facultativement substitué, alcynyle en C₂ à C₅;
R₃ désigne un atome d'hydrogène, un groupe alcényle en C₃ à C₅, alcynyle en C₃ à C₅ ou alkyle en C₁ à C₅ facultativement substitué ;
R₄ est un groupe alkyle en C₁ à C₅ facultativement substitué, alcényle en C₂ à C₅ facultativement substitué, alcynyle en C₂ à C₅ ;
R₅ et R₆ sont indépendamment l'un de l'autre un hydrogène ou un groupe alkyle en C₁-C₅ facultativement substitué, alcényle en C₂ à C₅ facultativement substitué, alcynyle en C₂ à C₅ ;
R₇ et R₈ sont indépendamment l'un de l'autre un hydrogène ou un groupe alkyle en C₁-C₅ facultativement substitué ;
W désigne un pont choisi parmi -O- ;
X désigne une liaison directe ;
a et b représentent indépendamment l'un de l'autre le chiffre 1, 2 ou 3 ; et
c vaut zéro,
dans lequel
Ar₁ représente un groupe aryle qui est facultativement substitué par n radicaux indépendamment choisis parmi R₉ ;
Ar₂ représente un groupe aryle qui est facultativement substitué par n radicaux indépendamment choisis parmi R'₉ et un radical R₁₀ ;

2. Composé selon la revendication 1,
dans lequel Ar₁ et Ar₂ représentent indépendamment l'un de l'autre un groupe phényle ; facultativement substitué par un atome d'halogène, un groupe alkyle en C₁ à C₄, halogénoalkyle en C₁ à C₄, alcoxy en C₁ à C₄, halogénoalcoxy en C₁ à C₄, cycloalkyle en C₃ à C₆, -CN et -CO-R₁₄ ; et Ar₂ est facultativement substitué par un atome d'halogène, un groupe alkyle en C₁ à C₄, halogénoalkyle en C₁ à C₄, alcoxy en C₁ à C₄, halogénoalcoxy en C₁ à C₄, cycloalkyle en C₃ à C₆, -CN, -CO-R₁₄, -NR₁₂R₁₃ , -NR₂-CO-NR₁₂, -NR₃-CO-OR₁₂, -NR₂-CO-NR₈R₉, -NR₂-CO-SR₁₂, -NR₂-CS-OR₁₂, -NR₂-CS-NR₈R₉, -NR₂-CS-SR₁₂, -S (O)ₚ-alkyle en C₁ à C₄, -S (O)ₚ-halogénoalkyle en C₁ à C₄, nitro, cyano et -CS-NH₂ ; et le substituant facultatif R₁₀ sur Ar₂ est choisi parmi un groupe phényle facultativement substitué, imidazolyle facultativement substitué, thiazolyloxy facultativement substitué, pyridyloxy facultativement substitué, pyridyle facultativement substitué, pyrimidinyloxy facultativement substitué, pyrimidinyle facultativement substitué, oxadiazolyle facultativement substitué, triazolyle facultativement substitué, pyrazolyle facultativement substitué, oxadiazolyloxy facultativement substitué, triazolyloxy facultativement substitué et pyrazolyloxy facultativement substitué.

3. Composé de formule I selon la revendication 1, dans lequel
Ar₁ et Ar₂ représentent indépendamment des groupes aryle facultativement substitués ; et
les substituants facultatifs R₉ de Ar₁ sont choisis de préférence dans le groupe comprenant un atome d' halogène, un groupe alkyle en C₁ à C₄, halogénoalkyle en C₁ à C₄, alcoxy en C₁ à C₄, halogénoalcoxy en C₁ à C₄, cycloalkyle en C₃ à C₆, -CN et -CO-R₁₄ ; et
les substituants facultatifs R'₉ de Ar₂ sont de préférence choisis dans le groupe comprenant un atome d'halogène, un groupe alkyle en C₁ à C₄, halogénoalkyle en C₁ à C₄, alcoxy en C₁ à C₄, halogénoalcoxy en C₁ à C₄, cycloalkyle en C₃ à C₆, -CN, -CO-R₁₄, -NR₁₂R₁₃, -NR₂-COR₁₂, -NR₃-CO-OR₁₂, -NR₂-CO-NR₈R₉, -NR₂-CO-SR₁₂, -NR₂-CS-OR₁₂, -NR₂-CS-SR₁₂, -S(O)ₚ-alkyle en C₁ à C₄, -S(O)p-halogénoalkyle en C₁ à C₄, -NR₂-SO₂-NR₈R₉, nitro, cyano et -CS-NH₂ ; et
le substituant facultatif R₁₀ sur Ar₂ est choisi parmi un atome d'halogène, un groupe alkyle en C₁ à C₄, halogénoalkyle en C₁ à C₄, alcoxy en C₁ à C₄, -CN, -NO₂, -NR₁₂R₁₃, -CO-R₁₄, et les cétals et acétals acycliques ou cycliques de -CO-R₁₄ ; -O-CO-R₁₄, un groupe phényle facultativement substitué, imidazolyle facultativement substitué, thiazolyloxy facultativement substitué, pyridyloxy facultativement substitué, pyridyle facultativement substitué, pyrimidinyloxy facultativement substitué, pyrimidinyle facultativement substitué, oxadiazolyle facultativement substitué, triazolyle facultativement substitué, pyrazolyle facultativement substitué, oxadiazolyloxy facultativement substitué, triazolyloxy facultativement substitué et pyrazolyloxy facultativement substitué ; et
R₁, R₂, R₅, R₆, R₇ et R₈ sont indépendamment les uns des autres, un atome d'hydrogène ou un groupe méthyle ; et
R₃ est un atome d'hydrogène ou un groupe alkyle en C₁ à C₄ facultativement substitué par un groupe alcoxy en C₁ à C₄, alcénylyloxy en C₃ à C₄, ou alcynyloxy en C₃ à C₄ ; et
R₄ est un atome d'hydrogène ou un groupe alkyle en C₁ à C₄ facultativement substitué par un atome d'halogène, un groupe alcoxy en C₁ à C₃, alkylamino en C₁ à C₃ ou di- (alkyl en C₁ à C₃) amino ; et
les suffixes (a) et (b) désignent le chiffre 1.

4. Composé de formule I selon la revendication 1, dans lequel
Ar₁ et Ar₂ représentent indépendamment l'un de l'autre un groupe phényle facultativement substitué ; et
les substituants facultatifs R₉ et R'₉ de Ar₁ et Ar₂ sont choisis dans le groupe comprenant un groupe alkyle en C₁ à C₄, halogénoalkyle en C₁ à C₄, alcoxy en C₁ à C₄, halogénoalcoxy en C₁ à C₄ et cycloalkyle en C₃ à C₆ ; et
le substituant facultatif R₁₀ sur Ar₂ est choisi parmi un groupe -CO-alkyle en C₁ à C₄, -CO-alcoxy en C₁ à C₄, -O-CO-alkyle en C₁ à C₄, phényle facultativement substitué, phénoxy facultativement substitué, imidazolyle facultativement substitué, imidazolyloxy facultativement substitué, thiazolyloxy facultativement substitué, thiazolyle facultativement substitué, thiadiazolyloxy facultativement substitué, thiadiazolyle facultativement substitué, pyridyloxy facultativement substitué, pyridyle facultativement substitué, pyrimidinyloxy facultativement substitué, pyrimidinyle facultativement substitué, oxadiazolyle facultativement substitué, oxadiazolyloxy facultativement substitué, triazolyle facultativement substitué, pyrazolyle facultativement substitué, triazolyloxy facultativement substitué et pyrazolyloxy facultativement substitué ; et
R₁ et R₅ sont indépendamment un groupe alkyle en C₁ à C₄ et R₂ et R₆ sont un atome d'hydrogène ; et
R₃ est un atome d'hydrogène, un groupe alkyle en C₁ à C₄ ou alcoxy en C₁ à C₄-alkyle en C₁ à C₄ ; et
R₄ est un groupe alkyle en C₁ à C₄ ou un groupe halogénoalkyle en C₁ à C₄ ; et
les suffixes (a) et (b) désignent le chiffre 1.

5. Composé de formule I selon la revendication 1, dans lequel Ar₁ et Ar₂ représentent indépendamment l'un de l'autre un groupe phényle facultativement substitué ; et
les substituants facultatifs R₉ et R'₉ de Ar₁ et Ar₂ sont choisis dans le groupe comprenant les groupes bromo, chloro, fluoro, iodo, cyano, hydroxy, amino, nitro, méthyle, éthyle, propyle, isopropyle, méthoxy, éthoxy, propoxy, isopropoxy, allyloxy, propargyloxy, benzyloxy, trifluorométhyle, trifluorométhoxy, 2-cyano-2-méthyl-butyloxy, méthylsulfonyle, méthylsulfinyle, méthylthio, cyclopentyle, cyclohexyle, aminocarbonylméthyle, méthoximinoéthyle, aminocarbonyle, butylcarbonylamino, tert-butylcarbonylamino, triazol-1-ylméthyle, 1,2,4-triazol-1-ylméthyle, N-morpholinocarbonylamino, aminocarbonyloxy-éthoxy, morpholinocarbonyloxyéthoxy et cyanopyridyloxyéthoxy ; et
le substituant facultativement R₁₀ sur Ar₂ est choisi parmi les groupes aminocarbonyle, diméthylaminocarbonyle, acétyle, propionyle, acétoxy, méthoxycarbonyle, éthoxycarbonyle, benzoyle, méthoximinoéthyle, 1-imidazolyle, 5-(3-méthyl-1,2,4-thiadiazolyloxy), 2-pyridyle, 2-pyridyloxy, 4-pyrimidinyle, 2-(3,5-dichloropyridyloxy), 2-(4,6-dichloropyridyloxy), 2-(4,6-diméthoxypyrimidinylthio), 2-oxadiazolyle, 2-(5-méthyl-oxadiazolyle), 2-(5-éthyloxadiazolyle), 1-triazolyle, 1-pyrazolyle, 1-(3,4-diméthylpyrazolyle), 4-(2-méthylthiazolyle), 2-(1,3,4-oxydiazolyle), N-pyrrolidin-2-onyle, et 2-quinoxalinyle, et
R₁ et R₅ sont indépendamment un groupe alkyle en C₁ à C₄ et R₂ et R₆ sont un atome d'hydrogène ; et
R₃ est un atome d'hydrogène, un groupe alkyle en C₁ à C₄ ou alcoxy en C₁ à C₄-alkyle en C₁ à C₄ ; et
R₄ est un groupe alkyle en C₁ à C₄ ou un groupe halogénoalkyle en C₁ à C₄ ; et
les suffixes (a) et (b) désignent le chiffre 1.

6. Composé selon la revendication 1, dans lequel
Ar₁ et Ar₂ représentent indépendamment l'un de l'autre un groupe phényle facultativement substitué ; et
les substituants facultatifs R₉ et R'₉ de Ar₁ et Ar₂ sont choisis dans le groupe comprenant les groupes bromo, chloro, fluoro, méthyle, éthyle, méthoxy, éthoxy, trifluorométhyle et trifluorométhoxy ; et
le substituant facultatif R₁₀ sur Ar₂ est choisi parmi les groupes aminocarbonyle, acétyle, méthoxycarbonyle, éthoxycarbonyle, 1-imidazolyle, 5-(3-méthyl-1,2,4-thiadiazolyloxy), 2-pyridyle, 2-pyridyloxy, 4-pyrimidinyle, 2-(3,5-dichloropyridyloxy), 2-(4,6-diméthoxypyrimidinylthio), 2-oxadiazolyle, 2-(5-méthyloxadazolyle), 2-(5-éthyl-oxadiazolyle), 1-triazolyle, 1-pyrazolyle, 4-(2-méthylthiazolyle), 2-(1,3,4-oxydiazolyle) et N-pyrrolidin-2-onyle, et
R₁ et R₅ sont un groupe méthyle et R₂ et R₅ sont un atome d'hydrogène ; et
R₃ est un atome d'hydrogène, un groupe méthyle, éthyle, propyle, éthoxyméthyle ou méthoxyméthyle ; et
R₄ est un groupe méthyle, éthyle, propyle ou fluorométhyle ; et
les suffixes (a) et (b) désignent le chiffre 1.

7. Composé de formule I selon la revendication 1, choisi dans le groupe comprenant
le 2-[(4-chlorophénoxy)-méthyl]-2-benzylsulfonylaminopropionitrile,
le 2-[(4-chlorophénoxy)-méthyl]-2-[(2-chlorophényl) - méthyl]-sulfonylamino-propionitrile,
le 2-[(4-chlorophénoxy)-méthyl]-2-[(2-fluorophényl)-méthyl]-sulfonylamino-propionitrile,
le 2-[(4-trifluorométhoxyphénoxy)-méthyl]-2-benzylsulfonylamino-propionitrile,
le 2-[(4-chloro-3-méthylphénoxy)-méthyl]-2-benzylsulfonylamino-propionitrile,
le 2-[(4-chlorophénoxy)-méthyl]-2-benzylsulfonylaminobutyronitrile,
le 2-[(4-chlorophénoxy)-méthyl]-2-benzylsulfonylamino-3-méthoxy-propionitrile,
le 2-[(4-acétylphénoxy)-méthyl]-2-benzylsulfonylaminopropionitrile,
le 2-[(4-méthoxyphénoxy)-méthyl]-2-benzylsulfonylaminopropionitrile,
le 2-[(4-acétylphénoxy)-méthyl]-2-benzylsulfonylaminobutyronitrile,
le 2-[(4-cyanophénoxy)-méthyl]-2-benzylsulfonylaminopropionitrile,
le (-)-2-[(4-cyanophénoxy)-méthyl]-2-benzylsulfonylamino-propionitrile,
le 2-[(4-propionylphénoxy)-méthyl]-2-benzylsulfonylamino-propionitrile,
le 2-[(4-éthoxyphénoxy)-méthyl]-2-benzylsulfonylaminopropionitrile,
le 2-[(4-[1,2,4]triazol-1-yl-phénoxy)-méthyl]-2-benzylsulfonylamino-propionitrile,
le 2-[(4-imidazol-1-yl-phénoxy)-méthyl]-2-benzylsulfonylamino-propionitrile,
le 2-[(4-cyanophénoxy)-méthyl]-2-benzylsulfonylaminobutyronitrile,
le 2-[(4-[1,3,4]oxadiazol-4-yl-phénoxy)-méthyl]-2-benzylsulfonylamino-propionitrile,
le 2-[(4-méthoxyphénoxy)-méthyl]-2-benzylsulfonylaminobutyronitrile,
le 2-[(4-éthoxyphénoxy)-méthyl]-2-benzylsulfonylaminobutyronitrile,
le (-)-2-[(4-éthoxyphénoxy)-méthyl]-2-benzylsulfonylamino-butyronitrile,
le 2-[(4-[1,2,4]triazol-1-yl-phénoxy)-méthyl]-2-benzylsulfonylamino-butyronitrile,
le 2-[(4-méthoxycarbonylphénoxy)-méthyl]-2-benzylsulfonylamino-propionitrile,
le 2-[(4-propionylphénoxy)-méthyl]-2-benzylsulfonylamino-butyronitrile,
le 2-[(4-chlorophénoxy)-méthyl]-2-benzylsulfonylamino-3-fluoro-propionitrile,
le 2-{[4-(2-méthyl-thiazol-4-yl)-phénoxy]-méthyl)-2-benzylsulfonylamino-butyronitrile,
le 2-[(4-pyrazol-l-yl-phénoxy)-méthyl]-2-benzylsulfonylamino-butyronitrile,
le 2-[(5-oxo-5,6,7,8-tétrahydronapht-2-yloxy)-méthyl]-2-benzylsulfonylamino-butyronitrile,
le 2-[(4-chloro-phénoxy)-méthyl]-2-benzylsulfonylamino-3-méthyl-butyronitrile,
le 2-[(4-iso-propyle-phénoxy)-méthyl]-2-benzylsulfonylamino-butyronitrile,
le 2-[(4-nitro-phénoxy)-méthyl]-2-benzylsulfonylaminobutyronitrile,
le 2-[(4-cyano-phénoxy)-méthyl]-2-benzylsulfonylaminobutyronitrile,
le 2-[(3-fluoro-4-propionylphénoxy)-méthyl]-2-benzylsulfonylamino-propionitrile,
le (-)-2-[(4-[1,2,4]triazol-1-yl-phénoxy)-méthyl]-2-benzylsulfonylamino-butyronitrile, et
le (-)-2-[(4-acétylphénoxy)-méthyl]-2-benzylsulfonylamino-propionitrile.

8. Procédé de préparation d'un composé de formule I selon la revendication 1, qui comprend les étapes consistant à
a) faire réagir l'agent de sulfonylation de formule II 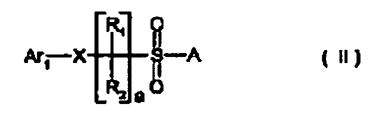 dans laquelle Ar₁, a, X, R₁ et R₂ sont définis comme pour la formule I, et A représente un groupe partant tel qu'un anhydride, c'est-à-dire un groupe -O-SO₂-(CR₁R₂)ₐ-X-Ar₁ ou -O-CO-alkyle en C₁ à C₄, mais de préférence un atome d'halogène, spécialement du brome ou de manière davantage préférée du chlore, avec un amino-acétonitrile de formule III 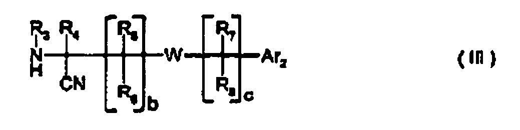 dans laquelle Ar₂, b, c, W et R₃ à R₈ sont définis comme pour la formule I, ou
b) coupler le composé de formule XIII 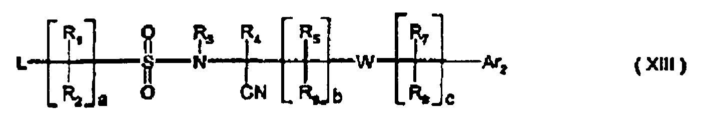
dans laquelle Ar₁, Ar₂, a, b, c, W et R₁ à R₈ sont définis comme pour la formule I et L est un groupe partant tel que par exemple un halogène, de préférence le chlore, le brome ou l'iode ou un groupe sulfonyloxy tel que, par exemple, un groupe méthylsulfonyloxy, toluylsulfonyloxy ou trifluorométhylsulfonyloxy, à un composé de formule XIV
**Ar₁—X'** **(XIV)**
dans laquelle Ar₁ est défini comme pour la formule I et X' est soit une espèce radicalaire anionique de X telle que O⁻, S⁻, S (O)ₘ⁻ combiné avec un cation de métal alcalin ou alcalino-terreux en tant que contre-ion ou est défini par X-H tel que OH, SH, NHR₃ si au même moment, la réaction est réalisée de manière générale en présence d'une base telle que des carbonates ou hydrogénocarbonates alcalins ou alcalino-terreux tels que, par exemple, le carbonate de sodium ou de potassium, l'hydrogénocarbonate de sodium ou de potassium, le carbonate de césium ou un agent capable de piéger l'acide formé.

9. Composition pour lutter contre et protéger vis-à-vis de micro-organismes phytopathogènes, comprenant un composé de formule I selon la revendication 1 en tant qu'ingrédient actif conjointement avec un support approprié.

10. Utilisation d'un composé de formule I selon la revendication 1 dans la protection de plantes vis-à-vis d'une infestation par des micro-organismes phytopathogènes.

11. Procédé pour lutter contre et prévenir une infestation de plantes cultivées par des micro-organismes phytopathogènes, qui comprend l'application d'un composé de formule I selon la revendication 1 en tant qu'ingrédient actif à la plante, à des parties de plantes ou à leur locus.

12. Procédé selon la revendication 11, dans lequel les micro-organismes phytopathogènes sont des organismes fongiques.
